# EUROPEAN PATENT SPECIFICATION

(11) **EP 3 286 301 B1**
(45) Date of publication and mention of the grant of the patent: **28.07.2021**
(21) Application number: 16782394.7
(22) Date of filing: 22.04.2016
(51) Int. Cl.: C12N 5/077, A61K 35/34, C12N 5/02, A61K 35/545, G01N 33/20, A61P 21/00, A61P 21/04

(54) **GENERATION OF MUSCLE-LINEAGE CELLS FROM STEM CELLS**
ERZEUGUNG VON DIFFERENZIERUNG VON MUSKELLINIENZELLEN AUS STAMMZELLEN
GÉNÉRATION DE CELLULES DE LIGNÉES MUSCULAIRES À PARTIR DE CELLULES SOUCHES

(30) Priority: 22.04.2015 US 201562151352 P
(43) Date of publication of application: 28.02.2018
(73) Proprietor: Sonic Master Limited, Road Town, VG1110 Tortola (VG)
(72) Inventor: SCHMIDT, Uli, Sydney, New South Wales 2000 (AU); CARON, Leslie, Sydney, New South Wales 2000 (AU)
(74) Representative: inCompass IP Europe Limited
(86) International application number: PCT/AU2016/000144
(87) International publication number: WO 2016/168890

(56) References cited:
- WO-A1-2013/138623
- WO-A1-2016/108288
- AU-A1- 2014 268 197
- ALESSANDRA CASTIGLIONI ET AL: "Isolation of Progenitors that Exhibit Myogenic/Osteogenic Bipotency In Vitro by Fluorescence-Activated Cell Sorting from Human Fetal Muscle", STEM CELL REPORTS, vol. 2, no. 1, 1 January 2014 (2014-01-01) , pages 92-106, XP055540947, United States ISSN: 2213-6711, DOI: 10.1016/j.stemcr.2013.12.006
- LESLIE CARON ET AL: "A Human Pluripotent Stem Cell Model of Facioscapulohumeral Muscular Dystrophy-Affected Skeletal Muscles : FSHD Stem Cell Model", STEM CELLS TRANSLATIONAL MEDICINE, vol. 5, no. 9, 23 May 2016 (2016-05-23), pages 1145-1161, XP055541391, US ISSN: 2157-6564, DOI: 10.5966/sctm.2015-0224
- BORCHIN B ET AL.: 'Derivation and FACS-Mediated Purification of PAX3+/PAX7+ Skeletal Muscle Precursors from Human Pluripotent Stem Cells' STEM CELL REPORTS vol. 1, 2013, pages 620 - 631, XP055324367
- SHELTON M ET AL.: 'Derivation and Expansion of PAX7-Positive Muscle Progenitors from Human and Mouse Embryonic Stem Cells' STEM CELL REPORTS vol. 3, 2014, pages 516 - 529, XP055281322
- MAHMOOD A ET AL.: 'Enhanced differentiation of human embryonic stem cells to mesenchymal progenitors by inhibition of TGF-(3/Activin/Nodal signaling using SB- 431542' JOURNAL OF BONE AND MINERAL RESEARCH vol. 25, 2010, pages 1216 - 1233, XP008160765
- CHAL J ET AL.: 'Differentiation of pluripotent stem cells to muscle fiber to model Duchenne muscular dystrophy' NATURE BIOTECHNOLOGY vol. 33, 2015, pages 962 - 969, XP055281323
- SHELTON M ET AL.: 'Robust generation and expansion of skeletal muscle progenitors and myocytes from human pluripotent stem cells' METHODS vol. 101, 2016, pages 73 - 84, XP055324368
- LEE H ET AL.: 'Identification of small molecules which induce skeletal muscle differentiation in embryonic stem cells via activation of the Wnt and inhibition of Smad2/3 and Sonic Hedgehog pathways' STEM CELLS vol. 34, 2016, pages 299 - 310, XP055324370

## Description

### RELATED APPLICATIONS

This application claims the benefit of U.S. Provisional Application No. 62/151,352, filed April 22, 2015, which is incorporated by reference herein in its entirety.

### BACKGROUND

Satellite cells are precursors to skeletal muscle cells. In adult muscle, satellite cells are generally quiescent, but can activate and undergo myogenesis in response to disease or mechanical strain such as injury or exercise. Satellite cells are also involved in the normal growth of muscle. Upon activation, satellite cells can proliferate. Additionally, some of these satellite cells can differentiate into myoblasts, which can fuse to form myotubes and other skeletal muscle components. The myoblasts can also augment existing muscle fibers by fusing with existing myotubes.

Muscular diseases and disorders, both developmental and degenerative, can cause the gradual or sudden loss of muscular function due to the decline or death of muscle cells, as well as lessened muscular development due to developmental diseases. Congenital myopathies are examples of muscular diseases that present these characteristics. Muscle loss may also occur from aging, from the treatment of diseases, or from a number of other causes. Examples of these types of muscle loss include sarcopenia and cachexia. There is a need in the art for therapies for the various types of muscle loss.

Borchin B et al.; "Derivation and FACS-Mediated Purification of PAX3+/PAX7+ Skeletal Muscle Precursors from Human Pluripotent Stem Cells" STEM CELL REPORTS, vol. 1, 2013, pages 620-631, XP055324637 discloses a method based on the use of a small-molecule GSK3β inhibitor to derive skeletal muscle from several human pluripotent stem cells (hPSC) lines. It discloses that early GSK3β inhibition is sufficient to create the conditions necessary for highly effective derivation of muscle cells.

### SUMMARY OF THE INVENTION

This disclosure provides artificially-produced satellite cells, or satellite-like cells, that can be used in potential therapies for muscle loss associated with various causes, including diseases, disorders and aging, as well as methods of making such cells. The cells can also be used in screens to identify drugs with particular effects on satellite cells.

In one aspect, a method is provided for producing cells expressing CD56/Pax3, CD56/Pax7, or Pax3/Pax7, the method comprising providing a pluripotent stem cell in an *in vitro* culture and contacting the pluripotent stem cell in the *in vitro* culture with two or more compounds at the same time, wherein the two or more compounds comprise a GSK3β inhibitor and/or a TGF-β receptor inhibitor, wherein the contacting the pluripotent stem cell in the *in vitro* culture with the two or more compounds comprising a GSK3β inhibitor and/or a TGF- β receptor inhibitor, directly results in generation of cells expressing CD56/Pax3, CD56/Pax7, or Pax3/Pax7. In some embodiments, the cells expressing CD56/Pax3, CD56/Pax7, or Pax3/Pax7 have the potential to form myoblasts. In some cases, the generation of cells expressing CD56/Pax3, CD56/Pax7, or Pax3/Pax7 is not caused by transfection of a nucleic acid. In some embodiments, the generation of cells expressing CD56/Pax3, CD56/Pax7, or Pax3/Pax7 occurs in a single step. In some embodiments, the pluripotent stem cell is a human embryonic stem cell, or human induced pluripotent stem cell. In some embodiments, the human pluripotent stem cell is a genetically modified human pluripotent stem cell; a human pluripotent stem cell comprising a mutation associated with a genetic muscle disease or disorder; or the human pluripotent stem cell is genetically modified to correct a phenotype of a subject with a genetic disease or disorder. In some embodiments, at least a portion of the cells expressing CD56/Pax3, CD56/Pax7, or Pax3/Pax7 are Pax3/Pax7/ CD56 cells. In some embodiments, less than 20 days from initially contacting the pluripotent stem cell in the *in vitro* culture with the two or more compounds at the same time, greater than five cells expressing CD56/Pax3, CD56/Pax7, or Pax3/Pax7 are produced from the pluripotent stem cell greater than 10 cells expressing CD56/Pax3, CD56/Pax7, or Pax3/Pax7 are produced from the pluripotent stem cell; or greater than five cells expressing MyoD, MYOG or MYF5 are produced from the pluripotent stem cell. In some embodiments, at least a portion of the cells expressing CD56/Pax3, CD56/Pax7, or Pax3/Pax7 are satellite-like cells. In some embodiments, at least a portion of the cells expressing CD56/Pax3, CD56/Pax7, or Pax3/Pax7 have a nucleus with a cross-sectional area of at least about 170µm². In some embodiments, the TGF-β receptor inhibitor is an Alk inhibitor or an Alk5 inhibitor. In some embodiments, the two or more compounds further comprise a G3K3β inhibitor, preferably, CHIR99021, or AZS1080. In some embodiments, the method comprises *in vitro* differentiation of the human pluripotent stem cell into a cell that is capable of forming a myoblast, wherein the method comprises contacting the human pluripotent stem cell with two or more compounds, and wherein the two or more compounds comprise a G3K3β inhibitor and a TGF-β receptor inhibitor. In some embodiments, the cell that is capable of forming a myoblast expresses CD56/Pax3, CD56/Pax7 or Pax3/Pax7. In some embodiments, the G3K3β inhibitor comprises CHIR99021 or AZD1080; and the TGF-β receptor inhibitor is an ALK5 inhibitor, preferably SB431542 or A83-01. In any of the above embodiments, the two or more compounds comprise a leucine-rich repeat kinase 2 (LRRK2) inhibitor. In some embodiments, the LRRK2 inhibitor is LRRK2-IN-1. embodiments, the two or more compounds comprise horse serum. In some embodiments, the two or more compounds comprise a compound selected from the group consisting of transferrin, XAV939, VEGF, SB431542, fibroblast growth factor, BIX01294, IGF-1, Noggin, Creatine, PD169316, SMO antagonist, and sodium butyrate. In some embodiments, the two or more compounds comprise a Wnt pathway activator or a GSK3I3 inhibitor. In some embodiments, the two or more compounds comprise a GSK313 inhibitor. In some embodiments, the GSK313 inhibitor is CHIR99021 or AZD1080. In some embodiments, less than 20 days from initially contacting the pluripotent stem cell in the *in vitro* culture with one compound, or with two or more compounds at the same time, greater than five cells expressing MyoD, MYOG or MYF5 are produced from the pluripotent stem cell.

In another aspect, a method is provided for *in vitro* differentiation of a human pluripotent stem cell into a cell that is capable of forming a myoblast, the method comprising contacting the human pluripotent stem cell with two or more compounds, wherein the two or more compounds comprise a Wnt pathway activator and a TGF-13 receptor inhibitor. In some embodiments, the cell that is capable of forming a myoblast expresses CD56/Pax3, CD56/Pax7 or Pax3/Pax7. In some embodiments, the Wnt pathway activator is a GSK3β inhibitor. In some embodiments, the Wnt pathway activator is present at a concentration of between 0.1 µM and 8 µM, inclusive. In some embodiments, the GSK3β inhibitor is CHIR99021 or AZD1080. In some embodiments, the TGF- β receptor inhibitor is an Alk5 inhibitor. In some embodiments, the TGF- β receptor inhibitor is SB431542. In some embodiments, the TGF- β receptor inhibitor is A83-01. In some embodiments, the TGF β receptor inhibitor is present at a concentration of between 0.1 µM and 10 µM, inclusive. In some embodiments, the differentiation of a human pluripotent stem cell into a cell that is capable of forming a myoblast occurs in a single step. In some embodiments, the human pluripotent stem cell is contacted with the Wnt pathway activator and a TGF- β receptor inhibitor in at least a 1:1 ratio of Wnt pathway activator to TGF- β receptor inhibitor. In some embodiments, the human pluripotent stem cell is contacted with the Wnt pathway activator and the TGF- β receptor inhibitor in at least a 3:2 ratio of Wnt pathway activator to TGF- β receptor inhibitor. In some embodiments, the contacting occurs for 10 days or less. In some embodiments, the contacting occurs for 20 days or less.

In any of the above embodiments, the one compound or the two or more compounds do not comprise a growth factor. In any of the above embodiments, the one compound or the two or more compounds do not comprise FGF, IGF, or HGF. In any of the above embodiments, the one compound or the two or more compounds do not comprise FGF2. In any of the above embodiments, the pluripotent stem cell is not contacted with a growth factor. In any of the above embodiments, the cells produced by the method do not express one or more genes selected from the group consisting of: MyoD, MYOG, and MYF5. In any of the above embodiments, the method does not comprise cell sorting. In any of the above embodiments at least about 50% of the cells generated by the method express CD56/Pax3, CD56/Pax7 or Pax3/Pax7. In any of the above embodiments, at least about 90% of the cells generated by the method express CD56/Pax3, CD56/Pax7 or Pax3/Pax7. In any of the above embodiments, the method further comprises culturing the cells on a culture surface coated with an extracellular matrix. In some embodiments, the extracellular matrix comprises laminin. In some embodiments, the extracellular matrix comprises a substance selected from the group consisting of: laminin 111, laminin 211 and laminin 521. In some embodiments, the extracellular matrix comprises collagen. In some embodiments, the collagen is collagen type I. In any of the above embodiments, the one compound or the two or more compounds comprise a leucine-rich repeat kinase 2 (LRRK2) inhibitor. In some embodiments, the LRRK2 inhibitor is LRRK2-IN-1. In any of the above embodiments, the one compound or the two or more compounds comprise a Rho-associated protein kinase (ROCK) inhibitor. In some embodiments, the ROCK inhibitor is Y-27632. In some embodiments, the ROCK inhibitor is present at a concentration of between 0.1 µM to 10 µM, inclusive. In any of the above embodiments, cells generated by the method can be further differentiated to generate a population of cells wherein at least 50% of the population of cells comprises myoblasts. In any of the above embodiments, cells generated by the method can be further differentiated to generate a population of cells wherein at least 50% of the population of cells form myotubes.

In other aspects, the present disclosure provides cells produced by any method described herein. In another aspect, the disclosure provides a cell produced using the method of any of the above embodiments, wherein the cell expresses CD56/Pax3. In another aspect, the disclosure provides a cell produced using the method of any of the above embodiments, wherein the cell expresses CD56/Pax7. In yet another aspect, the disclosure provides a cell produced using the method of any of the above embodiments, wherein the cell expresses CD56, Pax3, and Pax7. In yet another aspect, the disclosure provides a cell produced using the method of any of the above embodiments, wherein the cell expresses Pax3/Pax7. In another aspect, the disclosure provides a cell produced using the method of any of the above embodiments, wherein the cell is capable of differentiating to a myoblast *in vitro.* In another aspect, the disclosure provides a cell produced using the method of any of the above embodiments, wherein the cell is capable of differentiating to a myoblast *in vivo.* In another aspect, the disclosure provides a cell produced using the method of any of the above embodiments, wherein the cell does not occur in nature.

In another aspect, a method is provided comprising: (a) providing an HLA-null human pluripotent stem cell in an *in vitro* culture; and (b) differentiating the HLA-null human pluripotent stem cell into a lineage-committed HLA-null cell. In some embodiments, the lineage-committed HLA-null cell, or derivative thereof, is introduced into a subject in need thereof. In some embodiments, the differentiating the HLA-null human pluripotent stem cell into the lineage-committed HLA-null cell occurs in a single step. In some embodiments, the lineage-committed HLA-null cell is a skeletal muscle progenitor cell. In some embodiments, the lineage-committed HLA-null cell is capable of forming a myoblast cell. In some embodiments, the lineage-committed HLA-null cell is a satellite cell or satellite-like cell. In some embodiments, the lineage-committed HLA-null cell expresses at least one of Pax3, Pax7, MyoD, MF20, and CD56.

In yet another aspect, a method is provided comprising contacting a pluripotent stem cell with a CRISPR enzyme designed to alter genomic loci encoding HLA such that the genomic loci encoding HLA no longer produces functional HLA, thereby generating an HLA-null pluripotent stem cell. In some embodiments, the pluripotent stem cell is derived from a human. In some embodiments, the method further comprises differentiating the HLA-null pluripotent stem cell into an HLA-null skeletal muscle progenitor cell. In some embodiments, the HLA-null skeletal muscle progenitor cell is capable of forming a myoblast. In some embodiments, the CRISPR enzyme is introduced with two or more guide RNAs in order to delete a portion of the genomic loci encoding HLA that is sufficient such that the pluripotent stem cell no longer produces functional HLA. In some embodiments, the CRISPR enzyme is introduced with one or more guide RNAs in order to produce a mutation of the genomic loci encoding HLA that is sufficient such that the pluripotent stem cell no longer produces functional HLA. In some embodiments, the CRISPR enzyme is introduced with one or more guide RNAs and a template DNA molecule with similarity to the genomic loci encoding HLA, or surrounding HLA, in order to induce a recombination event that is sufficient such that the pluripotent stem cell no longer produces functional HLA. In some embodiments, the CRISPR enzyme is Cas9.

In another aspect, a method is provided comprising culturing an HLA-null pluripotent stem cell under conditions to promote differentiation of the HLA-null pluripotent stem cell into a cell expressing Pax3 and Pax7, thereby producing an HLA-null cell expressing Pax3 and Pax7. In some embodiments, the conditions of culturing the HLA-null pluripotent stem cell comprise contacting the HLA-null pluripotent stem cell with two or more compounds to promote differentiation of the HLA-null pluripotent stem cell into the cell expressing Pax3 and Pax7. In some embodiments, the conditions of culturing the HLA-null pluripotent stem cell comprise conditions of transfection.

In another aspect, a method of treating a subject with muscular deficiency is provided comprising: (a) obtaining cells produced by any of the methods of the above embodiments; and (b) introducing the cells into the subject with the muscular deficiency. In some embodiments, the deficiency is caused by muscular dystrophy. In some embodiments, the muscular deficiency is caused by Duchenne muscular dystrophy. In some embodiments, the muscular deficiency is caused by cachexia. In some embodiments, the muscular deficiency is caused by sarcopenia. In some embodiments, the cells obtained by any of the methods of the above embodiments are satellite-like cells that are implanted on a scaffold prior to introduction to the subject with the muscular deficiency. In some embodiments, following the introduction of the cells to the subject with the muscular deficiency, the subject with the muscular deficiency does not mount a significant immune response against the cells. In some embodiments, a dosage of the cells provided to the subject is based on severity of the disease of the subject. In some embodiments, a dosage of the cells is sufficient to cause an increase of muscle mass in the subject. In some embodiments, the introducing the cells to the subject with the muscular deficiency comprises injecting the cells into an arm muscle of the subject with the muscular deficiency. In some embodiments, the introducing the cells to the subject with the muscular deficiency comprises injecting the cells into a leg muscle of the subject with the muscular deficiency. In some embodiments, a dosage of the cells is sufficient to cause an increase of muscle mass in the leg muscle of the subject with the muscular deficiency. In some embodiments, the pluripotent stem cell is derived from the subject with the muscular deficiency. In some embodiments, the pluripotent stem cell derived from the subject with the muscular deficiency is genetically modified to reverse a phenotype associated with the muscular deficiency.

In another aspect, a cell culture is provided comprising: (a) cells expressing CD56/Pax3, CD56/Pax7 or Pax3/Pax7 that have the potential to form a myoblast; (b) a Wnt pathway activator; and (c) a TGF- β receptor inhibitor.

In another aspect, a method of screening a candidate agent is provided comprising: (a) providing one or more cells generated from the method of any of the above embodiments, wherein the one or more cells generated by the method comprise a phenotype; (b) contacting the one or more cells generated by the method with the candidate agent; and (c) detecting whether the candidate agent has an effect on the phenotype. In some embodiments, the effect on the phenotype by the candidate agent is a reduction or a reversal of the phenotype. In some embodiments, the effect on the phenotype by the candidate agent is an enhancement of the phenotype. In some embodiments, the phenotype is associated with a mutation in the one or more cells. In some embodiments, the one or more cells are derived from a patient with a muscular deficiency. In some embodiments, the one or more cells are derived from a patient with muscular dystrophy. In some embodiments, the one or more cells are derived from a patient with Duchenne muscular dystrophy. In some embodiments, the one or more cells are derived from a patient with a muscular deficiency that is caused by cachexia or sarcopenia. In some embodiments, the one or more pluripotent stem cells have been genetically modified to comprise a mutation that causes, or is associated with, a genetic disease or disorder.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
**FIG. 1** is an overview depicting methods of generating and using satellite cells or satellite-like cells in accordance with embodiments of the present disclosure;
**FIG. 2** is an overview of an approach to the differentiation process and uses of HLA-null satellite cells or satellite-like cells in accordance with embodiments of the present disclosure;
**FIG. 3** is an illustration of four stages of differentiation from pluripotent stem cells to myotubes in accordance with embodiments of the present disclosure;
**FIG. 4** is an illustration of pluripotent stem cells differentiating to satellite cells or satellite-like cells in a one-step process in accordance with embodiments of the present disclosure;
**FIG. 5** is an illustration of a comparison of serum components within a differentiation media for differentiation of a human embryonic stem cell line (GEN002) in accordance with embodiments of the present disclosure;
**FIG. 6** is an illustration of a comparison of serum components within a differentiation media for differentiation of a human embryonic stem cell line (GEN019) in accordance with embodiments of the present disclosure;
**FIG. 7** is an illustration of a comparison of serum components within a differentiation media for differentiation of a human embryonic stem cell line (GEN020) in accordance with embodiments of the present disclosure;
**FIG. 8** is an illustration of a comparison of cell counts in serum components within various differentiation media across the GEN002, GEN019, and GEN020 human embryonic stem cell lines in accordance with embodiments of the present disclosure;
**FIG. 9** is an illustration of cells presenting satellite cell-like markers in various differentiation media in accordance with embodiments of the present disclosure;
**FIG. 10** is an illustration of the propagation of satellite cells or satellite-like cells over three passages, in accordance with embodiments of the present disclosure;
**FIG. 11A** is an illustration of a comparison of cell counts of cells grown in various differentiation media in accordance with embodiments of the present disclosure. **FIG. 11B** is an illustration of a comparison of Pax3 expression of cells grown in various differentiation media in accordance with embodiments of the present disclosure. **FIG. 11C** is an illustration of a comparison of Pax7 expression of cells grown in various differentiation media in accordance with embodiments of the present disclosure;
**FIG. 12A** is an illustration of a comparison of Pax7 expression in cells grown on various extracellular matrix molecules in accordance with embodiments of the present disclosure. **FIG. 12B** is an illustration of a comparison of cell counts of cells grown on various extracellular matrix molecules in accordance with embodiments of the present disclosure;
**FIG. 13A** is an illustration of a comparison of cell counts of cells grown with or without the LRRK2 inhibitor, LRRK2-IN-1, in accordance with embodiments of the present disclosure. **FIG. 13B** is an illustration of a comparison of Pax3 expression in cells grown with or without the LRRK2 inhibitor, LRRK2-IN-1, in accordance with embodiments of the present disclosure;
**FIG. 14** is an illustration of a comparison of human embryonic cell lines (GEN019 and GEN067) grown in differentiation media with or without the LRRK2 inhibitor, LRRK2-IN-1, in accordance with embodiments of the present disclosure;

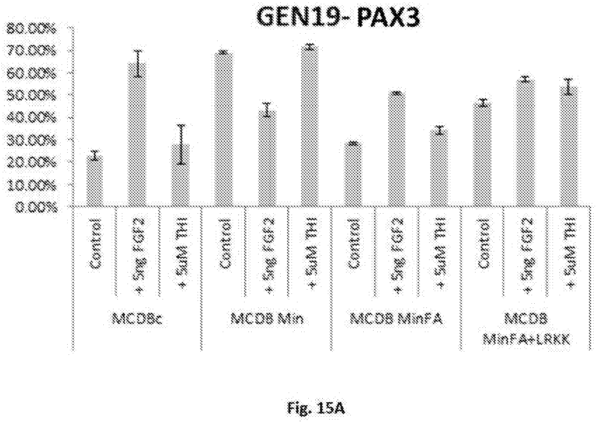

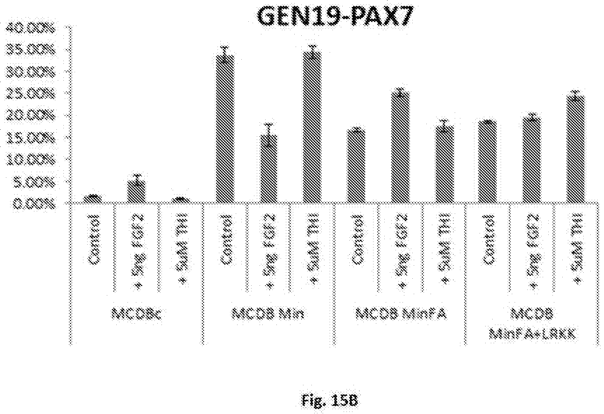

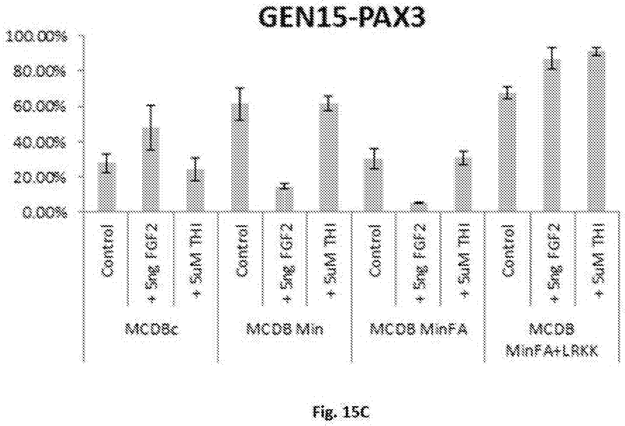

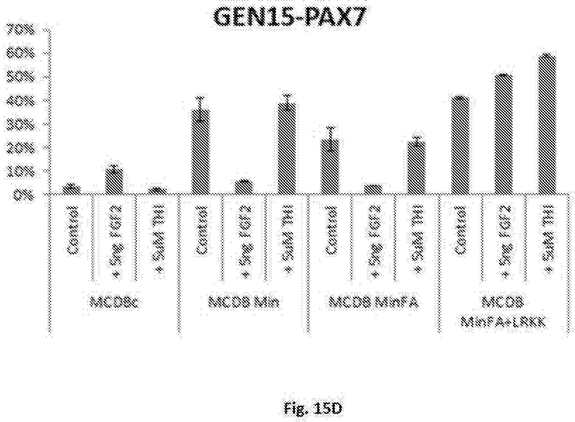

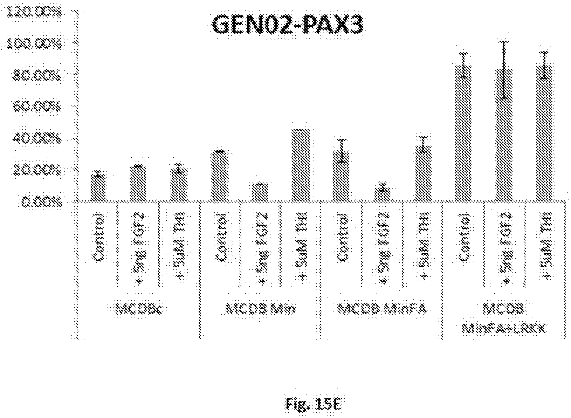

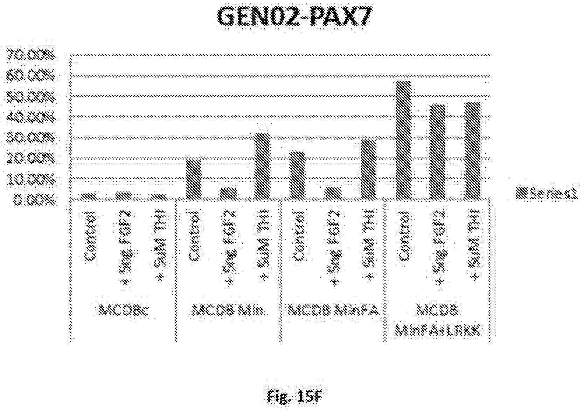

**FIG. 15A** is an illustration of a comparison of Pax3 expression levels in a human embryonic stem cell line (GEN019) grown in various differentiation media, in accordance with embodiments of the present disclosure. **FIG. 15B** is an illustration of a comparison of Pax7 expression levels in a human embryonic stem cell line (GEN019) grown in various differentiation media, in accordance with embodiments of the present disclosure. **FIG. 15C** is an illustration of a comparison of Pax3 expression levels in a human embryonic stem cell line (GEN015) grown in various differentiation media, in accordance with embodiments of the present disclosure. **FIG. 15D** is an illustration of a comparison of Pax7 expression levels in a human embryonic stem cell line (GEN015) grown in various differentiation media, in accordance with embodiments of the present disclosure. **FIG. 15E** is an illustration of a comparison of Pax3 expression levels in a human embryonic stem cell line (GEN02) grown in various differentiation media, in accordance with embodiments of the present disclosure. **FIG. 15F** is an illustration of a comparison of Pax7 expression levels in a human embryonic stem cell line (GEN02) grown in various differentiation media, in accordance with embodiments of the present disclosure;
**FIG. 16A** is an illustration of a comparison of Pax7 expression levels in a human embryonic stem cell line (GEN019) grown in the presence of various kinase inhibitors, in accordance with embodiments of the present disclosure. **FIG. 16B** is an illustration of a comparison of Pax7 expression levels in a human embryonic stem cell line (GEN015) grown in the presence of various kinase inhibitors, in accordance with embodiments of the present disclosure;
**FIG. 17A** is an illustration of a comparison of cell numbers (left panel) and the number of Pax3-positive cells (right panel) of a human embryonic stem cell line (GEN019) grown in various differentiation media, in accordance with embodiments of the present disclosure. **FIG. 17B** is an illustration of a comparison of the percentage of Pax3-positive cells (left panel) and the expression levels of Pax3 (right panel) in a human embryonic stem cell line (GEN019) grown in various differentiation media, in accordance with embodiments of the present disclosure;
**FIG. 18** is an illustration of a comparison of cell counts of human embryonic stem cell lines (GEN019 and GEN067) grown with or without a ROCK inhibitor, in accordance with embodiments of the present disclosure;
**FIG. 19** is an illustration of *in vitro* myotube formation of satellite-like cells cocultured with a mouse myoblast cell line (C2C12), in accordance with embodiments of the present disclosure;
**FIG. 20** is an illustration of an example of a region of interest on HLA class I from a human embryonic stem cell line (GEN015), in accordance with embodiments of the present disclosure; and
**FIG. 21** is an illustration of an example of a region of HLA-A exon 6 of a human embryonic stem cell line (GEN015) that is targeted by a guide RNA (gRNA) designed in accordance with embodiments of the present disclosure.

### DETAILED DESCRIPTION OF THE INVENTION

### I. Overview

### A. General

The present disclosure features unique methods for differentiating pluripotent stem cells into satellite-like cells, which are muscle-lineage cells that resemble satellite cells and may have the potential to give rise to myoblasts and other muscle cells and muscle fibers. The methods generally involve contacting the pluripotent stem cells with one or more compounds that mediate the differentiation of the cells into satellite-like cells. The methods often involve a one-step process and therefore tend to be highly efficient. In some instances, the one-step process involves contacting human pluripotent stem cells with a differentiation medium that includes two compounds (e.g., a Wnt pathway activator and a TGF- β receptor inhibitor), which launches the pluripotent stem cells along a muscle-differentiation pathway and results in the production of satellite-like cells, often through regulation of genes associated with satellite cells such as Pax3, Pax7, and/or CD56. The methods may also be highly efficient in the sense that they may produce high yields of the satellite-like cells, particularly when compared to the number of starting pluripotent stem cells.

Clinically, the satellite-like cells may be extremely useful in a number of settings, including the treatment of patients such as patients with muscular degenerative diseases or disorders stemming from a variety of causes, including but not limited to genetic disorders, sporadic diseases, cachexia, muscle strain, muscle injury, muscle atrophy, as well as sarcopenia and the general aging process. The satellite-like cells disclosed herein may be used in cell therapies for such patients, particularly therapies to replenish or supplement a patient's naturally occurring satellite cells. In such cell therapies, the satellite-like cells may be injected into a site in the patient such as a muscle site, and they may go on to form later-stage muscle cells (e.g., myoblasts) within the patient. The myoblasts may fuse - either with each other or with the patient's naturally-occurring myoblasts - and form myotubes and functioning skeletal muscle tissue within the patient's body. As a result, the patient may experience improvements in muscle tone or function, including improved muscle strength. In some instances, subjects seeking to strengthen muscle tone or function for cosmetic, athletic, or other purposes may benefit from the methods and compositions disclosed in this disclosure.

In some instances, the methods may involve treating subjects with satellite-like cells that are derived from genetically-modified cells (or even satellite-like cells that are directly genetically-modified). For example, a differentiated cell can be isolated from a subject with a genetic disease (e.g., Huntington's disease, Spinal Muscular Atrophy, Duchenne muscular dystrophy, etc.). The differentiated cell may then be subjected to conditions to become a pluripotent stem cell (e.g., to become an induced pluripotent stem cell). The pluripotent stem cell may be genetically modified or altered in order to rescue or improve the disease condition. These genetically modified pluripotent cells may then be differentiated to satellite cells or satellite-like cells, which are transplanted into the subject to reduce the effects of a disease or disorder. These cells may be less likely to invoke an immune response in the subject than cells derived from a different subject.

In another example, satellite cells or satellite-like cells may be differentiated from a human pluripotent stem cell that is modified to disable a Human Leukocyte Antigen (HLA) encoding region(s) so as to produce an HLA-null human pluripotent stem cell. HLA-null human pluripotent stem cells may then be differentiated into HLA-null satellite cells or satellite-like cells, which can be used in cell therapies. The cells may be particularly beneficial as they may be able to completely or partially evade an immune response by the subject.

The satellite-like cells disclosed herein (including cells derived from genetically-modified or unmodified pluripotent stem cells) can also be used in drug-screening assays, particularly assays to identify agents for ameliorating a muscle defect. The cells may also be useful for disease modeling and other types of disease research. In some instances, satellite cells or satellite-like cells may be differentiated from a human pluripotent stem cell that is genetically modified to have an identical or substantially similar mutation that causes a genetic disease in humans. Such satellite cells or satellite-like cells may then be screened for agents that reverse or reduce the effects of the mutation. These cells can be further differentiated into myoblasts and myotubes in order to study the disease or disorder or to perform drug screens.

### B. General Methods

This disclosure provides methods and compositions for producing, culturing and storing satellite cells or satellite-like cells that have the ability to give rise to other satellite cells or satellite-like cells or to differentiate into skeletal muscle cells to form functional skeletal muscle. The disclosure further describes cells differentiated from the satellite cells or satellite-like cells (e.g., myoblasts, myotubes), their preparation, and their storage.

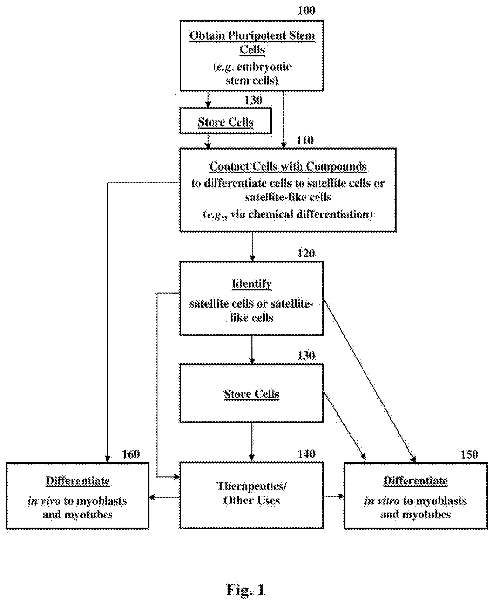

A general overview of a differentiation process is shown in **FIG. 1**. The steps may involve, in any order: obtaining pluripotent stem cells, e.g. embryonic stem cells or induced pluripotent stem cells (100). In some cases, the induced pluripotent stem cells are obtained from differentiated cells from a patient. In some cases, the pluripotent stem cells (e.g., embryonic stem cells or induced pluripotent stem cells) are genetically modified. The steps may also involve contacting the pluripotent stem cells with one or more compounds to differentiate the cells *(e.g.,* by chemical differentiation) to satellite cells or satellite-like cells (110); identifying satellite cells or satellite-like cells (120); and optionally storing the cells (130). Interspersed amongst these steps may be steps to maintain the cells, including culturing or expanding the cells. In addition, storage of the cells can occur after many steps in the process. Further, **FIG. 1** may optionally include a purification step after identifying satellite cells or satellite-like cells (120). In some cases, the methods disclosed herein comprise one or more purification steps, such as a step involving sorting cells with a particular phenotype from the rest of the population of cells. In some cases, the methods disclosed herein do not comprise a purification step. In some cases, the methods do not comprise sorting the cells.

In some cases, the satellite cells or satellite-like cells are subjected to conditions that enable them to generate further differentiated cells in the muscle lineage, e.g., myoblasts or myotubes. In particular, the satellite cells or satellite-like cells may be differentiated *in vitro* to myoblasts and myotubes (150). In other cases, the satellite cells or satellite-like cells may be differentiated *in vivo.*

The satellite-like cells can be used in many contexts, such as therapeutic or other uses (140). Examples of these uses include cell therapies, developmental studies, disease modelling and drug screening campaigns. In some examples of cell therapies, satellite cells or satellite-like cells may be transplanted into a subject (e.g., a patient) and then may differentiate *in vivo* into other muscle lineage cells such as myoblasts and myotubes (160). The transplanted cells may also multiply *in vivo* to produce additional satellite cells or satellite-like cells. The transplanted cells (or cells derived therefrom, such as myoblasts) may fuse *in vivo* with host myoblasts to form hybrid myotubes comprising nuclei from both transplanted cells (or transplant-derived cells) and host cells. In some cases, the transplanted satellite cells or satellite-like cell (or cells derived therefrom) may promote muscle growth in other ways, such as by secreting factors or providing mechanical support. In some cases, the transplanted cells or factors secreted therefrom may protect muscles by mitigating an inflammatory response. In some cases, the transplanted cells are cells produced from cells obtained from a different subject. In some cases, a subject receives transplanted cells that are derived from a cell sample originally obtained from the subject. In some cases, the cell sample obtained from the subject comprises differentiated cells (e.g., fibroblasts, blood cells) that are induced to form induced pluripotent stem cells. The induced pluripotent stem cells may be genetically modified, for example, to correct a phenotype.

In some cases, the satellite cells or satellite-like cells are differentiated into myoblasts and/or myotubes *in vitro.* The myoblasts and/or myotubes may then be used as a cell therapy (e.g., by introducing the cells into a subject), as a platform for a drug screening assay, or for other purposes.

In another example, satellite cells or satellite-like cells may be differentiated from a human pluripotent stem cell that is modified to disable a Human Leukocyte Antigen (HLA) encoding region(s) so as to produce an HLA-null human pluripotent stem cell. HLA-null human pluripotent stem cells may then be differentiated into HLA-null satellite cells or satellite-like cells. The HLA-null satellite cells or satellite-like cells may be produced from a less-differentiated cell (e.g., pluripotent stem cell, multipotent stem cell, muscle lineage restricted progenitor cell) by chemical differentiation, by forced expression of genetic markers that are associated with satellite cells or satellite-like cells, or by any other differentiation process known in the art. Forced expression can be achieved by introducing expression vectors encoding the proteins into the cell, transduction of cells with recombinant viruses, introduction of exogenous purified polypeptides of interest into cells, introduction of exogenous purified mRNAs encoding polypeptides of interest into cells, contacting cells with a non-naturally occurring reagent that induces expression of a marker of interest (e.g., Pax3, Pax7, or CD56), or any other biological, chemical, or physical process to induce expression of a gene encoding a polypeptide of interest.

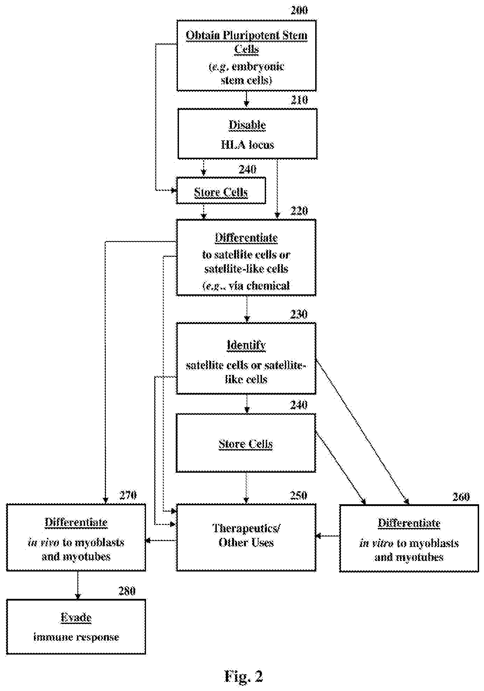

**FIG. 2** illustrates some basic steps of differentiating HLA-null pluripotent stem cells to HLA-null satellite cells or satellite-like cells. These steps may involve: obtaining pluripotent stem cells, e.g. embryonic stem cells or induced pluripotent stem cells (200); disabling at least one HLA locus (210); differentiating the cells, *e.g.*, by chemical differentiation to cells that express polypeptides such as Pax3, Pax7, or CD56 (220); identifying satellite cells or satellite-like cells (230); and optionally storing the cells (240). Interspersed amongst these steps may be steps to maintain the cells, including culturing or expanding the cells. In addition, storage of the cells can occur after many steps in the process. Further, **FIG. 2** may optionally include a purification step after identifying satellite cells or satellite-like cells (230). In some cases, the methods disclosed herein comprise one or more purification steps, such as a step involving sorting cells with a particular phenotype from the rest of the population of cells. In some cases, the methods disclosed herein do not comprise a purification step; in some cases, the methods do not comprise sorting the cells. In some cases, the HLA-null satellite cells or satellite-like cells are subjected to conditions that enable them to generate further differentiated cells in the muscle lineage, e.g., myoblasts or myotubes. In particular, the HLA-null satellite cells or satellite-like cells may be differentiated *in vitro* to form myoblasts and myotubes (260). In some instances, the HLA-null satellite cells or satellite-like cells may be transplanted into a subject (e.g., a patient) and then may differentiate *in vivo* into the muscle lineage cells such as myoblasts and myotubes (270). The transplanted cells may also multiply *in vivo* to produce additional satellite cells or satellite-like cells. Additionally, the transplanted HLA-null satellite cells or satellite-like cells may evade an immune response (280) from the subject. The transplanted HLA-null satellite cells or satellite-like cells may also evade a natural killer cell response by displaying host HLA antigens after fusing with host myoblasts in vivo. In some cases, the satellite cells or satellite-like cells are differentiated in myoblasts and/or myotubes *in vitro.* The myoblasts and/or myotubes may then be used as a cell therapy (e.g., by introducing the cells into a subject), as a platform for a drug screening assay, or for other purposes.

In other examples, satellite cells or satellite-like cells may be differentiated from disease-specific pluripotent stem cells such as an embryonic stem cell identified as carrying a mutation associated with a genetic disease or disorder or an induced pluripotent stem that is either (a) obtained from a subject with a genetic mutation or (b) genetically-altered to carry a genetic mutation. These disease-specific stem cells may then be differentiated into disease-specific satellite cells or satellite-like cells. Disease-specific satellite cells or satellite-like cells may be used for drug screening and other clinical applications.

### II. Preparation of Cells

### A. Pluripotent stem cells, multipotent stem cells, and other cells capable of differentiation into satellite cells or satellite-like cells

In some cases, satellite cells or satellite-like cells are produced by the differentiation of pluripotent stem cells, multipotent stem cells or lineage-committed cells (such as muscle-lineage committed). Pluripotent stem cells generally have the ability to differentiate into cells of all three germ layers (i.e., ectoderm, mesoderm, and endoderm), whereas multipotent stem cells can develop into more than one cell type, but are more limited than pluripotent stem cells. There are various sources for pluripotent stem cells, but generally pluripotent stem cells are derived from embryonic stem cells or induced pluripotent stem cells. Multipotent stem cells can be derived from various sources, including neo-natal cord blood or cells isolated from post-natal tissues. In an example, methods disclosed herein may be used to differentiate mesenchymal multipotent stem cells or muscle-lineage restricted progenitor cells to satellite cells.

Examples of cells that may be differentiated to satellite cells or satellite-like cells are preferably from a human subject but can also be derived from non-human subjects, e.g., non-human mammals. Examples of non-human mammals include, but are not limited to, non-human primates *(e.g.,* apes, monkeys, gorillas), rodents *(e.g.,* mice, rats), cows, pigs, sheep, horses, dogs, cats, or rabbits.

Embryonic stem cells (ESCs) may be isolated from the inner cell mass of a blastocyst about four-to-five days post-fertilization and are characterized by both pluripotency and self-renewal. As such, ESCs can be propagated indefinitely in an undifferentiated state. ESCs can also be obtained from previously isolated cells that have been propagated in culture for an indefinite period of time. ESCs can be obtained from blastocysts that are genotypically male or female. ESC's may be obtained from unfertilized eggs using parthenogenesis.

The ESCs may be collected from subjects with a variety of disease statuses. The cells can be collected from an embryo that is free of an adverse health condition. In other cases, the embryo may be identified by preimplantation genetic diagnosis (PGD) to have an elevated risk of developing a disease or disorder, e.g., a muscular degenerative disease such as muscular dystrophy, Huntington's disease, Merosin deficiency 1A, nemaline myopathy, and Spinal Muscular Atrophy (SMA). Examples of muscular dystrophy include Becker, congenital, facioscapulohumeral (FSH), myotonic (type I and II), oculopharyngeal, distal, myotonic muscular dystrophy, Duchenne muscular dystrophy, Limb-girdle muscular dystrophy, and Emery-Dreifuss muscular dystrophy. Duchenne and Becker muscular dystrophies are caused by a mutation of a gene located on the X chromosome and predominantly affect males, although females can sometimes have severe symptoms as well. Additionally, most types of muscular dystrophy are multi-system disorders with manifestations in body systems including the heart, gastrointestinal system, nervous system, endocrine glands, eyes and brain.

Induced pluripotent stem cells (iPSCs) may be induced from a variety of cell types. Examples of suitable populations of cells include, but are not limited to, fibroblasts, bone-marrow derived mononuclear cells, skeletal muscle cells, adipose cells, peripheral blood mononuclear cells, blood cells, peripheral blood lymphocytes, macrophages, keratinocytes, oral keratinocytes, hair follicle dermal cells, gastric epithelial cells, lung epithelial cells, synovial cells, kidney cells, skin epithelial cells, or osteoblasts.

Induced pluripotent stem cells can also originate from many different types of tissue, e.g., bone marrow, skin (e.g., dermis, epidermis), muscle, adipose tissue, peripheral blood, foreskin, skeletal muscle, or smooth muscle. The cells can also be derived from neonatal tissue, including, but not limited to: umbilical cord tissues (e.g., the umbilical cord, cord blood, cord blood vessels), the amnion, the placenta, or other various neonatal tissues (e.g., bone marrow fluid, muscle, adipose tissue, peripheral blood, skin, skeletal muscle etc.).

The induced pluripotent stem cells, multipotent cells, or lineage-committed cells can be derived from a subject that is male or female. The cells can be derived from neonatal or post-natal tissue collected from a subject within the period from birth, including cesarean birth, to death. In some cases, the induced pluripotent stem cells are derived from an aging subject including a subject experiencing premature aging. In some cases, the tissue may be from a subject who is > 10 minutes old, > 1 hour old, > 1 day old, > 1 month old, > 2 months old, > 6 months old, > 1 year old, >2 years old, >5 years old, >10 years old, >15 years old, >18 years old, >25 years old, >35 years old, >45 years old, >55 years old, >60 years old, >65 years old, >70 years old, > 75 years old, >80 years old, <80 years old, <70 years old, <60 years old, <50 years old, <40 years old, <30 years old, <20 years old or <10 years old. The subject may be a neonatal infant. In some cases, the subject is a child or an adult. In some examples, the tissue is from a human of age 2, 5, 10 or 20 hours. In other examples, the tissue is from a human of age 1 month, 2 months, 3 months, 4 months, 5 months, 6 months, 9 months or 12 months. In some cases, the tissue is from a human of age 1 year, 2 years, 3 years, 4 years, 5 years, 18 years, 20 years, 21 years, 23 years, 24 years, 25 years, 28 years, 29 years, 31 years, 33 years, 34 years, 35 years, 37 years, 38 years, 40 years, 41 years, 42 years, 43 years, 44 years, 47 years, 51 years, 55 years, 61 years, 63 years, 65 years, 70 years, 77 years, or 85 years old.

The iPSCs, multipotent cells, or lineage-committed cells may be collected from, or derived from, subjects with a variety of disease statuses, including any of the subjects or patients described herein. The cells can be collected from a subject who is free of an adverse health condition. In other cases, the subject has, or is at risk of having, a disease or disorder. In some cases, the subject has, or is at risk of having, a muscular degenerative disease or disorder such as a muscular deficiency disease described herein (e.g., muscular dystrophy such as Duchenne muscular dystrophy) or muscle wasting or atrophy. In some cases, the subject has, or is at risk of having, a genetic disease or disorder; in such cases, the methods disclosed herein may be used to treat or ameliorate the disease or disorder. The subject may also have other diseases or disorders, e.g., a chronic health condition such as cardiovascular disease, eye disease (e.g., macular degeneration), auditory disease, (e.g., deafness), diabetes, cognitive impairment, schizophrenia, depression, bipolar disorder, dementia, neurodegenerative disease, Alzheimer's Disease, Parkinson's Disease, multiple sclerosis, osteoporosis, liver disease, kidney disease, autoimmune disease, arthritis, or a proliferative disorder (e.g., a cancer). In certain cases, a subject provides cells for his or her future use (e.g., an autologous therapy), or for the use of another subject who may need cells for use, such as for treatment or therapy (e.g., an allogeneic therapy). In some cases, the donor and the recipient are immunohistologically compatible or HLA-matched.

The iPSCs can be obtained by modulating differentiated cells back to pluripotency. Additionally, the iPSCs can be obtained from a sample comprising a single cell or a population of cells. The population may be homogeneous or heterogeneous. The cells may be a population of cells found in a human cellular sample, e.g., a biopsy or blood sample. Often, the cells are somatic cells. The cells may be a cell line. In some cases, the cells are derived from cells fused to other cells. In some cases, the cells are not derived from cells fused to other cells. In some cases, the cells are not derived from cells artificially fused to other cells.

Production of iPSCs can be achieved by forcing the expression of polypeptides, particularly proteins that play a role in maintaining or regulating self-renewal and/or pluripotency of embryonic stem cells. Examples of such proteins are Oct3/4, Sox2, Klf4, L-Myc, N-myc and c-Myc transcription factors, all of which are highly expressed in embryonic stem cells. Additionally, in some examples iPSC cells are prepared without using c-Myc, N-myc, or L-myc or a protein that will cause cancer. Forced expression may include introducing expression vectors encoding polypeptides of interest into cells, transduction of cells with recombinant viruses, introducing exogenous purified polypeptides of interest into cells, introducing messenger RNAs encoding polypeptides of interest into the cells, contacting cells with a non-naturally occurring reagent that induces expression of an endogenous gene encoding a polypeptide of interest (e.g., Oct3/4, Sox2, Klf4, or c-Myc), or any other biological, chemical, or physical means to induce expression of a gene encoding a polypeptide of interest (e.g., an endogenous gene Oct3/4, Sox2, Klf4, or c-Myc). In additional examples, iPSCs may be produced using microRNA (miRNA) methods or gene knockdown methods that induce pluripotency. Production of iPSCs may also be achieved by other methods that result in the expression of markers of pluripotency and the capacity to form differentiated cells such as mesoderm.

The induced pluripotent stem cells, multipotent cells, or lineage-committed cells may be from non-embryonic tissue, *e.g.*, at a stage of development later than the embryonic stage. In other cases, the cells may be derived from an embryo. In some cases, the cells may be from tissue at a stage of development later than the fetal stage. In other cases, the cells may be derived from a fetus.

The pluripotent stem cells (e.g., ESCs, induced pluripotent stem cells), multipotent cells, or lineage-committed cells are preferably from a human subject but can also be derived from non-human subjects, e.g., non-human mammals Examples of non-human mammals include, but are not limited to, non-human primates *(e.g.,* apes, monkeys, gorillas), rodents *(e.g.*, mice, rats), cows, pigs, sheep, horses, dogs, cats, or rabbits.

The cellular population may include both differentiated and undifferentiated cells. In some cases, the population primarily contains differentiated cells. In other cases, the population primarily contains undifferentiated cells, e.g., undifferentiated stem cells. In some examples, the undifferentiated cells within the population may be induced to become pluripotent or multipotent. In some cases, differentiated cells within the cellular population are induced to become pluripotent or multipotent.

In further examples, the cellular population may include undifferentiated stem cells or naive stem cells. In some cases, the undifferentiated stem cells are stem cells that have not undergone epigenetic inactivating modification by heterochromatin formation due to DNA methylation or histone modification of at least four genes, at least three genes, at least two genes, at least one gene, or none of the following: Nanog, Oct3/4, Sox2 and Tert. Activation, or expression of such genes, e.g., Tert, Nanog, Oct3/4 or Sox2, may occur when human pluripotent stem cells are induced from undifferentiated stem cells present in a human postnatal tissue.

Morphological characteristics for identifying candidate multipotent or pluripotent stem cell colonies include, but are not limited to, a rounder, smaller cell size relative to surrounding cells and a high nucleus-to-cytoplasm ratio. The size of the candidate induced cell may be from about 5 µm to about 10 µm; from about 5 µm to about 15 µm; from about 5µm to about 30 µm; from about 10 µm to about 30 µm; or from about 20 µm to about 30 µm. In some examples, a high nucleus-to-cytoplasm ratio may be from about 1.5:1 to about 10:1, *e.g.*, about 1.5:1; about 2:1; about 3:1; about 4:1; about 5:1; about 7:1; about 8:1; about 9.5:1; or about 10:1. In some cases, the induced cell clones display a flattened morphology relative to ES cells. For example, candidate induced cells derived from peripheral blood cells or from cells cultured in feeder-free media may exhibit a flattened morphology compared to surrounding cells. Another morphological characteristic for identifying induced cell clones is the formation of small monolayer colonies within the space between parental cells (e.g., between fibroblasts).

Gene expression characteristics for identifying candidate multipotent or pluripotent stem cells include, but are not limited to, pluripotency marker expression *(e.g*., Oct4, Nanog, SSEA-4, Tral-60), characterization of global gene expression profiles *(e*.*g.,* Pluritest, Muller *et al*., 2011), and expression of additional naive hESC markers. As induced pluripotent stem cells, like embryonic stem cells, are also capable of self-renewal, such cells can also be obtained from previously isolated cells that have been propagated in culture for an indefinite period of time.

### B. Genetic modification of pluripotent or multipotent stem cells by CRISPR enzymes

Pluripotent stem cells (e.g., ESCs, iPSCs) or multipotent stem cells can be genetically modified, and the cells produced thereby can be isolated, cultured, and used to produce differentiated cells with a desired genotype. Such modifications can include altering a genomic locus of a pluripotent stem cell (or other cell type) by contacting the pluripotent stem cell with: (a) a nuclease enzyme that interacts with RNA molecules (e.g., clustered regularly interspaced short palindromic repeats sequence enzyme (CRISPR); and (b) RNA molecules. Such RNA molecules can comprise a CRISPR-RNA (crRNA) and a trans-activating crRNA (tracrRNA), or a chimeric cr/tracrRNA (referred to as a "guide RNA," or "gRNA"). The CRISPR enzyme generates nicks or double-strand breaks at sites in the genome that are identical or highly identical to regions of these RNA molecules. Examples of CRISPR enzymes include, but are not limited to, Cas3, Cas8a, Cas8b, Cas10d, Cas9, Csn2, Csn4, or Cas10. In a particularly preferred embodiment, the CRISPR enzyme is Cas9, which generates double-strand breaks.

CRISPR enzymes can induce mutations in the genome due to the imperfect repair of the double-strand breaks. Non-homologous end joining (NHEJ), in which the two ends are ligated without the need for a homologous template, can lead to insertion/deletion mutations that render the gene product non-functional. The double stranded break can also be resolved by a homology directed repair process, in which the double-strand break induces recombination of sequences near the break site with similar or identical sequences on a second DNA molecule. Homology directed repair processes can be used to integrate sequences into a genome. The integration of sequences into a genome can be used to interrupt a gene of interest. Homology directed repair can be used to induce recombination with a template molecule that results in the truncation or deletion of a gene. For example, if a template molecule is provided that consists of the genomic regions encoding the 5' and 3' untranslated regions of a gene with no intervening sequence, the resulting recombination product would lack any of the gene's coding sequence.

### C. Genetic modification of the HLA system in pluripotent or multipotent stem cells

The HLA system is comprised of genetic loci on chromosome 6 that encode polypeptides that present small polypeptide fragments to immune cells. Class I HLA polypeptides present polypeptides from inside the cell, such as the polypeptides produced by a healthy cell, but also polypeptides derived from exogenous sources, such as viruses, that are within the cell. Class I HLA polypeptides interact with T-cells expressing CD8 and can induce an immune response. Class II HLA polypeptides present short polypeptides derived from sources outside the cell. Some cells that present antigens with the Class II HLA polypeptides include dendritic cells, macrophages, and B-lymphocytes.

The genetic loci that encode the components of the HLA system are highly variable. Each individual has the same allelic combinations in their cells, but these alleles can differ from person to person. A person receiving tissue or organ transplantation from a person with a different combination of HLA alleles is at an increased risk for graft rejection. As such, the HLA locus is among the most commonly typed genetic loci prior to transplantation.

Treatments using stem cells derived from a source other than the subject being treated can be improved by the use of cells with HLA loci that reduce the risk of rejection. Stem cells can be genotyped at their HLA loci and matched with the subject to reduce the risk of transplant rejection. For example, stem cells or differentiated cells derived thereof can be transplanted into a subject (e.g., an autologous subject, HLA-matched subject) to treat a disease or condition.

In a preferred example, the methods disclosed herein comprise producing a cell that does not express a portion of the HLA loci, in order to reduce the risk of transplant rejection.
The HLA locus or loci may be removed or inactivated by any method known in the art, including genetic modification strategies, recombination, and others. In some cases, a pluripotent stem cell can be contacted with a CRISPR enzyme designed to alter genomic loci encoding HLA such that they no longer produce functional HLA, thereby generating an HLA-null pluripotent stem cell. The HLA loci can be rendered non-functional using Cas9 as the CRISPER enzyme.

Cas9 can be introduced with one or more gRNA to induce mutations that render the HLA loci non-functional. Alternatively, Cas9 can be introduced with two or more gRNA to induce deletions of HLA loci. In an example, Cas9, a CRISPR enzyme, may be introduced with one or more guide RNAs and a template DNA molecule with similarity to the genomic loci encoding HLA, or surrounding HLA, in order to induce a recombination event that is sufficient such that the pluripotent stem cell no longer produces functional HLA.

Cas9 can be introduced with one or more gRNA and one or more template nucleic acids to induce recombination events. Such recombination events can result in the deletion of an HLA locus. The recombination events may also result in the disabling of an HLA locus by deleting a portion of the HLA locus such that the HLA locus is no longer able to produce functional HLA polypeptides. The size of the deletions may range in size. In some examples, the deleted region(s) may be small. Examples of small deletions include deletions that generate early stop codons, induce degradation of the transcript, or produce a non-functional polypeptide. In other examples, one or more essential exons can be deleted. In other examples, the entire gene can be deleted.

In further examples, a region containing more than one HLA loci could be deleted. As such, the range of deletions can broadly be > 5 bp, > 10 bp, > 20 bp, > 50 bp, > 100 bp, > 500 bp, > 1 kbp, > 5 kbp, > 10 kbp, > 20 kbp, > 50 kbp, > 100 kbp, > 500 kbp, > 1 Mbp, > 2 Mbp, or > 3 Mbp. Alternatively, such recombination events can result in the integration of a sequence into an HLA locus, rendering it non-functional (HLA-null). Such mutations, deletions, or insertions can be targeted to promoters, transcription factors, or other genomic loci that affect expression or function of HLA loci.

HLA-null pluripotent stem cells can be tested in a 'humanized' mouse model ('Hu-mouse'). Hu-mice show a functional human immune system by the combined transplantation of human fetal thymus tissues and isogenic CD34⁺ fetal liver cells into immunodeficient NOD/SCID mice (Lan, P., Tonomura, N., Shimizu, A., Wang, S., & Yang, Y. (2006). Reconstitution of a functional human immune system in immunodeficient mice through combined human fetal thymus/liver and CD34+ cell transplantation. Blood, 108(2), 487-92.). HLA-null pluripotent stem cells generally have a reduced chance of being rejected by the mice compared to an HLA-pluripotent stem cell. Successful evasion of the immune system can be demonstrated by the detection of HLA-null teratoma.

### D. Genetic modifications to introduce disease-carrying mutations

Mutations can be introduced in healthy stem cell lines that are known to cause a genetic disease. For example, the dystrophin gene or part of the dystrophin gene, or one or more exons may be deleted in order to cause a frame-shift mutation or otherwise render the gene non-functional. Mutations may be heterozygous or homozygous, in male or female stem cell lines. The resulting modified stem cell lines can be differentiated to satellite cells or satellite-like cells and further to myoblasts or myotubes. These satellite cells, satellite-like cells, myoblasts, or myotubes may show disease-associated phenotypes caused by the introduced mutation(s). The genetically, unmodified stem cell line may serve as an isogenic control which may be particularly useful for drug screening, disease modeling, and disease research.

### E. Genetic modifications to rescue disease-causing mutations

Pluripotent stem cells, multipotent stem cells, or other type of stem cell may be genetically modified and then used in the methods disclosed herein. In some cases, stem cells, such as a stem cell line, carrying a genetic mutation or mutations causing a disease or disorder can be genetically modified to correct the mutation and thereby mitigate the disease or disorder experienced by a subject. The genetic modification may be accomplished by any method known in the art.

In some examples, cells (e.g., blood cells, skin cells) are obtained from a subject with a genetic disease or disorder affecting the subject's muscle tissue (e.g., muscular dystrophy, Duchenne muscular dystrophy, spinal muscular atrophy, etc.). The cells may then be subjected to conditions enabling them to become pluripotent stem cells or multipotent stem cells. For example, the cells may undergo de-differentiation and become induced pluripotent stem cells, particularly an induced pluripotent stem cell line. The pluripotent stem cells (or cell line) may be genetically modified to correct the mutation. For example, the subject may have one or more mutations in the dystrophin (DMD) gene and stem cells derived from the subject may be genetically modified to correct such mutations, or a portion of such mutations. The modified pluripotent stem cells may be differentiated into satellite-like, or satellite, cells using the methods described herein. The modified satellite-like cells or satellite cells may then be introduced into the subject with the genetic disease or disorder, in order to treat or ameliorate some aspect of the disorder.

### III. Culture of Pluripotent Stem Cells

After pluripotent stem cells have been generated or obtained, the pluripotent stem cells (e.g., ESCs, iPSCs) can be expanded. For example, the pluripotent stem cells may be cultured and expanded in any suitable medium. Pluripotent stem cells can be cultured in the presence of feeder cells. For example, the cells may be cultured on a layer, or carpet, of murine or human embryonic fibroblasts (e.g., irradiated or mitomycin-treated embryonic fibroblasts). Pluripotent stem cells can be cultured in feeder-free media. Such media may have defined content, wherein purified components are added in known quantities. Such media may have content that is not fully defined, such as media that has been conditioned in the presence of other cells, wherein such cells are removed but polypeptides and other molecules derived from them remain in the media.

Pluripotent stem cells can be plated and cultured directly on tissue culture-grade plastic. Alternatively, cells can be plated and cultured on a coated substrate, e.g., a substrate coated with fibronectin, gelatin, matrigel™ (BD Bioscience), extracellular matrix, collagen, or laminin, as well as combinations thereof Concentrations of the substances used to coat the plates can be about 5 µg /ml, 10 µg/ml, 20 µg/ml, 40 µg/ml, 60 µg/ml, 80 µg/ml, 100 µg/ml, or 200 µg/ml, or 1 mg/ml, or other concentrations as appropriate. In some cases, growing pluripotent stem cells on a substrate coated with extracellular matrix may increase the efficiency of differentiation to satellite-like cell populations. In some cases, untreated petri-dishes may be used. In some cases, the pluripotent stem cells may be grown on a plate coated with one or more of the following substances: collagen, for example, collagen type I, collagen type II, collagen type III, collagen type IV, collagen type V, collagen type VI, collagen type VII, collagen type VIII, collagen type IX, collagen type X, collagen type XI, collagen type XII, collagen type XIII, collagen type XIV, collagen type XV, collagen type XVI, collagen type XVII, collagen type XVIII, collagen type XIX, collagen type XX, collagen type XXI, collagen type XXII, collagen type XXIII, collagen type XXIV, collagen type XXV, collagen type XXVI, collagen type XXVII, or collagen type XXVIII. In some examples, the pluripotent stem cells may be grown on a plate coated with laminins, for example, laminin-111, laminin-211, laminin-121, laminin-221, laminin-332 / laminin-3A32, laminin-3B32, laminin-311 / laminin-3A11, laminin-321 / laminin-3A21, laminin-411, laminin-421, laminin-511, laminin-521, laminin-213, laminin-423, laminin-522, laminin-523, or a combination thereof In some cases, the pluripotent stem cells are grown on plates coated with collagen-I, laminin-111, laminin-211 or laminin-521.

Suitable cell culture vessels include, e.g., 35 mm, 60 mm, 100 mm, and 150 mm cell culture dishes, 6-well, 12-well, 96-well, 384-well, 1536-well cell culture plates, microtiter plates, and other size-equivalent cell culture vessels.

The pluripotent stem cells may be maintained in the presence of a rho, or rho-associated protein kinase (ROCK) inhibitor to reduce apoptosis. A ROCK inhibitor may be particularly useful when the cells are subjected to a harsh treatment, such as an enzymatic treatment. For example, the addition of GSK429286A (Selleckchem; water soluble), Y-27632 (Calbiochem; water soluble) or Fasudil (HA1077: Calbiochem) may be used to culture the pluripotent stem cells of the present disclosure. In some cases the concentration of GSK429286A,Y-27632 or Fasudil, is about 100 nM, 500 nM, 1 µM, 2.5 µM, 5 µM, 10 µM, 15 µM, or 20 µM.

The pluripotent stem cells may be cultured in medium supplemented with a particular serum component. In some embodiments, the serum is fetal bovine serum (FBS), human serum albumin, horse serum, PLT-Max human platelet extract, bovine serum albumin, or knock-out serum replacement. Mixtures of serum components may also be used, e.g. mixtures of FBS and horse serum, knock-out serum replacement and bovine serum albumin, FBS and bovine serum albumin (BSA).

Some representative media that may be used to culture pluripotent stem cells include but are not limited to: Primate ES medium (ReproCELL, Japan), TeSRTM (STEMCELL technologies), StemPro hESC SFMTM, hESF9, MC-ES, Embryonic Stem cell (ES) medium, MEF-conditioned ES (MC-ES), M2 culture medium (manufactured by Genea Biocells), or 10% FBS-supplemented Dulbecco's Modified Eagle Medium (DMEM).

hESF9 may comprise: hESF basal medium supplemented with heparin and four protein components: insulin, transferrin, albumin conjugated with oleic acid, and fibroblast growth factor-2 (FGF-2) (10 ng/ml). Additionally, ES medium may comprise: 40% Dulbecco's Modified Eagle's Medium (DMEM) 40% F12 medium, 2 mM L-glutamine, 1x non-essential amino acids (Sigma, Inc., St. Louis, MO), 20% Knockout Serum Replacement™ (Invitrogen, Inc., Carlsbad, CA), and 10 µg/ml gentamycin.

MC-ES medium may be prepared as follows. ES medium is conditioned on mitomycin C-treated murine embryonic fibroblasts (MEFs), for 20 to 24 hours, harvested, filtered through a 0.45- µM filter, and supplemented with about 0.1 mM B-mercaptoethanol, about 10 ng/ml bFGF or FGF-2, and, optionally, about 10 ng/ml Activin A. In some cases, irradiated MEFs are used in place of the mitomycin C-treated MEFs. In other cases, STO (ATCC) mouse cell line or human fibroblast cells are used in place of the MEFs.

In some cases, the pluripotent stem cells may be plated (or cultured) at a low density, which may be accomplished by splitting the cells from about 1:8 to about 1:3, e.g., about 1:8; about 1:6; about 1:5; about 1:4; or about 1:3. Cells may be plated at a density of from about 10³ cells/cm² to about 10⁴ cells/cm². In some examples, the cells may be plated at a density of from about 1.5 x 10³ cells/cm² to about 10⁴ cells/cm²; from about 2 x 10³ cells/cm² to about 10⁴ cells/cm²; from about 3 x 10³ cells/cm² out 10⁴ cells/cm² from about 4 x 10³ cells/cm² to about 10⁴ cells/cm²; or from about 10³ cells/ cm² to about 9 x 10³ cells/cm². In some embodiments, the cells may be plated at a density greater than 10⁴ cells/cm², e.g., from about 1.25 x 10⁴ cells/cm²
to about 3 x 10⁴ cells/cm².

The pluripotent stem cells may be cultured in a maintenance culture medium in a 37 °C, 5% CO₂ incubator (e.g., under an atmospheric oxygen level). Alternatively, the pluripotent stem cells may be cultured in a maintenance culture medium in a 37 °C, 5% CO₂, 5% O₂ incubator (e.g., under hypoxic conditions) with medium changes preferably every day or every other day. In some embodiments, in order to culture and grow pluripotent stem cells induced from the undifferentiated stem cells, it is preferred that the cells are subcultured every 5 to 7 days in a culture medium containing the additives described herein on a mouse embryonic fibroblast-covered plastic culture dish or a matrigel-coated plastic culture dish. Examples of maintenance culture media for induced cells include any and all complete ES cell media (e.g., MC-ES). The maintenance culture medium may be supplemented with b-FGF or FGF2. In some cases, the maintenance culture medium is supplemented with other factors, e.g., IGF-II, Activin A or other growth factor described herein, see, e.g., Bendall et al., (2007), Nature, 30:448(7157):1015-21. In some embodiments, the induced cells are cultured and observed for about 14 days to about 40 days, e.g., 15, 16, 17, 18, 19, 20, 23, 24, 27, 28, 29, 30, 31, 33, 34, 35, 36, 37, 38 days, or other period from about 14 days to about 40 days, prior to identifying and selecting candidate multipotent or pluripotent stem cell colonies based on morphological characteristics.

In some cases, the pluripotent stem cells may be cultured for about 1 day, 2 days, 3 days, 4.5 days, 5 days, 6.5 days, 7 days, 8 days, 9 days, 10 days, or any other number of days prior to undergoing the differentiation methods described herein. In other cases, the cells may be cultured for more than 12 days, e.g., from about 12 days to about 20 days; from about 12 days to about 30 days; or from about 12 days to about 40 days. In some cases the cells may be cultured indefinitely. In some cases aliquots of the cells can be frozen, and cultures later restarted from the frozen aliquots.

The pluripotent stem cells may be passaged multiple times *(e.g*., more than one time, more than five times, or more than ten times) prior to differentiation. Passaging can be accomplished by placing an aliquot of the cells in fresh medium. Such an aliquot can be a portion of the cells, such as half the cells, a third of the cells, a fourth of the cells, a tenth of the cells, or even a single cell. Such an aliquot can be from a frozen stock of cells. In order to start a culture from a single cell, it is often necessary to dissociate the cells. One means of dissociating the cells is through treatment with a protease *(e.g*., trypsin, chymotrypsin, etc...).

Prior to differentiation, cultured pluripotent stem cells can be dissociated to single-cells. This dissociation can be accomplished by incubating them with a protease, such as trypsin or chymotrypsin. In some cases, prior to differentiation, the cultured pluripotent stem cells are not dissociated into single cells.

In some cases, the pluripotent stem cells may be cultured at a temperature of about 12 °C, 14 °C, 16 °C, 18 °C, 20 °C, 22 °C, 24 °C, 26 °C, 28 °C, 30 °C, 32 °C, 34 °C, 36 °C, 37 °C, 38 °C, 40 °C, 42 °C, or 44 °C. In some cases, the pluripotent stem cells may be cultured at about < 1%, < 2%, < 3%, < 4%, < 5%, < 6%, < 7%, < 8%, < 9%, or < 10% CO₂ and at about < 1%, < 2%, < 3%, < 4%, < 5%, < 6%, < 7%, < 8%, < 9%, < 10%, or < 20% O₂.

### IV. Differentiation Process

### A. Overview

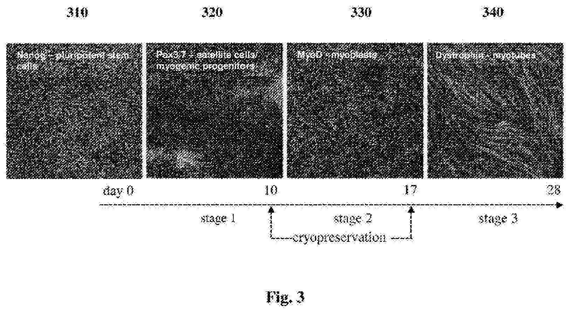

During the differentiation process, a less specialized cell becomes a more specialized cell type. Differentiation may impact aspects of a cell, such as a cell's size, shape, and/or functional capabilities. These changes are largely due to controlled modifications of gene expression. In one example of differentiation, a pluripotent stem cell is differentiated to a satellite cell or satellite-like cell. The differentiated satellite cell or satellite-like cell is then screened for a number of properties that characterize satellite cells or satellite-like cells (e.g., morphological, gene expression). Differentiated satellite cells or satellite-like cells that meet these screening criteria may then be subcloned and expanded. **FIG. 3** is an illustration of four stages of differentiation from pluripotent stem cells to myotubes in accordance with embodiments of the present disclosure. Panel 310 of **FIG. 3** shows pluripotent stem cells expressing a marker of pluripotency, Nanog, detected by immunofluorescent staining. Additionally, panel 320 of FIG. 3 shows a first stage of differentiation, in which pluripotent stem cells have been chemically differentiated to Pax3/Pax7-expressing satellite cells or satellite-like cells. Panel 330 of FIG. 3 shows a second stage of differentiation, in which satellite cells or satellite-like cells have been differentiated to myoblasts, which are immunofluorescently stained for MyoD, a myoblast marker. Further, panel 340 of FIG. 3 shows a third stage of differentiation, in which myoblasts join together to form myotubes, which is detected by immunofluorcent staining of dystrophin, a marker for myotube formation.

### B. Chemical differentiation of pluripotent stem cells into satellite cells or satellite-like cells

In order to differentiate pluripotent stem cells into satellite cells or satellite-like cells, pluripotent stem cells may be obtained from a frozen stock or from a growing culture. These pluripotent stem cells can be cultured in a basal medium in the presence of chemical compounds that induce differentiation to satellite cells or satellite-like cells in a one-step process, which may or may not involve multiple media changes. In some cases, the pluripotent stem cells are cultured in a basal medium in the presence of chemical compounds that induce differentiation to satellite cells or satellite-like cells in a multi-step process, such as a process involving consecutive addition of different chemical compounds. In general, the basal medium with one or more compounds added to it to induce differentiation may be referred to as a "differentiation medium."

### i. Differentiation Medium components

Examples of a differentiation medium that may be used in a chemical differentiation process may include a medium comprising: basal medium, a Wnt activator, and a TGF- β receptor inhibitor. In some cases, the differentiation medium may include a ROCK inhibitor, a serum component, or a combination thereof. In some cases, the differentiation medium may include a LRRK2 inhibitor. Often, a differentiation medium disclosed herein is growth factor free.

The basal medium that is used in examples of the differentiation medium can vary, but generally comprises a nutrient-replete medium. Examples of basal media that may be used are MCDB120, Skeletal Muscle Cell Basal Medium (manufactured by Promocell), SkBM Basal Medium (manufactured by Lonza), SkBM-2 Basal Medium (manufactured by Lonza), Stem Cell Technologies 'APEL Medium' (manufactured by Stem Cell Technologies), or DMEM/F12.

Additionally, a ROCK inhibitor may be present in the differentiation medium. The ROCK inhibitor may reduce apoptosis at low cell densities. In some cases the concentration of the ROCK inhibitor, such as GSK429286A, Y-27632 or Fasudil, is about 100 nM, 500 nM, 1 µM, 2.5 µM, 5 µM, 10 µM, 15 µM 20 µM, 40 µM, 50 µM, 60 µM or more. In some cases, the ROCK inhibitor is continuously present during the differentiation process from pluripotent stem cell to satellite-like cell. In some cases, the ROCK inhibitor is present during a substantial portion of the differentiation process from originating pluripotent stem cell to satellite-like cell (e.g., greater than 1 day, greater than 2 days, greater than 3 days, greater than 4 days, greater than 5 days, greater than 10 days, or greater than 15 days).

The basal medium may additionally comprise a media described, or a media similar to one described, above with additional serum-like components. Such serum-like components can include BSA, fibroblast growth factor (FGF), insulin, fetuin, epidermal growth factor (EGF), horse serum, knock-out replacement serum, dexamethasone, or a combination thereof

BSA may, in some examples, be present at a final concentration of at least about 0.1%, 0.5%, 1%, 2%, 3%, 4%, 5%, 6%, 7%, 8%, 9%, or 10%, or at most about 0.1%, 0.5%, 1%, 2%, 3%, 4%, 5%, 6%, 7%, 8%, 9%, or 10%. In some cases, cells can be contacted with BSA for more than 1 day, more than 2 days, more than 3 days, more than 4 days, more than 5 days, more than 6 days, more than 7 days, more than 8 days, or more than 9 days.

FGF (or other growth factor) may, in some examples, be present at a final concentration of at least about 0.5 ng/ml, 1 ng/ml, 1.5 ng/ml, 2 ng/ml, 2.5 ng/ml, 3 ng/ml, 3.5 ng/ml, 4 ng/ml, 4.5 ng/ml, 5 ng/ml, 6 ng/ml, 7 ng/ml, 8 ng/ml, 9 ng/ml, 10 ng/ml, 15 ng/ml, 20 ng/ml or 25 ng/ml. In some cases, FGF is present in a concentration of at most about 0.5 ng/ml, 1 ng/ml, 1.5 ng/ml, 2 ng/ml, 2.5 ng/ml, 3 ng/ml, 3.5 ng/ml, 4 ng/ml, 4.5 ng/ml, 5 ng/ml, 6 ng/ml, 7 ng/ml, 8 ng/ml, 9 ng/ml, 10 ng/ml, 15 ng/ml, 20 ng/ml or 25 ng/ml. In some cases, cells can be contacted with FGF for more than 1 day, more than 2 days, more than 3 days, more than 4 days, more than 5 days, more than 6 days, more than 7 days, more than 8 days, or more than 9 days.

Insulin may, in some examples, be present at a concentration of at least about 2 µg/ml, 3 µg /ml, 4 µg /ml, 5 µg /ml, 6 µg /ml, 7 µg /ml, 8 µg/ml, 9 µg/ml or 10 µg /ml. In some cases, cells can be contacted with insulin for more than 1 day, more than 2 days, more than 3 days, more than 4 days, more than 5 days, more than 6 days, more than 7 days, more than 8 days, or more than 9 days.

In some cases, the differentiation medium is substantially growth-factor free or absent of any growth factors (e.g., without EGF, FGF, FGF2, insulin, and the like). In some cases, the differentiation medium is substantially xenogeneic-free ("xeno-free") or substantially absent of components derived from non-human organisms. In some cases, the differentiation medium is both growth-factor free and xeno-free.

Fetuin may, in some examples, be present at a final concentration of 10 µg/ml, 20 µg/ml, 30 µg/ml, 40 µg/ml, 50 µg/ml, 60 µg/ml, 70 µg/ml, 80 µg/ml, 90 µg/ml, or 100 µg/ml. EGF can be added to a final concentration of 5 ng/ml, 10 ng/ml, 15 ng/ml, and 20 ng/ml. In some cases, cells can be contacted with fetuin for more than 1 day, more than 2 days, more than 3 days, more than 4 days, more than 5 days, more than 6 days, more than 7 days, more than 8 days, or more than 9 days.

Horse serum may, in some examples, be present at a final concentration of 0.1%, 0.5%, 1%, 2%, 3%, 4%, 5%, 6%, 7%, 8%, 9%, or 10%. In some cases, cells can be contacted with horse serum for more than 1 day, more than 2 days, more than 3 days, more than 4 days, more than 5 days, more than 6 days, more than 7 days, more than 8 days, or more than 9 days.

Knock-out replacement serum may, in some examples, be present at a final concentration of 0.1%, 0.5%, 1%, 2%, 3%, 4%, 5%, 6%, 7%, 8%, 9%, or 10%. In some cases, cells can be contacted with a knock-out replacement serum for more than 1 day, more than 2 days, more than 3 days, more than 4 days, more than 5 days, more than 6 days, more than 7 days, more than 8 days, or more than 9 days.

Dexamethasone may, in some examples, be present at a final concentration of about 0.1 µg/ml and 1 µg/ml, such as 0.1 µg/ml, 0.2 µg/ml, 0.3 µg/ml, 0.4 µg/ml, 0.5 µg/ml, 0.6 µg/ml, 0.7 µg/ml, 0.8 µg/ml, 0.9 µg/ml, or 1 µg/ml. In some cases, cells can be contacted with dexamethasone for more than 1 day, more than 2 days, more than 3 days, more than 4 days, more than 5 days, more than 6 days, more than 7 days, more than 8 days, or more than 9 days.

In addition to basal medium and, optionally, a ROCK inhibitor and/or a serum component, the differentiation medium may include compounds that contribute to differentiation of pluripotent stem cells (or other type of stem cell such as multipotent stem cell) to satellite cells or satellite-like cells. In particular, pluripotent stem cells (or other type of stem cells) that are exposed to a Wnt pathway activator as well as a TGF- β receptor inhibitor (singly or in combination) may differentiate into satellite cells or satellite-like cells. Additionally, the satellite cells or satellite-like cells that are produced using this method may be capable of forming myoblasts.

In some cases, a compound that is present in a differentiation medium for differentiation of pluripotent stem cells to satellite cells or satellite-like cells is a Wnt pathway activator. Such activators can include CHIR99021, QS11, IQ1, valproic acid (VPA), or deoxycholic acid (DCA). In particular, the use of a Wnt pathway activator may act as a GSK inhibitor, (e.g., GSK3- β inhibitor).

The Wnt pathway activator CHIR99021 may, in some examples, be present in the differentiation medium in concentrations of about 0.01 µM, 0.05µM, 0.1 µM, 0.2 µM 0.5 µM, 0.7 µM, 1 µM, 1.5 µM, 2 µM, 2.5 µM, 3 µM, 4 µM, 5 µM, 6 µM, 7 µM, 8 µM, 9 µM, 10 µM, 11 µM, 12 µM, 12.5 µM, 13 µM, 14 µM, 15 µM, 16 µM, 17 µM, 18 µM, 19 µN, 20 µM, 25 µM, 30 µM, 35 µM, 40 µM, or 50 µM, or more. In some cases, cells can be contacted with the Wnt pathway activator, CHIR99021, for more than 1 day, more than 2 days, more than 3 days, more than 4 days, more than 5 days, more than 6 days, more than 7 days, more than 8 days, or more than 9 days.

The Wnt pathway activator AZD1080 may, in some examples, be present in the differentiation medium in concentrations of about 0.01 µM, 0.05 µM, 0.1 µM, 0.2 µM 0.5 µM, 0.7 µM, 1 µM, 1.5 µM, 2 µM, 2.5 µM, 3 µM, 4 µM, 5 µM, 6 µM, 7 µM, 8 µM, 9 µM, 10 µM, 11 µM, 12 µM, 12.5µM, 13 µM, 14 µM, 15 µM, 16 µM, 17 µM, 18 µM, 19 µM, 20 µM, 25 µM, 30 µM, 35 µM, 40 µM, or 50 µM, or more. In some instances, cells can be contacted with AZD1080 for more than 1 day, more than 2 days, more than 3 days, more than 4 days, more than 5 days, more than 6 days, more than 7 days, more than 8 days, or more than 9 days.

The Wnt pathway activator QS11 may, in some examples, be present in the differentiation medium in concentrations of about 0.01 µM, 0.05 µM, 0.1 µM, 0.2 µM, 0.5 µM, 0.7 µM, 1 µM, 1.5 µM, 2 µM, 2.5 µM, 3 µM, 4 µM, 5 µM, 6 µM, 7 µM, 8 µM, 9 µM, 10 µM, 11 µM, 12 µM, 12.5 µM, 13 µM, 14 µM, 15 µM, 16 µM, 17 µM, 18 µM, 19 µM, 20 µM, 25 µM, 30 µM, 35 µM, 40 µM, or 50 µM, or more. In some cases, cells can be contacted with QS11 for more than 1 day, more than 2 days, more than 3 days, more than 4 days, more than 5 days, more than 6 days, more than 7 days, more than 8 days, or more than 9 days.

The Wnt pathway activator IQ1 may, in some examples, be present in the differentiation medium in concentrations ranging of about 1 µg/ml, 2 µg/ml, 3 µg/ml, 4 µg/ml, 5 µg/ml, 6 µg/ml, 7 µg/ml, 8 µg/ml, 9 µg/ml, 10 µg/ml, 11 µg/ml, 12 µg/ml, 13 µg/ml, 14 µg/ml, 15 µg/ml, 16 µg/ml, 17 µg/ml, 18 µg/ml, 19 µg/ml, or 20 µg/ml, or more. In some cases, cells can be contacted with IQ1 for more than 1 day, more than 2 days, more than 3 days, more than 4 days, more than 5 days, more than 6 days, more than 7 days, more than 8 days, or more than 9 days.

VPA may, in some examples, be present in the differentiation medium in concentrations of 0.005 µM, 0.01 µM, 0.05 µM, 0.1 µM, 0.5 µM, 1 µM, 2 µM, 2.5 µM, 3 µM, 4 µM, 5 µM, 6 µM, 7 µM, 8 µM, 9 µM, 10 µM, 11 µM, 12 µM, 12.5 µM, 13 µM, 14 µM, 15 µM, 16 µM, 17 µM, 18 µM, 19 µM, 20 µM, or more. In some cases, cells can be contacted with VPA for more than 1 day, more than 2 days, more than 3 days, more than 4 days, more than 5 days, more than 6 days, more than 7 days, more than 8 days, or more than 9 days.

The Wnt pathway activator DCA may, in some examples, be present in the differentiation medium in concentrations of about 0.1 µM, 0.5 µM, 1 µM, 5 µM, 10 µM, 15 µM, 20 µM, 25 µM, 30 µM, 35 µM, 40 µM, 45 µM, 50 µM, 55 µM, 60 µM, 65 µM, 70 µM, 75 µM, 80 µM, 85 µM, 90 µM, 95 µM, or 100 µM, or more. In some cases, cells can be contacted with DCA for more than 1 day, more than 2 days, more than 3 days, more than 4 days, more than 5 days, more than 6 days, more than 7 days, more than 8 days, or more than 9 days.

In some cases, a TGF- β receptor inhibitor is present in the differentiation medium, either singly or in combination with another chemical agent such as a Wnt pathway activator.
The TGF- β receptor inhibitor is generally capable of inhibiting at least a portion of a TGF- β receptor signaling pathway. In some cases, the TGF- β receptor inhibitor may inhibit at least a portion of a type I TGF- β receptor signaling pathway; in some cases, the TGF- β receptor inhibitor may inhibit type II TGF- β receptor signaling pathway. Examples of a TGF- β receptor inhibitor can include Alk5 inhibitor(s), SB431542, and A83-01. In particular, the use of a TGF- β receptor inhibitor may act as an Alk inhibitor, such as an Alk5 inhibitor.

The TGF- β receptor inhibitor (e.g., Alk5 inhibitor(s)) may, in some examples, be present in the differentiation medium at a concentration of about 0.01 µM, 0.05 µM, 0.1 µM, 0.2 µM 0.5 µM, 0.7 µM, 1 µM, 1.5 µM, 2 µM, 2.5 µM, 3 µM, 4 µM, 5 µM, 6 µM, 7 µM, 8 µM, 9 µM, 10 µM, 11 µM, 12 µM, 12.5 µM, 13 µM, 14 µM, 15 µM, 16 µM, 17 µM, 18 µM, 19 µM, 20 µM, 25 µM, 30 µM, 35 µM, 40 µM, or 50 µM, or more. In some cases, cells can be contacted with a TGF- β receptor inhibitor (e.g., Alk5 inhibitor(s)) for more than 1 day, more than 2 days, more than 3 days, more than 4 days, more than 5 days, more than 6 days, more than 7 days, more than 8 days, or more than 9 days.

SB431542 may, in some examples, be present in the differentiation medium in concentrations about 0.01 µM, 0.05 µM, 0.1 µM, 0.2 µM, 0.5 µM, 0.7 µM, 1 µM, 1.5 µM, 2 µM, 2.5 µM, 3 µM, 4 µM, 5 µM, 6 µM, 7 µM, 8 µM, 9 µM, 10 µM, 11 µM, 12 µM, 12.5 µM, 13 µM, 14 µM, 15 µM, 16 µM, 17 µM, 18 µM, 19 µM, 20 µM, 25 µM, 30 µM, 35 µM, 40 µM, or 50 µM, or more. In some cases, the cells can be contacted with SB431542 for more than 1 day, more than 2 days, more than 3 days, more than 4 days, more than 5 days, more than 6 days, more than 7 days, more than 8 days, or more than 9 days.

A83-01 may, in some examples, be present in the differentiation medium in concentrations of about 0.01 µM, 0.05 v, 0.1 µM, 0.2 µM, 0.5 µM, 0.7 v, 1 v, 1.5 µM, 2 µM, 2.5 µM, 3 µM, 4 µM, 5 µM, 6 µM, 7 µM, 8 µM, 9 µM, 10 µM, 11 µM, 12 µM, 12.5 µM, 13 µM, 14 µM, 15 µM, 16 µM, 17 µM, 18 µM, 19 µM, 20 µM, 25 µM, 30 µM, 35 µM, 40 µM, or 50 µM, or more. In some cases, cells can be contacted with A83-01 for more than 1 day, more than 2 days, more than 3 days, more than 4 days, more than 5 days, more than 6 days, more than 7 days, more than 8 days, or more than 9 days.

In addition to presence of a Wnt pathway activator and a TGF- β receptor inhibitor, additionally signaling molecules may be present. Such signaling molecules can include transferrin, ascorbic acid, XAV939, VEGF, FGF, BIX01294, IGF-1, noggin, creatine, PD169316, SMO antagonist(s), horse serum, or sodium butyrate.

Transferrin may, in some examples, be present in the cell culture in concentrations of about 10 µg/mL, 30 µg/mL, 50 µg/mL, 70 µg/mL, 90 µg/mL, 110 µg/mL, 130 µg/mL, 150 µg/mL, 170 µg/mL, 190 µg/mL, 210 µg/mL, 230 µg/mL, 250 µg/mL, 270 µg/mL, or 300 µg/mL, or more. In some cases, cells can be contacted with transferrin for more than 1 day, more than 2 days, more than 3 days, more than 4 days, more than 5 days, more than 6 days, more than 7 days, more than 8 days, or more than 9 days.

Ascorbic acid may, in some examples, be present in the differentiation medium in concentrations of about 10 µM, 30 µM, 50 µM, 70 µM, 90 µM, 110 µM, 130 µM, 150 µM, 170 µM, 190 µM, 210 µM, 230 µM, 250 µM, 270 µM, or 290 µM, 310 µM, 330 µM, 350 µM, 370 µM, or 400 µM, or more. In some cases, cells can be contacted with ascorbic acid for more than 1 day, more than 2 days, more than 3 days, more than 4 days, more than 5 days, more than 6 days, more than 7 days, more than 8 days, or more than 9 days.

XAV939 may, in some examples, be present in the differentiation medium in concentrations of about 0.01 µM, 0.05 µM, 0.1 µM, 0.2 µM 0.5 µM, 0.7 µM, 1 µM, 1.5 µM, 2 µM, 2.5 µM, 3 µM, 4 µM, 5 µM, 6 µM, 7 µM, 8 µM, 9 µM, 10 µM, 11 µM, 12 µM, 12.5 µM, 13 µM, 14µM, 15 µM, 16 µM, 17 µM, 18 µM, 19 µM, 20 µM, 25 µM, 30 µM, 35 µM, 40 µM, or 50 µM, or more. In some cases, cells can be contacted with XAV939 for more than 1 day, more than 2 days, more than 3 days, more than 4 days, more than 5 days, more than 6 days, more than 7 days, more than 8 days, or more than 9 days.

VEGF may, in some examples, be present in the differentiation medium in concentrations of 5 ng/ml, 10 ng/ml, 15 ng/ml, 20 ng/ml, 25 ng/ml, 30 ng/ml, 35 ng/ml, 40 ng/ml, 45 ng/ml, or 50 ng/ml. In some cases, cells can be contacted with VEGF for more than 1 day, more than 2 days, more than 3 days, more than 4 days, more than 5 days, more than 6 days, more than 7 days, more than 8 days, or more than 9 days.

A fibroblast growth factor (FGF) family member (e.g., FGF, FGF1, FGF2, FGF3, etc.) may, in some examples, be present in the differentiation medium in concentrations of about 1 ng/ml, 5 ng/ml, 10 ng/ml, 15 ng/ml, 20 ng/ml, 25 ng/ml, 30 ng/ml, 35 ng/ml, 40 ng/ml, 45 ng/ml, 50 ng/ml, 100 ng/ml, 250 ng/ml or 500 ng/ml, or more. Cells, in some cases, can be contacted with FGF for more than 1 day, more than 2 days, more than 3 days, more than 4 days, more than 5 days, more than 6 days, more than 7 days, more than 8 days, or more than 9 days.

A histone methyltransferase inhibitor (e.g., BIX01294) may, in some examples, be present in the differentiation medium in concentrations of about 0.01 µM, 0.05 µM, 0.1 µM, 0.2 µM 0.5 µM, 0.7 µM, 1 µM, 1.5 µM, 2 µM, 2.5 µM, 3 µM, 4 µM, 5 µM, 6 µM, 7 µM, 8 µM, 9 µM, 10 µM, 11 µM, 12 µM, 12.5 µM, 13 µM, 14 µM, 15 µM, 16 µM, 17 µM, 18 µM, 19 µM, 20 µM, 25 µM, 30 µM, 35 µM, 40 µM, or 50 µM, or more. Cells, in some examples, can be contacted with BIX01294 for more than 1 day, more than 2 days, more than 3 days, more than 4 days, more than 5 days, more than 6 days, more than 7 days, more than 8 days, or more than 9 days.

IGF-1 may, in some examples, be present in the differentiation medium in concentrations of 0.5 ng/ml, 1 ng/ml, 5 ng/ml, 10 ng/ml, 15 ng/ml, 20 ng/ml, 25 ng/ml, 30 ng/ml, 35 ng/ml, 40 ng/ml, 45 ng/ml, or 50 ng/ml. Cells, in some examples, can be contacted with FGF for more than 1 day, more than 2 days, more than 3 days, more than 4 days, more than 5 days, more than 6 days, more than 7 days, more than 8 days, or more than 9 days.

Noggin may, in some examples, be present in the differentiation medium in concentrations of 10 ng/ml, 30 ng/ml, 50 ng/ml, 70 ng/ml, 90 ng/ml, 110 ng/ml, 130 ng/ml, 150 ng/ml, 170 ng/ml, or 190 ng/ml, 210 ng/ml, 230 ng/ml, or 250 ng/ml. Cells, in some examples, can be contacted with noggin for more than 1 day, more than 2 days, more than 3 days, more than 4 days, more than 5 days, more than 6 days, more than 7 days, more than 8 days, or more than 9 days.

Creatine may, in some examples, be present in the differentiation medium in concentrations of about 0.1 mM, 0.5 mM, 1 mM, 2 mM, 5 mM, 10 mM, 20 mM, 50 mM, or more. Cells can be contacted, in some cases, with creatine for more than 1 day, more than 2 days, more than 3 days, more than 4 days, more than 5 days, more than 6 days, more than 7 days, more than 8 days, or more than 9 days.

PD 169316 may, in some examples, be present in the differentiation medium in concentrations of about 0.001 µM, 0.005 µM, 0.01 µM, 0.05 µM, 0.1 µM, 0.2 µM 0.5 µM, 0.7 µM, 1 v, 1.5 v, 2 v, 2.5 µM, 3 µM, 4 µM, 5 v, 6 µM, 7 µM, 8 µM, 9 µM, 10 µM, 11 µM, 12 µM, 12.5 µM, 13 µM, 14 µM, 15 µM, 16 µM, 17 µM, 18 µM, 19 µM, 20 µM, 25 µM, 30 µM, 35 µM, 40 µM, or 50 µM. In some cases, cells can be contacted with PD169316 for more than 1 day, more than 2 days, more than 3 days, more than 4 days, more than 5 days, more than 6 days, more than 7 days, more than 8 days, or more than 9 days.

SMO antagonist(s) may, in some examples, be present in the differentiation medium in concentrations of about 0.001 µM, 0.005 µM, 0.01 µM, 0.05 µM, 0.1 µM, 0.2 µM 0.5 µM, 0.7 µM, 1 vM, 1.5 µM, 2 µM, 2.5 µM, 3 µM, 4 µM, 5 µM, 6 µM, 7 µM, 8 µM, 9 µM, 10 µM, 11µM, 12 µM, 12.5 µM, 13 µM, 14 µM, 15 µM, 16 µM, 17 µM, 18 µM, 19 µM, 20 µM, 25 µM, 30 µM, 35 µM, 40 µM, or 50 µM, or more. In some cases, cells can be contacted with SMO antagonist for more than 1 day, more than 2 days, more than 3 days, more than 4 days, more than 5 days, more than 6 days, more than 7 days, more than 8 days, or more than 9 days.

Horse serum may, in some examples, be present in the differentiation medium in concentrations of about 0.1%, 0.5%, 1%, 2%, 3%, 4%, 5%, 6%, 7%, 8%, 9%, or 10% or more. In some cases, cells can be contacted with horse serum for more than 1 day, more than 2 days, more than 3 days, more than 4 days, more than 5 days, more than 6 days, more than 7 days, more than 8 days, or more than 9 days.

Sodium butyrate may, in some examples, be present in the differentiation medium in concentrations of about 0.1 µM, 0.5 µM, 1 µM, 2 µM, 5 µM, 10 µM, 30 µM, 50 µM, 70 µM, 90 µM, 110 µM, 130 µM, 150 µM, 170 µM, 190 µM, 210 µM, 230 µM, 250 µM, 270 µM, or 290 µM, 310 µM, 330 µM, 350 µM, 370 µM, or 400 µM, or more. In some instances, cells can be contacted with sodium butyrate for more than 1 day, more than 2 days, more than 3 days, more than 4 days, more than 5 days, more than 6 days, more than 7 days, more than 8 days, or more than 9 days.

In some cases, Alk5 inhibitors can comprise 2-(3-(6-methylpyridine-2-yl)-1H-pyrazol-4-yl)-1,5-naphthyridine, that may, in some examples, be present in concentrations of 0.01 µM, 0.05 µM, 0.1 µM, 0.5 µM, 1 µM, 2 µM, 2.5 µM, 3 µM, 4 µM, 5 µM, 6 µM, 7 µM, 8 µM, 9 µM, 10 µM, 11 µM, 12 µM, 12.5 µM, 13 µM, 14 µM, 15 µM, 16 µM, 17 µM, 18 µM, 19 µM, 20 µM, 25 µM, 30 µM, 35 µM, 40 µM, or 50 µM, or more. In one particular embodiment the concentration of AlkS inhibitor 2-(3-(6-methylpyridine-2-yl)-1H-pyrazol-4-yl)-1,5-naphthyridine is 2 µM or about 2 µM.

In some cases, a compound that is present in a differentiation medium for differentiation of pluripotent stem cells to satellite cells or satellite-like cells is a Leucine-rich repeat kinase 2 (LRRK2) inhibitor. Such inhibitors can include, without limitation, LRRK2-IN-1, CZC 54252, GSK2578215A, GNE-0877, GNE-7915, GNE-9605 and PF 06447475.

In some examples, the LRRK2 inhibitor is LRRK2-IN-1. LRRK2-IN-1 may be present in the differentiation medium at a concentration of about 1 nM, 5 nM, 10 nM, 20 nM, 30 nM, 40 nM, 50 nM, 60 nM, 70 nM, 80 nM, 90 nM, 100 nM, 200 nM, 300 nM, 400 nM, 500 nM, 600 nM, 700 nM, 800 nM, 900 nM, 1 µM, 2 µM, 3 µM, 4 µM, 5 µM, 10 µM, 20 µM, 30 µM, 40 µM, 50 µM, 60 µM, 70 µM, 80 µM, 90 µM, 100 µM or more than 100 µM. In some cases, the cells can be contacted with LRRK2-IN-1 for more than 1 day, more than 2 days, more than 3 days, more than 4 days, more than 5 days, more than 6 days, more than 7 days, more than 8 days, or more than 9 days.

### ii. Exposing Pluripotent Stem Cells to Differentiation Medium

Pluripotent stem cells (or other type of stem cell) may be differentiated to satellite cells, or satellite-like cells, by contacting the pluripotent stem cells (or other type of stem cell) with one or more differentiation media. The methods disclosed herein include one-step methods of differentiating a pluripotent stem cell (or other stem cell) wherein a single agent, or single combination of agents provided at the same time, triggers the differentiation pathway. In some cases, the method may comprise introducing a nucleic acid into a pluripotent stem cell (e.g., via transfection, transduction, viral transduction, electroporation, etc.) such that the pluripotent stem cell expresses the nucleic acid. In some cases, the method does not comprise introducing a nucleic acid into a pluripotent stem cell, or does not comprise transfecting a nucleic acid into a pluripotent stem cell, or does not comprise electroporating a nucleic acid into a pluripotent stem cell, or does not comprise transducing a nucleic acid (e.g., via viral vector) into a pluripotent stem cell, such that the nucleic acid is expressed by the cell and causes, or contributes to the differentiation of the pluripotent stem cell into a satellite cell or satellite-like cell. In some cases, the method comprises introducing a myogenic protein to the pluripotent stem cells. In some cases, the method does not comprise introducing a myogenic protein to the pluripotent stem cells.

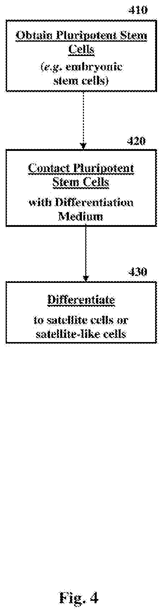

FIG. 4 illustrates an example of differentiating pluripotent stem cells to satellite cells or satellite-like cells. The pluripotent stem cells (410) can be plated and cultured as described herein or by any method known in the art, e.g., by plating as single cells in appropriate culture medium. In some cases, the pluripotent stem cells are contacted with the differentiation medium (420) in a single step, thereby causing differentiation of the pluripotent stem cells into satellite cells or satellite-like cells or otherwise generating satellite cells or satellite-like cells.

In general, the single-step contacting may comprise contacting the pluripotent stem cells with a single differentiation medium that is provided to the cells at once, or serially over time (e.g., via media changes). In some cases, the single-step contacting may comprise contacting the pluripotent stem cells with a single differentiation medium that is provided to the cells at different concentrations over time (e.g., media changes involving altering the concentrations of differentiation media). In some embodiments, the components present in the single differentiation medium are sufficient to cause the pluripotent stem cells to differentiate into satellite cells or satellite-like cells (e.g., cells with functional, structural, morphological, or expression marker characteristics resembling those of a naturally-occurring satellite cell). In some embodiments, the component(s) present in the single differentiation medium are sufficient to cause satellite cells or satellite-like cells to be generated from the pluripotent stem cells. In some cases, the components present in the single differentiation medium are sufficient to cause the pluripotent stem cells to differentiate into satellite cells or satellite-like cells when the cells are serially exposed to the components (e.g., via one or more media changes). In some cases, contacting the pluripotent stem cells with the single differentiation medium comprises continuously contacting the cells with the differentiation medium. In other cases, contacting the pluripotent stem cells with the single differentiation medium comprises sporadically or serially contacting the cells with the differentiation medium.

In some cases, a component or set of components within a medium disclosed herein may be able to directly cause the generation of satellite cells or satellite-like cells from one or more pluripotent stem cells. For example, in some cases, a Wnt pathway activator and a TGF- β receptor inhibitor may, together, be capable of causing the generation of satellite cells or satellite-like cells from pluripotent stem cells without the addition of an additional differentiation agent.

In some cases, the contacting comprises contacting the pluripotent stem cells with two or more different differentiation media. The two or more different differentiation media may comprise different components. In some cases, the two or more different differentiation media are 2 or more, 3 or more, 4 or more, 5 or more, 6 or more, 7 or more, 8 or more, 9 or more, or 10 or more different differentiation media.

In some cases, the pluripotent stem cells may be contacted by or exposed to the one or more differentiation media (with or without media changes) for at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 21 days, or at least 28 days. In some cases, the pluripotent stem cells may be contacted by the one or more differentiation media (with or without media changes) for at most 1 day, at most 2 days, at most 3 days, at most 4 days, at most 5 days, at most 6 days, at most 7 days, at most 8 days, at most 9 days, at most 10 days, at most 11 days, at most 12 days, at most 13 days, at most 14 days, at most 21 days, or at most 28 days. In some cases, the pluripotent stem cells are contacted with or exposed to the differentiation medium for about 12 hours, 1 day, 2 days, 3 days, 4 days, 5 days, 6 days, 7 days, 8 days, 9 days, 10 days, 11 days, 12 days, 13 days, 14 days, 21 days, 28 days, 30 days, 35 days, 40 days, 45 days, 50 days, 55 days, 60 days, 65 days, or 70 days.

The one or more pluripotent stem cells may be concurrently contacted by compounds of the differentiation medium, e.g., two or more compounds are administered to the pluripotent stem cells during an overlapping time-frame. For example, the pluripotent stem cells may be contacted with one compound on days 1-3 and with a second compound from days 2-5. In some cases, the one or more pluripotent stem cells may be simultaneously contacted by compounds of the differentiation medium. For example, the pluripotent stem cells may be contacted with two compounds during the same timeframe (e.g., contacted with two compounds for days 1-3). In some cases, the pluripotent stem cells are serially or sequentially contacted with two or more compounds of the differentiation media. For example, the pluripotent stem cells may be contacted with one compound on days 1-3 and with a second compound from days 4-6.

Components of the differentiation medium can be added in a single step. Components of the medium can be added sequentially. Additionally, components of the differentiation medium can be added simultaneously. Components of the differentiation medium can also be added in an overlapping manner, by contacting the cells with one component for a period of time before applying a second component. Components of the medium can also be added to cells individually or in mixtures. These additions can be performed without changing the composition of the medium throughout the process, such that the differentiation occurs due to the exposure to a single medium composition.

As mentioned herein, the differentiation medium (or media) may be changed or exchanged over time. In some cases, the differentiation medium is changed, added to, or replaced. Often, throughout these media exchanges the composition of the differentiation medium stays steady throughout the differentiation of the pluripotent stem cells to satellite cells or satellite-like cells. In some cases, the composition of the differentiation medium is varied. Media changes can be performed regularly, such as every six hours, every 12 hours, every day, every other day, every third day, every fourth day, or every fifth day. In some cases, the differentiation medium (or media) is changed at least 1, 2,3, 4, 5, 6, 7, 8, 9, 10 or 15 times during the process of differentiating the pluripotent stem cells into satellite cells or satellite-like cells. Media can also be continuously added and removed, such as in a chemostat culture.

Pluripotent stem cells can be exposed to the differentiation medium continuously. Pluripotent stem cells can be exposed to the differentiation medium for a period of time before being returned to a maintenance medium. Further, differentiation can continue for a specific quantity of time (e.g. three days, five days, seven days, ten days, or fifteen days) or until a given gene or morphological marker is detected.

### iii. Yield, Efficiency, and Other Beneficial Features of the Methods of producing Satellite cells and Satellite-like Cells using Differentiation Medium

The methods disclosed herein may result in high yields of satellite cells or satellite-like cell and/or may have high efficiencies. For example, when a plurality of pluripotent stem cells in an *in vitro* culture are differentiated using a differentiation medium as described herein, greater than 40% of the cells differentiated from said plurality of pluripotent stem cells may express Pax3, Pax7, and/or CD56. In some cases, greater than 20%, 30%, 40%, 45%, 50%, 55%, 60%, 65%, 70%, 75%, 80%, 85%, 90%, 91%, 92%, 93%, 94%, 95%, 96%, 97%, 98%, or 99% of the cells differentiated from said plurality of pluripotent stem cells may express Pax3, Pax7, and/or CD56. In some cases, greater than 20%, 30%, 40%, 45%, 50%, 55%, 60%, 65%, 70%, 75%, 80%, 85%, 90%, 91%, 92%, 93%, 94%, 95%, 96%, 97%, 98%, or 99% of the cells differentiated from said plurality of pluripotent stem cells are capable of differentiating into myoblasts. In some cases, greater than 20%, 30%, 40%, 45%, 50%, 55%, 60%, 65%, 70%, 75%, 80%, 85%, 90%, 91%, 92%, 93%, 94%, 95%, 96%, 97%, 98%, or 99% of the cells differentiated from said plurality of pluripotent stem cells are capable of differentiating into functional myoblasts.

In some cases, the time (or duration) to generate satellite cells or satellite-like cells from a plurality of pluripotent stem cells by performing the methods disclosed herein may be on the order of days to weeks. In some cases, the duration from pluripotent stem cell to satellite cell or satellite-like cell may be about 1 day, about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, or about 20 days. Duration may be calculated as the time from start of differentiation (e.g., plating pluripotent stem cells in differentiation media) to the time when a majority of the pluripotent stem cells have differentiated to satellite cells or satellite-like cells, for example, when at least about 50%, 55%, 60%, 65%, 70%, 75%, 80%, 85%, 86%, 87%, 88%, 89%, 90%, 91%, 92%, 93%, 94%, 95%, 96%, 97%, 98%, 99% or 100% of the pluripotent stem cells in the culture have differentiated to satellite cells or satellite-like cells.

In some cases, the duration of differentiation of pluripotent stem cells to myoblasts by performing the methods disclosed herein may be on the order of days to weeks. For example, the duration from differentiation of pluripotent stem cells to myoblasts may be about 10 days, about 11 days, about 12 days, about 13 days, about 14 days, about 15 days, about 16 days, about 17 days, about 18 days, about 19 days, about 20 days, about 21 days, about 22 days, about 23 days, about 24 days, about 25 days, about 26 days, about 27 days, about 28 days, about 29 days or about 30 days. Duration may be calculated as the time from start of differentiation (e.g., plating pluripotent stem cells in differentiation media) to the time when a majority of the pluripotent stem cells have differentiated to myoblasts, for example, when at least about 50%, 55%, 60%, 65%, 70%, 75%, 80%, 85%, 86%, 87%, 88%, 89%, 90%, 91%, 92%, 93%, 94%, 95%, 96%, 97%, 98%, 99% or 100% of the pluripotent stem cells in the culture have differentiated to myoblasts.

A pluripotent stem cell provided in an *in vitro* culture may be contacted with one compound, or two or more compounds concurrently, thereby differentiating said human pluripotent stem cell into a cell expressing Pax3, Pax7, and/or CD56 (e.g., Pax3/CD56, Pax7/CD56, Pax3/Pax7/CD56). In particular, the cell expressing Pax3, Pax7, and/or CD56 may have the potential to form a myoblast, with a yield such that greater than five cells expressing Pax3, Pax7, and/or CD56 are generated from said pluripotent stem cell within a certain period of time (e.g., a nine-day period). Accordingly, when pluripotent stem cells are grown as a population in culture, the culture may produce cells expressing Pax3, Pax7, and/or CD56 in at least a 5:1 ratio to the initial number of pluripotent stem cells. In some cases the ratio is at least 2:1, 3:1, 4:1, 5:1, 6:1, 7:1, 8:1, 9:1, 10:1, 11:1, 12:1, 13:1, 14:1, 15:1, 20:1, 50: 1 or 100:1. In some cases, the ratio is achieved within 2, 3, 4, 5, 6, 7, 8, 9, 10, 12, 13, 14, 15, 16, 17, 18, 19, 20, 21, 22, 23, 24, 25, 30, 35, 40 ,45, 50, 70 or 100 days.

The methods disclosed herein may generate satellite cells or satellite-like cells from pluripotent stem cells at a high purity. Purity may refer to the percentage (%) or fraction of total cells in the culture that are Pax3, Pax7 and/or CD56 positive. Purity may be assessed at each stage of differentiation, for examples, the purity of satellite cells or satellite-like cells, the purity of myoblasts, and/or the purity of myotubes can be assessed. In some cases, the purity of satellite cells or satellite-like cells in the culture, after performing a differentiation method as disclosed herein, is at least about 50%, 55%, 60%, 65%, 70%, 75%, 80%, 85%, 86%, 87%, 88%, 89%, 90%, 91%, 92%, 93%, 94%, 95%, 96%, 97%, 98%, 99%, or 100%. In some cases, the purity of myoblasts, after performing a differentiation method as disclosed herein, is at least about 50%, 55%, 60%, 65%, 70%, 75%, 80%, 85%, 86%, 87%, 88%, 89%, 90%, 91%, 92%, 93%, 94%, 95%, 96%, 97%, 98%, 99%, or 100%. In some cases, the purity of myotubes, after performing a differentiation method as disclosed herein, is at least about 50%, 55%, 60%, 65%, 70%, 75%, 80%, 85%, 86%, 87%, 88%, 89%, 90%, 91%, 92%, 93%, 94%, 95%, 96%, 97%, 98%, 99%, or 100%. In some cases, the purity is obtained without first performing one or more purification or enrichment steps, such as one or more sorting steps (e.g., flow cytometry). In some cases, the purity of satellite cells or satellite-like cells, myoblasts, or myotubes is at least 90%, 91%, 92%, 93%, 94%, 95%, 96%, 97%, 98%, 99% or 100% without performing one or more purification or enrichment steps, such as one or more sorting steps (e.g., flow cytometry).

In some cases, the population of cells after performing a differentiation method of the present disclosure is substantially free of neural cells or neural progenitor cells. For example, the population of cells after performing a differentiation method disclosed herein contains no more than about 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 15%, 20%, 25%, 30%, 35% or 40% of neural cells or neural progenitor cells.

After differentiation, satellite cells or satellite-like cells can be maintained in any media suitable for myogenic culturing, such as any of the described basal media. Satellite-like cells can be maintained in media containing the compounds used to induce differentiation. The resulting satellite cells or satellite-like cells can be used directly. The resulting satellite cells or satellite-like cells can be expanded in culture for an additional doubling, two doublings, three doublings, four doublings, five doublings, or six doublings, eight doublings, ten doublings, twelve doublings, fourteen doublings, sixteen doublings, eighteen doublings, twenty doublings, twenty-two doublings, twenty-four doublings, twenty-six doublings, twenty-eight doublings, or thirty doublings.

### C. Differentiation of HLA-null pluripotent stem cells into satellite cells or satellite-like cells

HLA-null satellite cells or satellite-like cells described herein may be produced from a less-differentiated cell (e.g., pluripotent stem cell, multipotent stem cell, muscle-lineage restricted progenitor cell) by chemical differentiation, by forced expression of genetic markers that are associated with satellite cells or satellite-like cells, or by any other differentiation process known in the art. Forced expression can be achieved by introducing expression vectors encoding the proteins into the cell, transduction of cells with recombinant viruses, introduction of exogenous purified polypeptides of interest into cells, introduction of exogenous purified mRNAs encoding polypeptides of interest into cells, contacting cells with a reagent (e.g., non-naturally occurring reagent) that induces expression of a marker of interest (e.g., Pax3, Pax7, or CD56), or any other biological, chemical, or physical process to induce expression of a gene encoding a polypeptide of interest.

In some examples, the HLA-null pluripotent stem cells can be differentiated by the chemical differentiation methods described herein. Additionally, the HLA-null pluripotent stem cells may be differentiated to satellite cells or satellite-like cells under culturing conditions that comprise transfection

An HLA-null pluripotent stem cell may be cultured under conditions to promote differentiation of the HLA-null pluripotent stem cell into a cell expressing Pax3, Pax7 and/or CD56, thereby producing an HLA-null cell expressing Pax3, Pax 7, and/or CD56. In particular, conditions of culturing the HLA-null pluripotent stem cell may comprise contacting said HLA-null pluripotent stem cell with a compound to promote differentiation of said HLA-null pluripotent stem cell into said cell expressing Pax3, Pax7 and/or CD56.

For example, the HLA-null pluripotent stem cell may be differentiated into an HLA-null skeletal muscle progenitor cell. An HLA-null skeletal muscle progenitor cell may express at least one of Pax3, Pax7, MyoD, MF20 and CD56. The HLA-null skeletal muscle progenitor cell may be capable of forming a myoblast. Additionally, the HLA-null human pluripotent stem cell may be differentiated into an HLA-null cell that is capable of forming a precursor for a specific tissue. Examples may include differentiating the HLA-null pluripotent stem cell to mesenchymal tissue or a muscle-lineage specific progenitor. The specific tissue or differentiated cells may then be provided for a subject in need thereof. Additionally, the differentiation of the HLA-null human pluripotent stem cell may be differentiated using a method of differentiation that occurs in a single step.

HLA-null pluripotent stem cells can be treated with an exogenous polypeptide to induce differentiation. For example, the myogenic factor can be provided to the stem cells by i) treating said cells with a myogenic factor protein; ii) inducing said myogenic factor expression in said cells; or iii) introducing in said cells a nucleic acid capable of expressing the myogenic factor. In an embodiment, the myogenic factor is provided by introducing in the cells a nucleic acid capable of expressing the myogenic factor. In an embodiment, the myogenic factor is a nucleic acid encoding MyoD.

Furthermore, HLA-null pluripotent stem cells can be differentiated to satellite cells or satellite-like cells by first culturing in conditions that promotes the differentiation to mesenchymal stem cells, such as by treatment with Activin A. These cells can be sorted to obtain a population highly enriched for PDGFRα-positive cells, which can then be treated with lithium chloride to induce differentiation to satellite cells or satellite-like cells.

### V. Features of Satellite-like Cells, and Detection of Satellite-like features

As used herein, the term "satellite-like cell" refers to any cell that possesses structural or functional features associated with a naturally-occurring satellite cell (e.g., satellite cells within an organism such as a human) but yet also possesses at least one structural or functional feature distinguishing the satellite-like cell from a naturally-occurring satellite cell. In preferred embodiments, a satellite-like cell is a cell that is (a) produced *in vitro* from a less-differentiated cell such as a stem cell, preferably a pluripotent stem cell or (b) derived from a satellite-like cell, such as resulting cells from proliferation of a satellite-like cell. As used herein, the term "satellite cell" refers to a cell that possesses the structural and functional features exhibited by a naturally-occurring satellite cell or myosatellite cell, and may or may not possess at least one structural or functional feature that distinguishes it.

The satellite-like cells disclosed herein possess features in common with naturally-occurring satellite cells, and also possess features different from those found in a naturally-occurring satellite cell. The satellite-like cells disclosed herein express specific markers, at the RNA and/or protein level. In some cases, the markers are one or more of the following markers: Pax3, Pax7, Myf5, or CD56 (which can also be referred to as leukocyte antigen, or Leu-19, or membrane-bound neural cell adhesion molecule, or N-CAM). In some cases, the markers include myocyte nuclear factor (MNF) or c-met protooncogene (a receptor for hepatocyte growth factor, HGF). In preferred embodiments, the satellite-like cells express Pax 3, Pax 7 and CD56. In some embodiments, satellite-like cells that have been partially differentiated might only express subsets of these markers, such as CD56 alone. In some cases, the satellite-like cells do not express significant levels of CSPG4. In some cases, the satellite-like cells do not express significant levels of myogenic regulatory factors (MRFs) such as, for example, MyoD, Myf5, myogenin or MRF4. Naturally occurring adult satellite cells express Pax7, whereas Pax3 is predominantly a marker of naturally occurring embryonic satellite cells.

The satellite-like cell markers may be detected by any method known in the art. Expression of markers can be assayed by detection of mRNA transcript, such as by RT-PCR, RNA sequencing or sequencing cDNA complements of the mRNA transcript. Global expression of RNA markers (or expression of multiple markers) can be detected by any technique known in the art, including microarray and sequencing (e.g., Sanger sequencing, high-throughput sequencing, Next-Generation sequencing, massively parallel high-throughput sequencing, etc.). Expression of protein markers can be assayed by immunofluorescence, the production of gene-fusion products (e.g., a Pax7-GFP reporter construct), radiolabeling, immunoprecipitation, western blot, or any other method known in the art. Proteomic techniques (e.g., protein arrays) may also be used to detect protein markers associated with satellite-like cells. Expression of markers can be inferred indirectly by assaying downstream effects of their expression, such as by detecting the activation or repression of genes whose activity is controlled by the markers.

Satellite cells or satellite-like cells can be identified by their morphology. Satellite-like or satellite cells can have a high nuclear-to-cytoplasmic volume ratio in comparison to myoblast, fibroblast or epithelial cells. Satellite cells or satellite-like cells can have few organelles (e.g. ribosomes, endoplasmic reticulum, mitochondria, golgi complexes) in comparison to myoblast, fibroblast or epithelial cells. Satellite-like cells can have a nucleus with a cross-sectional area of approximately 170 µm². Satellite cells or satellite-like cells can have a large quantity of nuclear heterochromatin relative to myonuclei. Activated satellite cells or satellite-like cells may have an increased number of caveolae, cytoplasmic organelles, and decreased levels of heterochromatin compared to quiescent satellite cells or satellite-like cells. Satellite-like cells or satellite cells are less elongated and less spindle-shaped compared to myoblasts.

In general, the satellite cells or satellite-like cells disclosed herein are capable of forming myoblasts, particularly myoblasts that can fuse to form myotubes. This capability can be detected functionally, such as by demonstrating differentiation into myoblasts or myotubes. Differentiation can be performed in culture. Differentiation can be performed *in vivo,* for example, by injecting satellite cells or satellite-like cells into an appropriate animal subject and tracking their differentiation.

For HLA-null satellite cells or satellite-like cells, the absence of HLA gene product can be demonstrated by immunofluorescence, western blot, or any known method. In some cases, the presence or absence of the HLA gene can be assessed using methods known in the art, e.g., PCR.

### VI. Differentiation of Satellite Cells or Satellite-like Cells into Myoblasts and Myotubes

Satellite cells or satellite-like cells may be capable of differentiating into myoblasts and functioning myotubes. Satellite cells or satellite-like cells can be further differentiated into myoblasts and myotubes *in vivo.* This can be accomplished by administering the satellite cells or satellite-like cells to an appropriate subject, such as a hu-mouse treated with cardiotoxin or an appropriate human recipient in need thereof. For example, a human recipient in need thereof may be a subject with a muscular degenerative disease or disorder.

In some instances, satellite cells or satellite-like cells can be further differentiated into myoblasts and myotubes *in vitro.* Such differentiation can occur through the application of growth factors, such as IGF-1 or TGF- β. Such differentiation can occur by the application of small molecules, such as BIX01294 or SB431542.

### VII. Applications

Satellite cells or satellite-like cells disclosed herein (including HLA-null satellite cells and satellite-like cells that are derived from HLA-null pluripotent stem cells), may be used in a wide variety of clinical applications such as cell therapies which involve introducing the cells into a subject, such as a patient with a muscular degenerative disease or disorder. The cells disclosed herein may also be useful for drug screening, and research on normal development and physiology, as well as diseases.

### A. Subjects

The satellite-like and satellite cells disclosed herein can be used to treat or ameliorate the symptoms of a wide variety of subjects. Subjects who may generally benefit from the cells and methods disclosed herein are subjects with a muscular disease or disorder that affects muscle function, tone or physiology. In some cases, the subjects may have a genetic disease (e.g., Huntington's disease, muscular dystrophy); in some cases, the subjects may have an acquired disorder (e.g., muscle atrophy caused by inactivity). Additionally, subjects with muscular dystrophy may have multi-system disorders with manifestations in body systems including the heart, gastrointestinal system, nervous system, endocrine glands, eyes and brain. Subjects in need of treatment can include those who have undergone muscle strain or injury. The muscle injury may be the result of a traumatic event, such as a slip or fall during an activity such exercise.

Subjects in need of treatment may also include those experiencing muscle atrophy or wasting, including muscle atrophy that may occur as a result of cachexia or wasting syndrome. Cachexia may be accompanied by muscle atrophy, loss of weight, fatigue, weakness, and significant loss of weight. The methods of treatment disclosed herein may help reverse some of these symptoms, particularly muscle atrophy and weakness. Subjects with cachexia may include patients with cancer, acquired immune deficiency syndrome (AIDS), chronic obstructive lung disease, multiple sclerosis, congestive heart failure, tuberculosis, familial amyloid polyneuropathy, gadolinium poisoning, mercury poisoning (acrodynia) and hormonal deficiency.

In some cases, subjects in need of treatment are patients with sarcopenia, or loss of muscle mass or function associated with the aging process. The treatments disclosed herein may help reverse or improve the sarcopenia, or loss of muscle mass or function; in some cases, the treatments disclosed herein help prevent the sarcopenia, or loss of muscle mass or function, from worsening over time.

Subjects who may benefit from the disclosed compositions and methods include subjects who desire prophylactic treatment, such as subjects at risk of loss of muscle mass. Such subjects may include those about to undergo treatment regimens that can reduce muscle mass, such as chemotherapy. Such subjects also can include subjects who have been immobilized or partially immobilized for periods of time sufficient to reduce muscle mass, such as due to unconsciousness or wearing an immobilizing cast. Examples of subjects may include those who have recently undergone surgery which has damaged or reconnected muscle tissue. Examples of subjects may also include those born without a specific muscle or in need of a muscle graft. Subjects may also be subjects seeking improved muscle mass or function for cosmetic reasons or to improve athletic performance.

Subjects in need of satellite cell or satellite-like cell transplants may include men or women. Such subjects may be of a range of ages, which may include > 10 minutes old, > 1 hour old, > 1 day old, > 1 month old, > 2 months old, > 6 months old, > 1 year old, >2 years old, >5 years old, >10 years old, >15 years old, >18 years old, >25 years old, >35 years old, >45 years old, >55 years old, >65 years old, >80 years old, <80 years old, <70 years old, <60 years old, <50 years old, <40 years old, <30 years old, <20 years old or <10 years old. The subject may be a neonatal infant. In some cases, the subject is a child or an adult. In some examples, the tissue is from a human of age 2, 5, 10 or 20 hours. In other examples, the tissue is from a human of age 1 month, 2 months, 3 months, 4 months, 5 months, 6 months, 9 months or 12 months. In some cases, the tissue is from a human of age 1 year, 2 years, 3 years, 4 years, 5 years, 18 years, 20 years, 21 years, 23 years, 24 years, 25 years, 28 years, 29 years, 31 years, 33 years, 34 years, 35 years, 37 years, 38 years, 40 years, 41 years, 42 years, 43 years, 44 years, 47 years, 51 years, 55 years, 61 years, 63 years, 65 years, 70 years, 77 years, or 85 years. Subjects may have differing genetic backgrounds, including different racial groups or genetically admixed populations.

### B. Cell Therapies

The satellite cells or satellite-like cells may be used as a therapy to treat a subject with a disease or disorder (e.g., a genetic defect), particularly a disease or disorder affecting muscle function. The therapy may be directed to treating the cause of the disease and/or to treat the effects of the disease or condition. The satellite cells or satellite-like cells may be transferred to, or close to, an injured site in a subject; or the cells can be introduced to the subject in a manner allowing the cells to migrate, or home, to an injured site. For example, the cells may be enclosed in a material, such as a microcapsule, designed to shuttle the cells to a site of interest. In some examples, the transferred cells may advantageously replace the damaged, diseased, or injured cells and allow improvement in the overall condition of the subject. In some instances, the transferred cells may stimulate tissue generation or repair.

In a representative example, a subject with a muscular degenerative disease or other muscular disorder (e.g., muscle injury) is treated with satellite cells or satellite-like cells that have been derived from pluripotent stem cells in methods described herein. In particular, the pluripotent stem cells may be differentiated by contacting the pluripotent stem cells with an agent or agents to differentiate the pluripotent stem cells into satellite cells or satellite-like cells, which are transplanted into the subject. The satellite cells or satellite-like cells that are derived from the pluripotent stem cells may be capable of forming myoblasts, in *vitro* and/or *in vivo.* In some instances, the satellite cells or satellite-like cells may be introduced to the subject in need thereof. The satellite cells or satellite-like cells may be introduced into the muscle of the subject having a muscular degenerative disease or disorder. In some cases, cells derived from the satellite cells or satellite-like cells such as myoblasts, myotubes, or genetically-modified satellite or satellite-like cells are introduced into the subject.

In some examples, satellite cells that are genetically modified, or derived from genetically altered cells (such as genetically modified pluripotent stem cells) are introduced into the subject. In some examples, an induced pluripotent stem cell line may be generated from a patient with a muscular deficiency disease or disorder such as muscular dystrophy that is caused by a genetic mutation. The mutation may be corrected in the induced pluripotent stem cells which may then be differentiated into satellite cells or satellite-like cells according to the present disclosure. The satellite-like cells or satellite cells with the corrective mutation may then be transplanted into the patient in order to restore, improve, or enhance muscle function.

In some specific examples, induced pluripotent stem cells (or an induced pluripotent stem cell line) may be generated from a patient with a mutation causing muscular dystrophy (e.g., Duchenne muscular dystrophy). The mutation may be corrected in the induced pluripotent stem cells using genetic-modification techniques known in the art. The genetically-modified induced pluripotent stem cells may be differentiated to satellite cells or satellite-like cells according to the present disclosure. The satellite cells or satellite-like cells may be transplanted into the patient where they may produce functional, non-mutated proteins so as to restore or enhance muscle function.

The treatment of a muscular degenerative disease such as muscular dystrophy (e.g., Duchenne muscular dystrophy) can be accomplished by injection of satellite cells or satellite-like cells that have the ability to restore muscle loss, into muscles that are diseased or injured. The satellite cells or satellite-like cells may produce myoblasts and fuse with existing myotubes. As myotubes have contiguous cytoplasms, the dystrophin produced by the myoblasts derived from the transplanted satellite cells or satellite-like cells may be found throughout the myotube and may complement the endogenous defect. A similar procedure may be performed to correct or improve any condition, disease, or disorder caused by the lack of sufficient quantity of a specific gene product.

The satellite cells or satellite-like cells may be transferred to subjects suffering from a wide range of diseases and disorders. Subjects suffering from neurological and/or neuromuscular diseases or disorders could especially benefit from satellite cell therapies. In some approaches, the differentiated cells may be transplanted to a muscle site to treat a neuromuscular condition, e.g. muscular dystrophy, Duchenne muscular dystrophy, etc. A muscular disease or disorder that may be treated by, or ameliorated by, the disclosed satellite-like cells and satellite cells may be a genetic disease or disorder, or may have non-genetic causes. In some cases, the disease or disorder is chronic; in others, the disease or disorder is acute or subacute; in still other cases, the disease or disorder is a recurrent disease or disorder. Exemplary diseases or disorders that may be treated by, or ameliorated by, the disclosed cells may include genetic diseases as well as non-genetic diseases. Exemplary diseases or disorders may include: muscular dystrophy, Huntington's disease, Merosin deficiency 1A, nemaline myopathy, and Spinal Muscular Atrophy (SMA). Examples of muscular dystrophies that may be treated or improved by the disclosed cells include Becker, congenital, facioscapulohumeral (FSH), myotonic (type I and II), oculopharyngeal, distal, Duchenne muscular dystrophy, and Emery-Dreifuss muscular dystrophy. Duchenne and Becker muscular dystrophies are caused by a mutation of a gene located on the X chromosome and predominantly affect males, although females can sometimes have severe symptoms as well. Additional diseases or disorders that may be treated by, or ameliorated by, the disclosed methods and compositions may include: cachexia, sporadic diseases, sarccopenia, muscle wasting, muscle atrophy, muscle strain, muscle injury, multiple sclerosis, Parkinson's Disease, or muscle wasting associated with aging.

Satellite cells or satellite-like cells, or cells derived therefrom, may be placed into a patient to treat a disease or disorder. For example, satellite cells or satellite-like cells may be transplanted using direct injection into skeletal muscle. Additionally or alternatively, the satellite cells or satellite-like cells may be transplanted into a subject using a scaffold, using a scaffold-free method, or using other transplantation devices. The scaffold may be made of any material known in the art. In some cases, the scaffold is a biodegradable scaffold, a resorbable scaffold, or other type of scaffold. In some cases, the scaffold comprises a matrix (e.g., biodegradable matrix, resorbable matrix). In some cases, the satellite cells or satellite-like cells, or cells derived therefrom, are encapsulated in microcapsule(s) prior to transplantation. In some cases, the microcapsule may possess homing features enabling the cells to be directed to a location of interest.

Satellite cells or satellite-like cells, such as skeletal muscle progenitor cells, may be injected at a number of locations across the body of a subject. For example, the satellite cells or satellite-like cells may be injected at locations to access muscle formation, e.g. arm muscles such as coracobrachialis, biceps brachii, and brachialis, leg muscles such as tibialis anterior; extensor hallucis longus; extensor digitorum; and fibularis tertius, or other muscle locations.

The number of administrations of treatment to a subject may vary. Introducing the differentiated cells into the subject may be a one-time event; but in certain situations, such treatment may elicit improvement for a limited period of time and require an on-going series of repeated treatments. In other situations, multiple administrations of the cells may be required before an effect is observed. The exact protocols depend upon the disease or condition, the stage of the disease, and parameters of the individual subject being treated.

In some examples, the cells may be introduced to the subject via any of the following routes: parenteral, intravenous, intraarterial, intramuscular, subcutaneous, transdermal, intraperitoneal, or into spinal fluid. In particular, the cells may be introduced to the subject via direct injection of the cells into skeletal muscle of the subject.

During transplantation of the satellite cells or satellite-like cells, drugs may be given to the subject during the same period of time. For example, drugs may be administered prior to, during, or subsequent to transplantation of satellite-like cells, or a combination thereof. Examples of drugs that may be administered to the subject include drugs to treat the disease or injury to the subject; immunosuppressant drugs; no immunosuppressant drugs; or combinations thereof. Exemplary immunosuppressive drugs include calcineurin inhibitors, such as cyclosporine or tacrolimus, mTOR inhibitors, such as sirolimus or everolimus, purine synthesis inhibitors or purine analogues, such as mycophenolate mofetil or azathioprine, or steroids, such as prednisone.

Satellite cells and satellite-like cells can be administered using a variety of instruments, such as syringes. Satellite cells and satellite-like cells can be also be injected with a buffer, such as saline, phosphate-buffered saline or serum. Satellite cells and satellite-like cells may be administered with antibiotics, such as vancomycin or levofloxacin.

The dosage of satellite cells or satellite-like cells that may be transplanted into a subject may differ based on the disease or injury of the subject, the progression of the disease or injury of the subject, and the degree of severity of the disease or injury of the subject.
Additionally, the number of treatments provided to a subject may vary. A single treatment may be administered to the subject or multiple treatments may be given to the subject. In some cases, the subject may be treated about 1, 2, 3, 4, 5, 6, 7, 8, 9, 10, 11, 12, 13, 14, 15, 20, 25 or more times with the cells disclosed herein. In some cases, the subject may be treated less than 1, 2, 3, 4, 5, 6, 7, 8, 9, 10, 15 or 20 times within a year period. The treatments themselves may also vary in the number of sites that are provided with satellite cells or satellite-like cells. In examples, a single treatment of satellite cell or satellite-like cell transplantation may include 1, 2, 3, 4, 5, 6, 7, 8, 9, 10, 20, 50 or 100 or more injection sites for the direct skeletal muscle injection of satellite cells or satellite-like cells. In some cases, a single dose of cells comprises about 10', about 50, about 10², about 5x10², about 10³, about 5x10³, about 10⁴, about 5 x 10⁴, 10⁵, about 5x10⁵, about 10⁶, about 5x10⁶, about 10⁷, about 5x10⁷, about 10⁸, about 5x10⁸, about 10⁹, about 5 x 10⁹, about 10¹⁰, about 5x10¹⁰, about 10¹¹ about 5x10¹¹ ,or more cells. In some cases, a single dose of cells comprises at most 10², at most 5x10², at most 10³, at most 5x10³, at most 10⁴, at most 5 x 10⁴, at most 10⁵, at most 5x10⁵, at most 10⁶, at most 5x10⁶, at most 10⁷, at most 5x10⁷, at most 10⁸, at most 5x10⁸, at most 10⁹, at most 5 x 10⁹, at most 10¹⁰, at most 5x10¹⁰, at most 10¹¹, or at most 5x1¹¹ cells.

Once satellite cells or satellite-like cells are provided to the patient, the satellite cells or satellite-like cells may fuse with myoblasts of the subject and may form fused muscle cell components. Consequences of treatment may include restoration of muscle; halting of muscle degradation; slowing of muscle degradation; improvement of factors associated with a subject's disease or injury such as production of dystrophin; or combinations thereof.

Improvement of factors associated with a subject's disease or injury may be associated with tests of muscle restoration or muscle function. The degree of muscle restoration may be assessed by one or more tests of certain muscle attributes, such as muscle mass, muscle strength as measured by resistance to a force, amount of contraction in response to a stimulus, and strength of contraction in response to a stimulus such as an electric shock, the time performance of a given task, or other examples of muscle-based tests.

The restoration of muscle can be assessed based on the amount, or degree, of improvement of certain muscle attributes. In particular, the muscle attribute (e.g., muscle mass, strength, etc.) may improve by about 1-fold, 2-fold, 3-fold, 4-fold, 5-fold, 6-fold, 7-fold, 8-fold, 9-fold, 10-fold, 20-fold, 30-fold, 50-fold, 60-fold, 70-fold, 80-fold, 100-fold, 150-fold, 200-fold, 250-fold, or 300-fold or more. In some cases, the muscle attribute may improve by > 1%, > 5%, > 10%,> 15%, > 20%, > 25%, > 50 %, > 60%, > 70%, > 75%, > 80% , > 90%, > 95%, > 99 %, > 100% or more. In some more particular cases, muscle strength improves by > 1%, > 5% , > 10%, > 15%, > 20%, > 25%, > 50 %, > 60%, > 70%, > 75%, > 80% , > 90%, > 95%, > 99 %, > 100%, > 200% or more. The improvement to the muscle attribute may occur within a certain time period, such as within about 1 day, 2 days, 3 days, 4 days, 5 days, 7 days, 10 days, 2 weeks, 3 weeks, 4 weeks, 1 month, 6 weeks, 2 months, 10 weeks, 3 months, 3.5 months, 4 months, 4.5months, 5 months, 5.5 months, 6 months, 6.5 months, 7 months, 7.5 months, 8 months, 8.5 months, 9 months, 9.5 months, 10 months, 10.5 months, 11 months, 11.5 months, 12 months, 1.5 years, or 2 years, or more from the time of introduction of the satellite-like or satellite cells. For example, in some cases, the improvement of the muscle attribute (e.g., strength, mass, etc.) may be > 1% within a month, > 5% within a month, > 10% within a month, > 15% within a month, > 20% within a month, > 25% within a month, > 50 % within a month, > 60% within a month, > 70% within a month, > 75% within a month, > 80% within a month, > 90% within a month, > 95% within a month, > 99 % within a month, > 100% within a month, >200% within a month, >250% within a month, >300% within a month, >400% within a month, >500% within a month or an even higher percentage within a month.

In some cases, treatment with satellite cells or satellite-like cells can result in the halting or slowing of muscle degeneration within a certain time period. In some cases, the rate of muscle degeneration can be slowed by about > 1% within a month, > 5% within a month, > 10% within a month, > 15% within a month, > 20% within a month, > 25% within a month, > 50 % within a month, > 60% within a month, > 70% within a month, > 75% within a month, > 80% within a month, > 90% within a month, > 95% within a month, > 99 % within a month, > 100% within a month, >200% within a month, >250% within a month, >300% within a month, >400% within a month, >500% within a month or by an even higher percentage within a month of treatment with the cells. Additionally, muscle degeneration may be completely halted based on cell therapy using satellite cells or satellite-like cells. Muscle degeneration may be completely halted within a certain period of time, such as within about 1 day, 2 days, 3 days, 4 days, 5 days, 7 days, 10 days, 2 weeks, 3 weeks, 4 weeks, 1 month, 6 weeks, 2 months, 10 weeks, 3 months, 3.5 months, 4 months, 4.5 months, 5 months, 5.5 months, 6 months, 6.5 months, 7 months, 7.5 months, 8 months, 8.5 months, 9 months, 9.5 months, 10 months, 10.5 months, 11 months, 11.5 months, 12 months, 1.5 years, or 2 years, or more from the time of introduction of the satellite-like or satellite cells.

### C. Drug Screening

In addition to uses in cell therapies, satellite cells or satellite-like cells may be used to serve as a platform for drug screening. In particular, drugs may be assayed to test effects on a phenotype of the satellite cells or satellite-like cell such as cell morphology, marker expression, proliferation or differentiation. In some cases, the phenotype is associated with muscle function. The cells disclosed herein may also be useful for drug screening, disease modeling and disease research for such genetic diseases or disorders.

In one example, cells that are tested are healthy satellite cells or satellite-like cells that are chemically differentiated from healthy pluripotent stem cells. In another example, cells that are tested are diseased satellite cells or satellite-like cells that are differentiated from diseased pluripotent stem cells. Diseased pluripotent stem cells may include pluripotent stem cells that have particular genetic mutations associated with genetic diseases, such as neuromuscular genetic diseases, such as muscular dystrophy. In some cases, the diseased pluripotent stem cells are derived from a subject carrying a genetic mutation associated with a muscular degenerative disease. In some cases, the diseased pluripotent stem cells are genetically engineered to carry a mutation that causes -- or is associated with -- a muscular genetic disease. The mutation may be identical to a mutation carried by a subject (e.g., human subject), or may be substantially similar to such mutation. The diseased satellite cells or satellite-like cells may be further differentiated to myoblasts or myotubes to assay drugs, test the phenotypes of diseased myoblasts or myotubes, characterize the effects of the disease at a cellular and tissue level, or perform other assessments. Characterizing the effects of the disease may include function and morphology of the myoblasts or myotubes, marker expression of the myoblasts or myotubes, proliferation and differentiation of the myoblasts and myotubes, myotube length, myotube diameter, myotube branching, fusion index, or the number of nuclei per myotube.

In some cases, an embryonic stem cell line carrying a disease-causing mutation or mutations can be genetically modified to correct the mutation. Using the methods disclosed herein, the embryonic stem cells carrying the disease-causing mutation(s) and/or the modified, corrected embryonic stem cells may be differentiated into satellite-like or satellite cells (or cell line). The genetically modified cell line may serve as an isogenic control to the disease-affected cell line which may be particularly useful for drug screening, disease modeling, and disease research.

In another example, drugs are assayed on satellite cells or satellite-like cells to identify drugs that result in increased proliferation of the satellite cells or satellite-like cells or that result in increased or enhanced differentiation to myoblasts. These effects could be measured by any method known in the art such as EdU assays or immunofluorescence staining for Ki67 to identify proliferating cells, cell counts, immunofluorescence staining for MyoD or desmin to identify myobalsts. Such drugs may be useful to boost and activate endogenous satellite cells in patients resulting in increased muscle mass or function.

In another example, cells that are tested are healthy satellite cells or satellite-like cells that are differentiated from HLA-null pluripotent stem cells. In another example, cells that are tested are diseased HLA-null pluripotent stem cells that may include HLA-null pluripotent stem cells that have particular genetic mutations associated with genetic diseases, such as neuromuscular genetic diseases, such as muscular dystrophy.

The drug screening assays and disease modeling assays using the disclosed satellite and satellite-like cells may be designed for a wide variety of diseases and disorders, particularly genetic diseases or disorders. Exemplary diseases or disorders include, but are not limited to: Huntington's disease, Merosin deficiency 1A, nemaline myopathy, and Spinal Muscular Atrophy (SMA), and muscular dystrophy. Examples of muscular dystrophy include Becker, congenital, facioscapulohumeral (FSH), myotonic (type I and II), oculopharyngeal, distal, Duchenne muscular dystrophy, and Emery-Dreifuss muscular dystrophy. Duchenne and Becker muscular dystrophies are caused by a mutation of a gene located on the X chromosome and predominantly affect males, although females can sometimes have severe symptoms as well. Additionally, most types of muscular dystrophy are multi-system disorders with manifestations in body systems including the heart, gastrointestinal system, nervous system, endocrine glands, eyes and brain.

### D. Avoiding Transplant Rejection

In other applications, HLA-null satellite cells or satellite-like cells may be used to transplant skeletal muscle progenitor cells so as to limit rejection of the transplanted cells. In particular, HLA-null satellite cells or satellite-like cells may be transplanted in a subject and fuse with the subject's myoblasts to obtain the subject's HLA profile so as to avoid elimination of the HLA-null satellite cells or satellite-like cells by natural killer cells.

When HLA-null satellite cells or satellite-like cells are provided to a subject in need thereof, the lack of HLA characterizations allow the HLA-null satellite cells or satellite-like cells to interact with immune cells, thereby avoiding an adverse reaction of immune cells to an unrecognized cell. In this way, the subject in need thereof does not mount a significant immune rejection against said satellite cells or satellite-like cells after said introduction of said satellite cells or satellite-like cells into said subject in need thereof.

In examples, the transplantation of HLA-null satellite cells or satellite-like cells may reduce or eliminate the necessity to take immunosuppressive drugs. Examples of immunosuppressant drugs include calcineurin inhibitors, such as cyclosporine or tacrolimus, mTOR inhibitors, such as sirolimus or everolimus, purine synthesis inhibitors or purine analogues, such as mycophenolate mofetil or azathioprine, or steroids, such as prednisone. **VIII.** Storage of Cells

The cells, embryonic stem cells, the pluripotent stem cells, the induced pluripotent stem cells, HLA-null pluripotent stem cells, the satellite cells or satellite-like cells that are chemically differentiated and the HLA-null satellite cells or satellite-like cells that are differentiated from HLA-null pluripotent stem cells, or other cells described herein may be stored. Thus, cells or materials from any point during the process may be stored for future completion of the process modification for use.

The methods of storage may be any method including the methods described herein, e.g., using cryopreservation medium. Some exemplary cryopreservation media include 1-15% DMSO, glycerol, high concentrations of carbohydrates, such as trehalose, high concentrations of serum or albumin. The cells preferably are frozen at a controlled cooling rate to temperatures below -70°C, and stored in a liquid nitrogen storage vessel. Other suitable cryopreservation media and methods for cryopreservation/thawing of cells generated by the methods described herein are vitrification, encapsulation, stabilization at 4°C with suitable reagents or adherent cells overlayed with suitable cryopreservation media.

### IX. Some Definitions

As used herein, the term "or" is used to refer to a nonexclusive or, such as "A or B" includes "A but not B," "B but not A," and "A and B," unless otherwise indicated.

As used herein, the term "about" when referring to a number or a numerical range means that the number or numerical range referred to is an approximation within experimental variability (or within statistical experimental error), and thus the number or numerical range may vary from, for example, between 1% and 15% of the stated number or numerical range. In examples, the term "about" refers to ±10% of a stated number or value.

As used herein, the terms "treat," "ameliorate," "treatment," and "treating" are used interchangeably. These terms refer to an approach for obtaining beneficial or desired results including, but are not limited to, therapeutic benefit and/or a prophylactic benefit. By therapeutic benefit is meant eradication or amelioration of the underlying disorder being treated. Also, a therapeutic benefit is achieved with the eradication or amelioration of one or more of the physiological symptoms associated with the underlying disorder such that an improvement is observed in the patient, notwithstanding that the patient can still be afflicted with the underlying disorder. For prophylactic benefit, the satellite cells or satellite-like cells may be administered to a patient at risk of developing a particular disease, or to a patient reporting one or more of the physiological symptoms of a disease, even though a diagnosis of this disease may not have been made.

### X. Examples

### Reference Example 1: Screening for myogenic induction conditions.

Human pluripotent stem cells (hPSC) were expanded feeder-free on collagen I-coated surfaces using commercially available M2 culture medium (manufactured by Genea Biocells) and following standard protocols. This method dissociates cultures to single cells at each passage. Batches of each reference cell line were frozen in M2 medium plus 10% DMSO following standard protocols. Each batch was quality control tested for viability, morphology, sterility, karyotype, DNA fingerprint, pluripotency marker expression (Oct4, Nanog, SSEA-4, Tral-60) and Pluritest (Muller et al., 2011). screen for myogenic induction culture conditions. Basal culture medium was prepared by adding Skeletal Muscle Cell Growth Medium Supplement Mix (manufactured by Promocell) to Skeletal Muscle Cell Basal Medium (manufactured by Promocell) according to the manufacturer's instructions to produce a medium similar to MCDB120 (U.S. Patent No. 5,143,842), to which Rho-associated kinase inhibitor Y27632 (10 FM) was also added. Cells were cultured in 384-well optical bottom microtiter plates coated with collagen **I** (100µg/mL), hFibronectin (10 µg/mL), mLaminin (5 µg/mL) or hFibronectin (10 µg/mL) with mLaminin (5 µg/mL).

To screen for myogenic induction culture conditions, the reference cell lines were dissociated to single cells and plated in 20 µL of basal culture medium at a density of 8x10³ cells/cm² in the 384-well plates. Compounds from Table 1 were added to basal culture medium in combinations of two (for a total of 378 combinations) in a 384-deep-well plate at 2x the final concentration. 20 FL of this compound-supplemented medium was added to the cultured cells in order to culture the cells in 40 µL of culture medium containing the compounds at their final concentrations. The cells were cultured at 37 °C, 5% CO2 and 5% 0₂ for nine days. Media were performed every other day while maintaining the concentration of compounds being screened at their final concentration.

At the end of the culture period, cells were fixed with 4% formalin solution, immunofluorescence stained for satellite cell markers Pax3, Pax7 and CD56 and analyzed by high-content imaging. None of the cells grown only in basal medium exhibited staining for satellite-cell markers. Of the 378 conditions screened, 34 were highly toxic to the cells. Four conditions resulted in cells that were positive for CD56 but negative for both Pax3 and Pax7. Fifteen of the conditions tested resulted in more than 50% of the cells exhibiting satellite-cell characteristics and positive staining for CD56, Pax3 and Pax7. Cells were further stained for myoblast marker MyoD, but no positive cells were observed, indicating that the satellite cells had not further differentiated to myoblasts.

**Table 1. Compounds used in screen.**

| # | Compound/Component | Final Concentration |
|---|---|---|
| 1 | Retinoic acid | 3 nM |
| 2 | dbcAMP | 1 mM |
| 3 | Creatine | 1 mM |
| 4 | Noggin | 100 ng/mL |
| 5 | IGF-1 | 10 ng/ml |
| 6 | Activin A | 6 ng/mL |
| 7 | Transferrin | 150 µg/mL |
| 8 | FGE20 ng/mL | 20 ng/mL |
| 9 | Horse scrum | 5% |
| 10 | XAV939 | 2.5 µM |
| 11 | VEGF | 25 ng/mL |
| 12 | 5-azacytidine | 10 mM |
| 13 | CHIR99021 | 3 µM |
| 14 | Forskolin | 100 µM |
| 15 | DAPT | 10 µM |
| 16 | Valproic acid (VPA) | 0.5 µM |
| 17 | PD173074 | 0.02 µM |
| 18 | SU5402 | 10 µM |
| 19 | SMO antagonist | 0.5 µM |
| 20 | Ascorbic Acid | 200 µM |
| 21 | BMP4 | 10 ng/mL |
| 22 | Alk5 inhibitor | 2 µM |
| 23 | SB431542 | 2 µM |
| 24 | BIX01294 | 1 µM |
| 25 | PD0325901 | 0.5 µM |
| 26 | PD169316 | 5 µM |
| 27 | sodium butyrate | 250 µM |
| 28 | blank | Medium w/o compound |

### Reference Example 2: Myogenic conditions are not critically dependent on scrum. growth factors specific basal media.

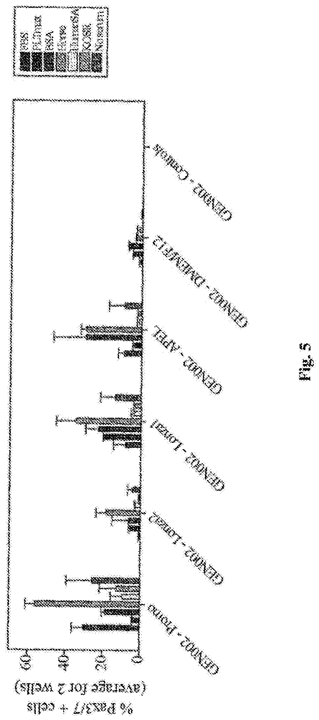

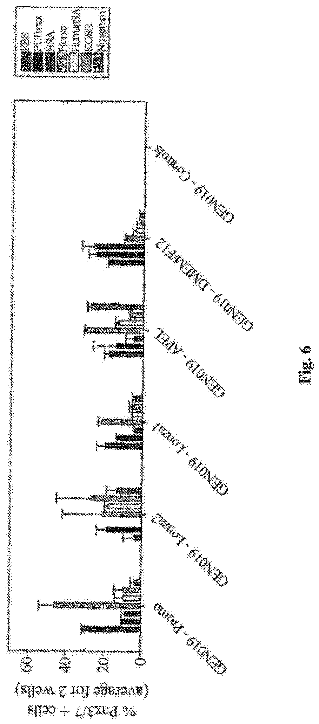

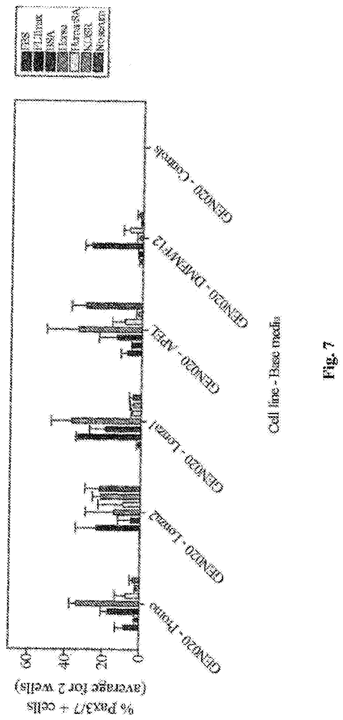

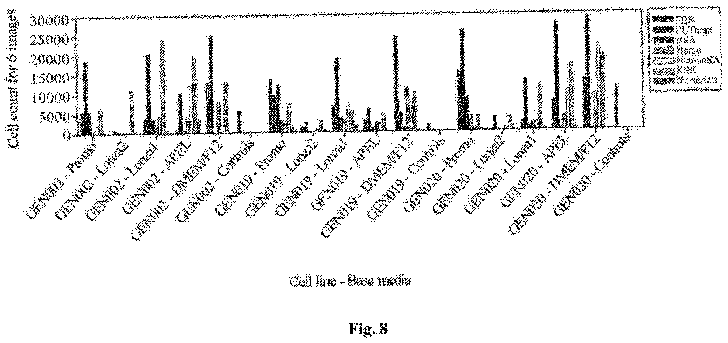

Satellite cells were prepared from GENEA002, GENEA019 and GENEA020 as described in Reference Example 1 using 041-R99021 and Alk5 inhibitor while varying the composition of the basal medium. The components of the Skeletal Muscle Cell Growth Medium Supplement were kept at their final concentration (e.g. 50 µg/mL bovine fetuin, 10 ng/mL EGF, 1 ng/mL bFGF, 10 µg/mL insulin and 0.4 µg/mL dexamethasone) and the basal medium and scrum listed in Table 2 were mixed in combinations of two, Pax3, Pax7 and CD56 positive satellite-like cells were obtained under all conditions. For example, FIGs. 5, 6, and 7 show cell lines GENEA002, GENEA019, and GENEA020 treated with compounds to induce differentiation and show the percentage of cells in each medium that express markers Pax7 and Pax3 of satellite-like cells. However, cell viability was poor in the absence of serum or albumin. For examples FIG. 8 shows CiENEA002, GENEA019, and GENEA020 grown in various differentiation media with different scrum components. The cell density is dependent on media and serum component used, indicating that differentiation is robust across different conditions and the effect of media is
largely on cell viability. The proportion of positive cells, cell expansion, and robustness across all reference cell lines varied. The Promocell and Lonna I basal media performed very similarly since they are both based on MC DB120. Horse serum appeared to support differentiation most consistently for all reference cell lines.

**Table 2. Basal media and serum components tested.**

| # | Basal Media |
|---|---|
| 1 | Promocell 'Skeletal Muscle Cell Basal Medium' (Promocell) |
| 2 | Lonza 'SkBM Basal Medium' (Lonza 1) |
| 3 | Lonza 'SkBM-2 Basal Medium' (Lonza 2) |
| 4 | Stem Cell Technologies 'APEL Medium' (APEL) |
| 5 | DMEM/F12 |

| # | Serum component |
|---|---|
| 1 | 5% fetal bovine serum (FBS) |
| 2 | 2.5% horse serum (HS) |
| 3 | 5% human serum albumin |
| 4 | 2.5% PLT-Max human platelet extract |
| 5 | 1.8% bovine serum albumin |
| 6 | 5% knock-out serum replacement (KOSR) |
| 7 | No serum |

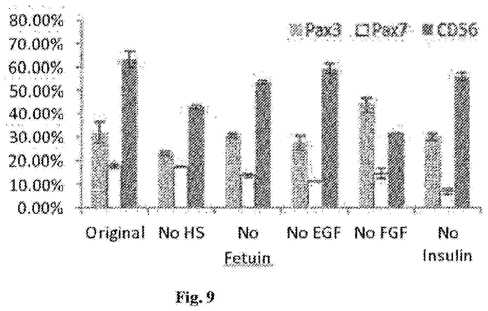

Next, the dependence on the components of the Skeletal Muscle Cell Growth Medium Supplement was tested by differentiating GENEA019 in MCDB 120-like basal medium supplemented with CHIR990214 and Alk5 inhibitor, but in which one of the components of the Skeletal Muscle Cell Growth Medium Supplement (50 µg/ml bovine fetuin, 10 ng/ml EGF, 1 ng/ml bFGF, 10 µg/ml insulin and 0.4 µg/ml dexamethasone) had been omitted or, in the case of 5% horse serum, replaced with 1.5% Albumax (bovine serum albumin manufactured by Life Technologies). Additionally, satellite-like cells positive for PaX3, Pax7 and CDS6 were obtained under each condition, demonstrating that no single growth factor is required for myogenic induction. For example, FIG. 9 shows percentage of cells expressing Pax3, Pax7, or CD56 under culture conditions containing different serum components. Differentiation is largely similar across all conditions, indicating that one serum component is critical for differentiation.

### Reference Example 3: Preparation of satellite cells from pluripotent stem cells using CHIR99021 (3 µM) and Transferrin (150 µg/mL) as contributing components.

Cultures of hPSC were grown as described in Reference Example 1 in basal medium supplemented with CHIR99021 (3 µM) and Transferrin (150 µg/mL) to induce differentiation. Cells that had been differentiated were fixed and immunostained for satellite cell markers Pax3, Pax7 and CD56. While no positive cells were observed in cells cultured in basal medium only, those cultured in basal medium supplemented with CHIR99021 (3 µM) and Transferrin (150 µg /mL) resulted in >50% of cells positively staining for said markers. These satellite-like cells were produced in all extracellular matrices tested, although hFibronectin produced the highest levels of satellite-like cells. Cells were further stained for myoblast marker MyoD, but no positive cells were identified, indicating that the cells had not further differentiated to myoblasts.

**Table 3. Efficiency of myogenic induction and myoblast differentiation.**

| **Human PSC Seeded Day 0 (2630cells/cm²) Yield of satellite-like cells at ∼Day10** | | |
|---|---|---|
| | (cells/cm²) | Ratio/Fold Increase |
| AVG | 144,179 | 54.8 |
| MIN | 45,000 | 17.1 |
| MAX | 248,143 | 94.4 |

### Reference Example 4: Preparation of satellite cells from pluripotent stem cells using CHIR99021 (3 p.M) and Ascorbic Acid (200 µg/mL) as contributing components.

Cultures of hPSC were grown as described in Reference Example 1 in basal medium supplemented with CHIR99021 (3 µM) and Ascorbic Acid (200 µg/mL) to induce differentiation. Cells that had been differentiated were fixed and immunostained for satellite cell markers Pax3, Pax7 and CD56. While no positive cells were observed in cells cultured in basal medium only, those cultured in basal medium supplemented with CHIR99021 (3 µM) and Ascorbic Acid (200 µg/mL) resulted in >50% of cells positively staining for said markers. These satellite-like cells were produced in all extracellular matrices tested, although hFibronectin produced the highest levels of satellite-like cells. Cells were further stained for myoblast marker MyoD, but no positive cells were identified, indicating that the cells had not further differentiated to myoblasts.

### Reference Example 5: Preparation of satellite cells from pluripotent stem cells using CHIR99021 (3 µM) and XAV939 (2.5 µM) as contributing components.

Cultures of hPSC were grown as described in Reference Example 1 in basal medium supplemented with CHIR99021 (3 µM) and XAV939 (2.5 µM) to induce differentiation. Cells that had been differentiated were fixed and immunostained for satellite cell markers Pax3, Pax7 and CD56. While no positive cells were observed in cells cultured in basal medium only, those cultured in basal medium supplemented with CHIR99021 (3 µM) and XAV939 (2.5 µM) resulted in >50% of cells positively staining for said markers. These satellite-like cells were produced in all extracellular matrices tested, although hFibronectin produced the highest levels of satellite-like cells. Cells were further stained for myoblast marker MyoD, but no positive cells were identified, indicating that the cells had not further differentiated to myoblasts.

### Reference Example 6: Preparation of satellite cells from pluripotent stem cells using CHIR99021 (3 µM) and VEGF (25 ng/mL) as contributing components.

Cultures of hPSC were grown as described in Reference Example 1 in basal medium supplemented with CHIR99021 (3 µM) and VEGF (25 ng/mL) to induce differentiation. Cells that had been differentiated were fixed and immunostained for satellite cell markers Pax3, Pax7 and CD56. While no positive cells were observed in cells cultured in basal medium only, those cultured in basal medium supplemented with CHIR99021 (3 µM) and VEGF (25 ng/mL) resulted in >50% of cells positively staining for said markers. These satellite-like cells were produced in all extracellular matrices tested, although hFibronectin produced the highest levels of satellite-like cells. Cells were further stained for myoblast marker MyoD, but no positive cells were identified, indicating that the cells had not further differentiated to myoblasts.

### Reference Example 7: Preparation of satellite cells from pluripotent stem cells using CHIR99021 (3 µM) and Alk5 inhibitor (2 µM) as contributing components.

Cultures of hPSC were grown as described in Reference Example 1 in basal medium supplemented with CHIR99021 (3 µM) and Alk5 inhibitor (2 µM) to induce differentiation. Cells that had been differentiated were fixed and immunostained for satellite cell markers Pax3, Pax7 and CD56. While no positive cells were observed in cells cultured in basal medium only, those cultured in basal medium supplemented with CHIR99021 (3 µM) and Alk5 inhibitor (2 µM) resulted in >50% of cells positively staining for said markers. These satellite-like cells were produced in all extracellular matrices tested, although hFibronectin produced the highest levels of satellite-like cells. Cells were further stained for myoblast marker MyoD, but no positive cells were identified, indicating that the cells had not further differentiated to myoblasts.

### Reference Example 8: Preparation of satellite cells from pluripotent stem cells using CHIR99021 (3 µM) and SB431542 (2 µM) as contributing components.

Cultures of hPSC were grown as described in Reference Example 1 in basal medium supplemented with CHIR99021 (3 µM) and SB431542 (2 µM) to induce differentiation. Cells that had been differentiated were fixed and immunostained for satellite cell markers Pax3, Pax7 and CD56. While no positive cells were observed in cells cultured in basal medium only, those cultured in basal medium supplemented with CHIR99021 (3 µM) and SB431542 (2 µM) resulted in >50% of cells positively staining for said markers. These satellite-like cells were produced in all extracellular matrices tested, although hFibronectin produced the highest levels of satellite-like cells. Cells were further stained for myoblast marker MyoD, but no positive cells were identified, indicating that the cells had not further differentiated to myoblasts.

### Reference Example 9: Preparation of satellite cells from pluripotent stem cells using CHIR99021 (3 µM) and FGF (20 ng/mL) as contributing components.

Cultures of hPSC were grown as described in Reference Example 1 in basal medium supplemented with CHIR99021 (3 µM) and FGF (20 ng/mL) to induce differentiation. Cells that had been differentiated were fixed and immunostained for satellite cell markers Pax3, Pax7 and CD56. While no positive cells were observed in cells cultured in basal medium only, those cultured in basal medium supplemented with CHIR99021 (3 µM) and FGF (20 ng/mL) resulted in >50% of cells positively staining for said markers. These satellite-like cells were produced in all extracellular matrices tested, although hFibronectin produced the highest levels of satellite-like cells. Cells were further stained for myoblast marker MyoD, but no positive cells were identified, indicating that the cells had not further differentiated to myoblasts.

### Reference Example 10: Preparation of satellite cells from pluripotent stem cells using CHIR99021 (3 µM) and BIX01294 (1 µM) as contributing components.

Cultures of hPSC were grown as described in Reference Example 1 in basal medium supplemented with CHIR99021 (3 µM) and BIX01294 (1 µM) to induce differentiation. Cells that had been differentiated were fixed and immunostained for satellite cell markers Pax3, Pax7 and CD56. While no positive cells were observed in cells cultured in basal medium only, those cultured in basal medium supplemented with CHIR99021 (3 µM) and BIX01294 (1 µM) resulted in >50% of cells positively staining for said markers. These satellite-like cells were produced in all extracellular matrices tested, although hFibronectin produced the highest levels of satellite-like cells. Cells were further stained for myoblast marker MyoD, but no positive cells were identified, indicating that the cells had not further differentiated to myoblasts.

### Reference Example 11: Preparation of satellite cells from pluripotent stem cells using CHIR99021 (3 µM) and IGF-1 (10 ng/mL) as contributing components.

Cultures of hPSC were grown as described in Reference Example 1 in basal medium supplemented with CHIR99021 (3 µM) and IGF-1 (10 ng/mL) to induce differentiation. Cells that had been differentiated were fixed and immunostained for satellite cell markers Pax3, Pax7 and CD56. While no positive cells were observed in cells cultured in basal medium only, those cultured in basal medium supplemented with CHIR99021 (3 µM) and IGF-1 (10 ng/mL) resulted in >50% of cells positively staining for said markers. These satellite-like cells were produced in all extracellular matrices tested, although hFibronectin produced the highest levels of satellite-like cells. Cells were further stained for myoblast marker MyoD, but no positive cells were identified, indicating that the cells had not further differentiated to myoblasts.

### Reference Example 12: Preparation of satellite cells from pluripotent stem cells using CHIR99021 (3 µM) and Noggin (100 ng/mL) as contributing components.

Cultures of hPSC were grown as described in Reference Example 1 in basal medium supplemented with CHIR99021 (3 µM) and Noggin (100 ng/mL)to induce differentiation. Cells that had been differentiated were fixed and immunostained for satellite cell markers Pax3, Pax7 and CD56. While no positive cells were observed in cells cultured in basal medium only, those cultured in basal medium supplemented with CHIR99021 (3 µM) and Noggin (100 ng/mL) resulted in >50% of cells positively staining for said markers. These satellite-like cells were produced in all extracellular matrices tested, although hFibronectin produced the highest levels of satellite-like cells. Cells were further stained for myoblast marker MyoD, but no positive cells were identified, indicating that the cells had not further differentiated to myoblasts.

### Reference Example 13: Preparation of satellite cells from pluripotent stem cells using CHIR99021 (3 µM) and Creatine (1 mM) as contributing components.

Cultures of hPSC were grown as described in Reference Example 1 in basal medium supplemented with CHIR99021 (3 µM) and Creatine (1 mM) to induce differentiation. Cells that had been differentiated were fixed and immunostained for satellite cell markers Pax3, Pax7 and CD56. While no positive cells were observed in cells cultured in basal medium only, those cultured in basal medium supplemented with CHIR99021 (3 µM) and Creatine (1 mM) Transferrin (150 µg/mL) resulted in >50% of cells positively staining for said markers. These satellite-like cells were produced in all extracellular matrices tested, although hFibronectin produced the highest levels of satellite-like cells. Cells were further stained for myoblast marker MyoD, but no positive cells were identified, indicating that the cells had not further differentiated to myoblasts.

### Reference Example 14: Preparation of satellite cells from pluripotent stem cells using CHIR99021 (3 µM) and PD169316 (150 ug/mL) as contributing components.

Cultures of hPSC were grown as described in Reference Example 1 in basal medium supplemented with CHIR99021 (3 µM) and PD169316 (150 µg/mL) to induce differentiation. Cells that had been differentiated were fixed and immunostained for satellite cell markers Pax3, Pax7 and CD56. While no positive cells were observed in cells cultured in basal medium only, those cultured in basal medium supplemented with CHIR99021 (3 µM) and PD169316 (150 µg/mL) resulted in >50% of cells positively staining for said markers. These satellite-like cells were produced in all extracellular matrices tested, although hFibronectin produced the highest levels of satellite-like cells. Cells were further stained for myoblast marker MyoD, but no positive cells were identified, indicating that the cells had not further differentiated to myoblasts.

### Reference Example 15: Preparation of satellite cells from pluripotent stem cells using CHIR99021 (3 µM) and SMO Antagonist (150 ug/mL) as contributing components.

Cultures of hPSC were grown as described in Reference Example 1 in basal medium supplemented with CHIR99021 (3 µM) and SMO Antagonist (150 µg/mL) to induce differentiation. Cells that had been differentiated were fixed and immunostained for satellite cell markers Pax3, Pax7 and CD56. While no positive cells were observed in cells cultured in basal medium only, those cultured in basal medium supplemented with CHIR99021 (3 µM) and SMO Antagonist (150 µg/mL resulted in >50% of cells positively staining for said markers. These satellite-like cells were produced in all extraceullar matrices tested, although hFibronectin produced the highest levels of satellite-like cells. Cells were further stained for myoblast marker MyoD, but no positive cells were identified, indicating that the cells had not further differentiated to myoblasts.

### Reference Example 16: Preparation of satellite cells from pluripotent stem cells using CHIR99021 (3 µM) and Horse Serum (150 ug/mL) as contributing components.

Cultures of hPSC were grown as described in Reference Example 1 in basal medium supplemented with CHIR99021 (3 µM) and Horse Serum (5%) to induce differentiation. Cells that had been differentiated were fixed and immunostained for satellite cell markers Pax3, Pax7 and CD56. While no positive cells were observed in cells cultured in basal medium only, those cultured in basal medium supplemented with CHIR99021 (3 µM) and Horse Serum (5%) resulted in >50% of cells positively staining for said markers. These satellite-like cells were produced in all extraceullar matrices tested, although hFibronectin produced the highest levels of satellite-like cells. Cells were further stained for myoblast marker MyoD, but no positive cells were identified, indicating that the cells had not further differentiated to myoblasts.

### Reference Example 17: Preparation of satellite cells from pluripotent stem cells using CHIR99021 (3 µM) and Sodium Butyrate (250 µM) as contributing components.

Cultures of hPSC were grown as described in Reference Example 1 in basal medium supplemented with CHIR99021 (3 µM) and sodium butyrate (250 µM) to induce differentiation. Cells that had been differentiated were fixed and immunostained for satellite cell markers Pax3, Pax7 and CD56. While no positive cells were observed in cells cultured in basal medium only, those cultured in basal medium supplemented with CHIR99021 (3 µM) and sodium butyrate (250 µM) resulted in >50% of cells positively staining for said markers. These satellite-like cells were produced in all extracellular matrices tested, although hFibronectin produced the highest levels of satellite-like cells. Cells were further stained for myoblast marker MyoD, but no positive cells were identified, indicating that the cells had not further differentiated to myoblasts.

### Reference Example 18. Myoblasts from different reference cell lines fuse to form mixed myotubes.

Pluripotent stem cells from one reference cell line are differentiated to myoblasts, plated in a culture dish, and labeled with green fluorescent CellTracker nanocrystals, e.g., as obtained from Life Technologies (TM). CellTracker is taken up by the cells and remains passively present until it is diluted out by ongoing cell divisions. Pluripotent stem cells from another reference cell line will be differentiated to myoblasts and labeled with red fluorescent CellTracker nanocrystals before dissociating them and adding hem t the first myoblasts. Myotube formation will be induced using Genea Biocells' myotube medium. After approximately 1 week in culture cells will be fixed and counter stained with Hoechst 33242 dye. High-content imaging will be used to detect and quantify mixed (green and red fluorescent) myotubes.

### Reference Example 19. Propagation of satellite cells or satellite-like cells.

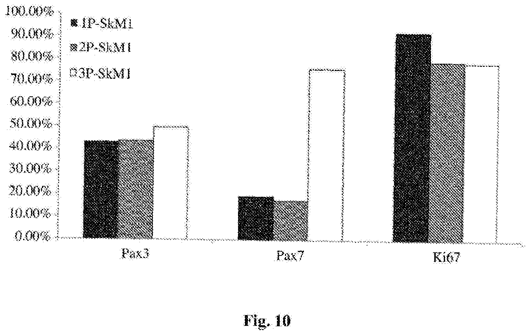

Additionally, **FIG. 10** is an illustration of the propagation of satellite cells or satellite-like cells over three passages, in accordance with embodiments of the present disclosure. The proportion of Pax3-positive cells remains roughly constant throughout the three passages, whereas more Pax7-positive cells are found at later passages. As seen in **FIG. 10****,** the majority of cells seem to be proliferating (Ki67-positive).

### Reference Example 20. Xeno-free, growth-factor free preparation of satellite cells.

The generation of satellite-like cells in xeno-free conditions was tested. Satellite-like cells were prepared from a human embryonic stem reference cell line (GENEA019) using the following media:
1) Control medium: Skeletal Muscle Cell Basal Medium (Lonza) with Skeletal Muscle Cell Growth Supplement (Lonza) and Horse Serum (5%) supplemented with Y-27632 (10 µM), CHIR99021 (3 µM) and Alk5 inhibitor (2 µM).
2) MCDBc: MCDB120 (2g/L) with Skeletal Muscle Cell Growth Supplement (Lonza) and horse serum (5%) supplemented with Y-27632 (10 µM), CHIR99021 (3 µM) and Alk5 inhibitor (2 µM).
3) MCDB FA: MCDB120 (2g/L) with no horse serum, fetuin, EGF, or FGF and supplemented with human albumin (2.5%), oleic acid (100ng), linoleic acid (100ng), Y-27632 (10 µM), CHIR99021 (3 µM), and Alk5 inhibitor (2 µM).
4) MCDB FA ITS: same as MCDB FA (#3), additionally with insulin/transferrin/selenium (ITS, 100X).

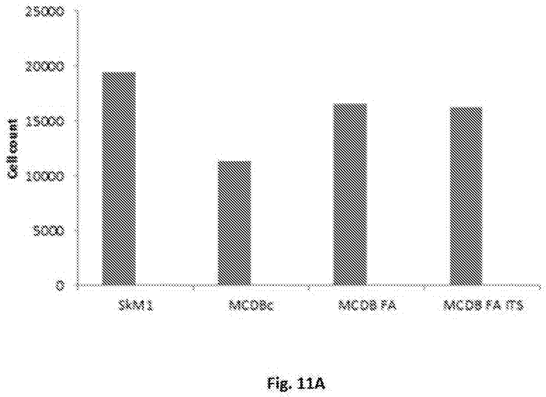

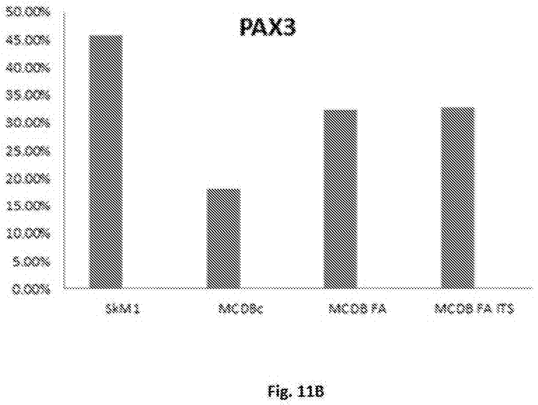

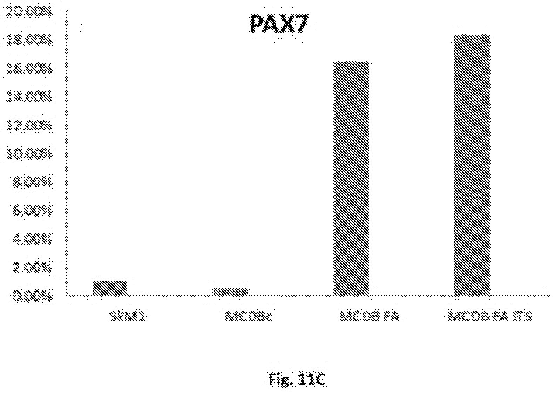

MCDB FA is a xeno-free, growth-factor-free formulation and MCDB FA ITS is a xeno-free formulation. Cells were fixed after 3 days of myogenic induction and stained for nuclei (cell count) and Pax3 and Pax7. **FIGS. 11A-11C** depict robust induction of myogenic cells in all tested media, including increased cell counts (**FIG. 11A**), and increased expression of Pax3 (**FIG. 11B**) and Pax7 (**FIG. 11C**).

### Reference Example 21. Myogenic induction on laminins affects satellite-like cell populations.

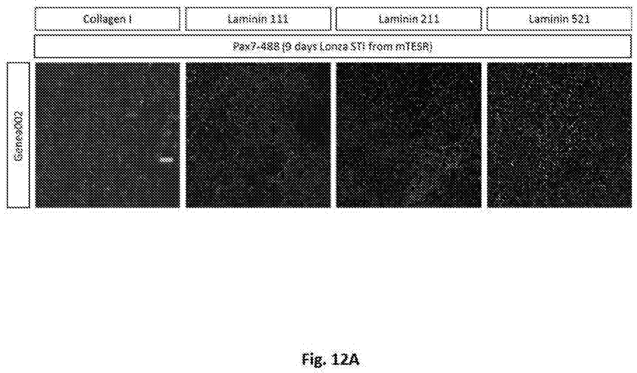

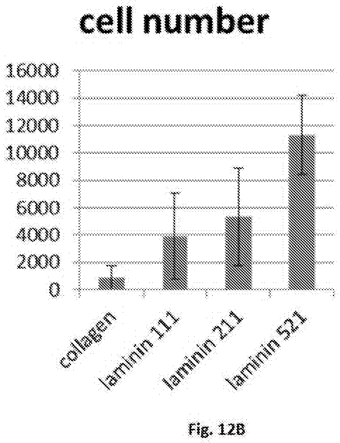

GENEA019 human embryonic cells were plated in Skeletal Muscle Cell Basal Medium (Lonza) with Skeletal Muscle Cell Growth Supplement (Lonza) and horse serum (5%) supplemented with Y-27632 (10 µM), CHIR99021 (3 µM) and Alk5 inhibitor (2 µM) on tissue culture plates coated with the extracellular matrix molecules collagen I, laminin 111, laminin 211 and laminin 521. After 9 days, cells were fixed and stained for DNA (Hoechst) and Pax7. Laminins, and particularly laminin 521, were found to promote cell proliferation (**FIG. 12B**) and favor a satellite-like cell sub population that is Pax7-positive (**FIG. 12A**).

### Reference Example 22. Improved preparation of satellite cells using LRRK2/DCLK inhibitors.

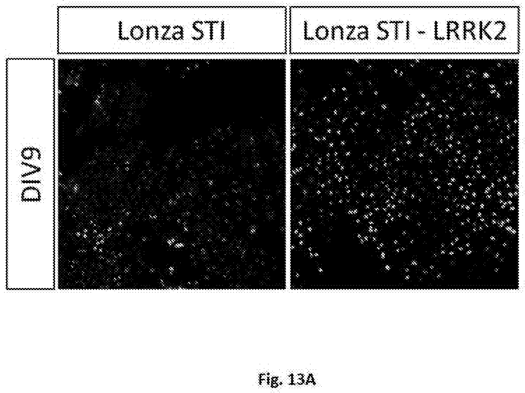

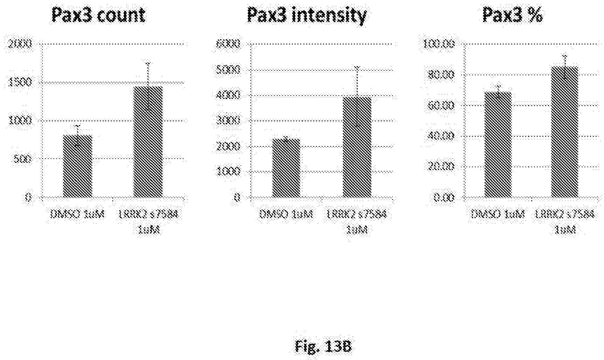

GENEA019 human embryonic stem cells were plated in Skeletal Muscle Cell Basal Medium (Lonza) with Skeletal Muscle Cell Growth Supplement (Lonza) and horse serum (5%) supplemented with Y-27632 (10 µM), CHIR99021 (3 µM), and Alk5 inhibitor (2 µM) with or without the LRRK2 inhibitor, LRRK2-IN-1 (1 µM). After 9 days, cells were fixed and stained for DNA (Hoechst) and Pax3. The addition of LRRK2-IN-1 promoted cell proliferation (**FIG. 13A**) and increased expression of Pax3 (**FIG. 13B**).

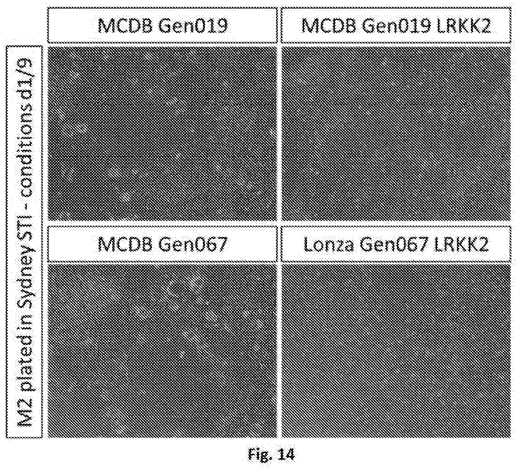

The same experiment was repeated using GENEA019 and GENEA067 human embryonic stem reference cell line lines. Cell populations were observed by phase contrast microscopy. As seen in **FIG. 14****,** the addition of LRRK2-IN-1 resulted in purer cell populations, suppressing flat cells with large cytoplasms that are observed as contaminating cells.

### Reference Example 23. Improved preparation of satellite cells using LRRK2/DCLK inhibitors.

GENEA002, GENEA015, and GENEA019 human embryonic stem reference cell lines were plated on collagen I-coated culture dishes in the following culture media:
1) MCDBc: MCDB120 (2g/L) with Skeletal Muscle Cell Growth Supplement (Lonza) and horse serum (5%), supplemented with Y-27632 (10 µM), CHIR99021 (3 µM) and Alk5 inhibitor (2 µM).
2) MCDB Min: MCDB120 (2g/L) with no horse serum, fetuin, EGF or FGF, and supplemented with human albumin (2.5%), Y-27632 (10 µM), CHIR99021 (3 µM) and Alk5 inhibitor (2 µM).
3) MCDB Min FA: MCDB120 (2g/L) basal medium with no horse serum, fetuin, EGF or FGF, and supplemented with human albumin (2.5%), oleic acid (100ng), linoleic acid (100ng), Y-27632 (10 µM), CHIR99021 (3 µM) and Alk5 inhibitor (2 µM).
4) MCDB FA ITS: same as MCDB FA with insulin/transferrin/selenium (ITS, 100X)

A control compound, 5 µM THI (2-acetyl-4(5)-(1,2,3,4-Tetrahydroxybutyl)imidazole), or 5 ng/L FGF2 was optionally added to the media. The LRRK2 inhibitor, LRRK2-IN-1 (1 µM), was optionally added to MCDB MinFA medium. Cells were fixed after 4 days and stained for DNA (Hoechst), Pax3 and Pax7. As seen in **FIGS. 15A-F**, neither THI nor FGF2 enhanced cell proliferation or the yield of Pax3/7-positive cells. However, surprisingly, LRRK2 inhibitor LRRK2-IN-1 resulted in a strong increase in the proportion of Pax3-positive and Pax7-positive cells.

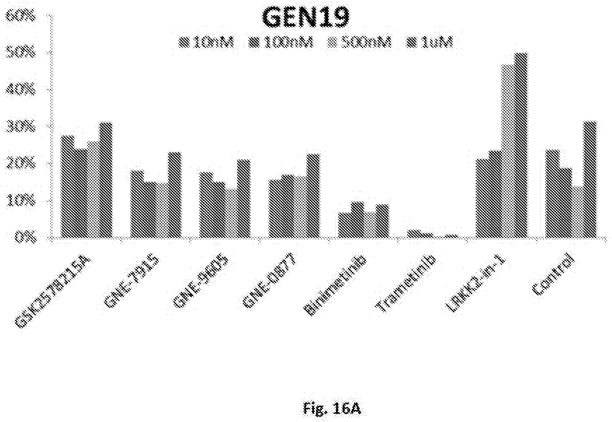

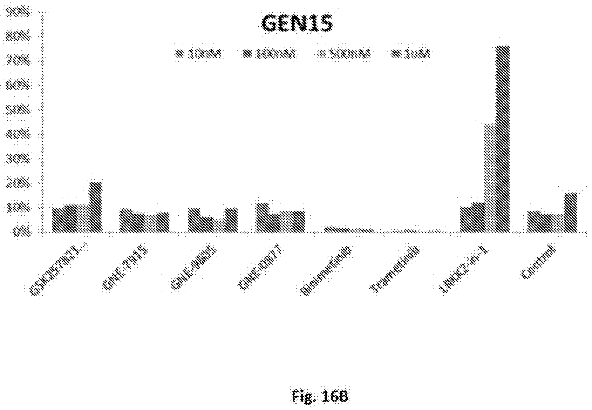

The same experiment was repeated with GENEA015 and GENEA019 human embryonic stem cells in Skeletal Muscle Cell Basal Medium (Lonza) with Skeletal Muscle Cell Growth Supplement (Lonza) and horse serum (5%) supplemented with Y-27632 (10 µM), CHIR99021 (3 µM) and Alk5 inhibitor (2 µM). In addition to LRRK2-IN-1, various other kinase inhibitors were tested in a dose range from 10 nM to 1 µM. Cells were fixed after 4 days and stained for DNA (Hoechst) and Pax7. As shown in **FIGS. 16A** and **16B****,** the LRKK2 inhibitor, LRRK2-IN-1, but not the other kinase inhibitors, strongly promoted the formation of Pax7-positive cells in a dose-dependent manner.

### Reference Example 24. Myogenic induction depends on Wnt activation/GSK3b inhibition.

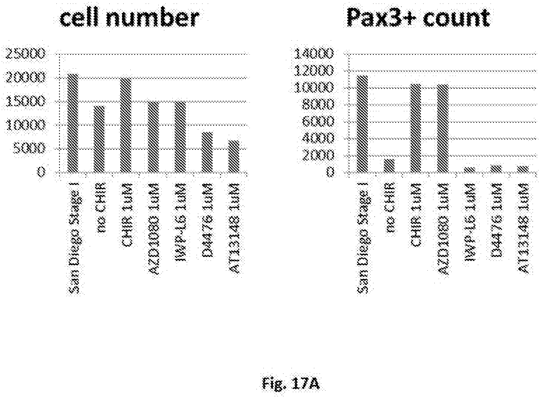

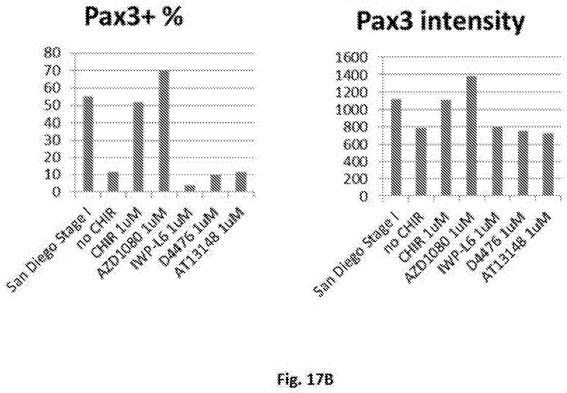

GENEA019 human embryonic stem cells were plated in Skeletal Muscle Cell Basal Medium (Lonza) with Skeletal Muscle Cell Growth Supplement (Lonza) and horse serum (5%) supplemented with Y-27632 (10 µM) and Alk5 inhibitor (2 µM), and a series of Wnt pathway modulators, including CHIR99021 (1 µM), AZD1080 (1 µM), IWP-L6 (1 µM), D4476 (1 µM), and AT13148 (1 µM). After 9 days in culture, cells were fixed and stained for DNA (Hoechst) and Pax3. As seen in **FIGS. 17A** and **17B****,** in the absence of Wnt modulation, no myogenic induction was observed. Both the GSK3 kinase inhibitors, CHIR99021 and AZD1080 promoted myogenic induction and resulted in Pax3-positive cells. Blocking Wnt-signaling via IWP-L6 blocked myogenic induction. Other indirect Wnt-pathway activators did not result in myogenic induction.

### Reference Example 25. Inhibition of Rho-associated kinase (ROCK) is important throughout the differentiation process.

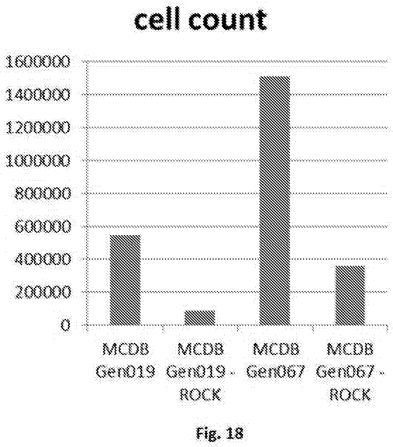

GENEA019 and GENEA067 human embryonic stem cells were plated in Skeletal Muscle Cell Basal Medium (Lonza) with Skeletal Muscle Cell Growth Supplement (Lonza) and horse serum supplemented with Y-27632 (10 µM), CHIR99021 (3 µM) and Alk5 inhibitor (2 µM) on collagen I-coated plates. Cells were either differentiated further in the same medium with media changes every other day, or one day after plating and after cells had attached, cells were changed to and maintained in the same culture medium but without the ROCK inhibitor, Y-27632 (10 µM). Surprisingly, the cells without continuous ROCK inhibition formed highly heterogeneous cell populations during differentiation and were significantly growth retarded (**FIG. 18**; "-ROCK" indicates Y-27632 was removed after cells had attached), suggesting there is a more specific role for ROCK inhibition during myogenic induction.

### Reference Example 26. In vitro fusion of satellite-like cells with mouse myoblasts.

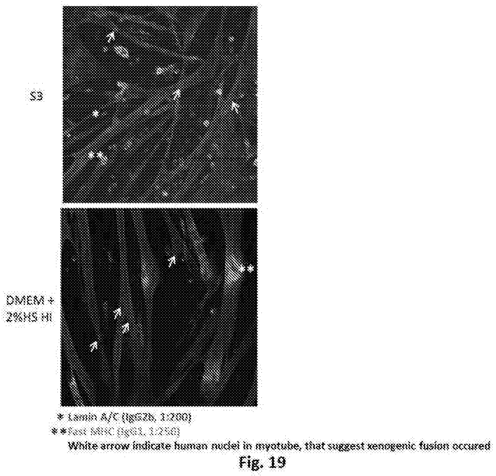

GENEA019 human embryonic stem cells were plated in Skeletal Muscle Cell Basal Medium (Lonza) with Skeletal Muscle Cell Growth Supplement (Lonza) and horse serum supplemented with Y-27632 (10 µM), CHIR99021 (3 µM) and Alk5 inhibitor (2 µM) on collagen I-coated plates. Cells were passaged after 9 days and re-plated in Genea Biocells Myoblast Medium (Genea Biocells). After 7 days, cells were harvested and re-plated 1:1 together with immortalized mouse myoblast C2C12 cells at a total cell density of 60,000 cells/cm². The following day, the culture medium was changed to either Genea Biocells Myotube Medium (Genea Biocells) or DMEM low glucose + 2% horse serum. After a further 2 days, cells were fixed and stained for DNA (Hoechst), fast MHC and lamin A/C. Cells were analyzed by confocal fluorescence microscopy. As shown in **FIG. 19**, the presence of human lamin A/C-positive nuclei within myotubes with mouse lamin A/C-negative nuclei demonstrated the potential of stem cell-derived GENEA019 myoblasts to fuse with another myoblast population.

### Reference Example 27. Selection of guide RNAs (gRNAs) targeting HLA class I genes.

The HLA super locus, which is situated on chromosome 6p21.3, has the highest frequency of polymorphism of the human genome making it difficult to create a general set of gRNAs that targets the multiple HLA class I alleles and constitutes a major obstacle for genome edition of the HLA region. The complete excision of the 6p21.3 region is also problematic because it comprises a number of essential genes which includes POU5F1 (also known as Oct4) which is critical for the maintenance of pluripotency. Alternatively, higher specificity gRNAs may be generated against each target gene individually. Ideally, reference cell lines which are homozygous for one or more HLA groups can be used for genomic edition.

A selection of embryonic stem reference cell lines were selected for genome edition based on their efficiency in producing "good quality" skeletal muscle once submitted to a differentiation protocol (using the Genea Biocells Skeletal Muscle Differentiation Kit). The chosen lines were sent for HLA class I typing by sequencing. The sequencing provides information regarding the type (allele group), subtype (specific HLA protein) and synonymous nucleotide substitutions. The HLA class I profile of three human embryonic reference cell lines is depicted in **Table 4,** and the particular sequence for each of the alleles can be assessed at the IMGT/HLA database.

**Table 4. Representative HLA-typing by sequencing of three human embryonic stem reference cell lines.**

| *Sample ID* | *IMGT*/ *A3.19.0.1 2015-01-19* | | *IMGT*/*B 3.19.0.1 2015-01-19* | | *IMGT*/ *C3.19.0.1 2015-01-19* | |
|---|---|---|---|---|---|---|
| Genea 002 | A *02:01:01 | A *32:01:01 | B*14:02:01 | B*15:01:01 | C*03:03:01 | C*08:02:01 |
| Genea 015 | A *01:01:01 | A *32:01:01 | B*44:02:01 | B*57:01:01 | C*05:01:01 | C*06:02:01 |
| Genea 016 | A *01:01:01 | A *24:02:01 | B*39:06:02 | B*57:01:01 | C*06:02:01 | C*07:02:01 |

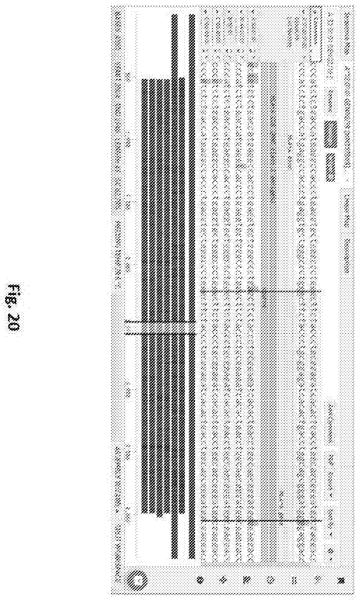

The "regions of interest" were defined by first aligning all HLA class I genes of a given reference cell line and finding areas of homology among all three HLA class I (A, B and C) alleles. Besides the polymorphisms, there is a high degree of sequence homology between the members of class I. As an example, the sequences of both alleles for HLA-A, HLA-B and HLA-C of the human embryonic reference cell line GENEA015 were aligned (**FIG. 20**). All exonic homolog regions which are longer than 20 nucleotides were investigated for the presence of protospacer adjacent motif (PAM) sequences for Cas9, or other genome editing enzymes.

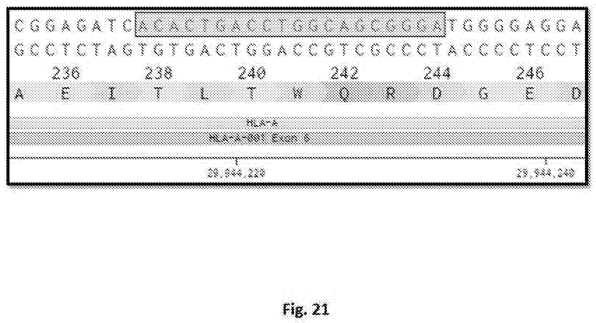

As depicted in **FIG. 20****,** the sequence "gcttctaccctgcggagatcacactgacctggcagcgggatgg" is 43 nucleotides long and is a common sequence for all GENEA015 HLA class I alleles. A search for PAM sequences in this region returned 6 distinct potential targets against which gRNAs were synthesized. Each gRNA had an intrinsic specificity (according to the specificity score from Hsu et al., 2013) and efficiency (according to the activity score from Doench et al., 2014) (**see Table 5**) and, as a general rule, the higher the value for both indexes, the better the quality of the guide. As shown in **FIG. 21****,** the target sequence sits on exon 6 of HLA-A.

**Table 5. List of gRNAs that target a consensus region between HLA-A, B and C in GEN015. Score is 0-100, higher score indicates more specific or efficient**

| **Sequence** | **PAM** | **Specifidty Score** | **Efficiency Score** |
|---|---|---|---|
| acactgacctggcagcggga | tgg | 15.813 | 32.082 |
| caggtcagtgtgatctccgc | agg | 12.967 | 4.687 |
| aggtcagtgtgatctccgca | ggg | 12.771 | 92.145 |
| ctgcggagatcacactgacc | tgg | 12.574 | 8.953 |
| gatcacactgacctggcagc | ggg | 11.725 | 36.069 |
| agatcacactgacctggcag | egg | 11.007 | 51.946 |

### Reference Example 28. Knock-out of HLA locus in human embryonic stem reference cell lines.

Once suitable HLA target sequences are identified, for example, as demonstrated in Reference Example 27, gRNAs can be synthesized and delivered to cells in combination with an RNA-guided DNA endonuclease enzyme in a form of nucleic acid or as a protein. The delivery can be done via many different methods (e.g., transfection, lipofection, transduction, calcium chloride, nucleofection, electroporation, proteofection). The induction of expression of surface proteins, like CD4, or fluorescent proteins may aid in the identification and further sorting of the cells which successfully received the enzyme. Alternatively, an antibiotic resistance cassette can be introduced to allow for selection of the positive cells by incubating the cells with the appropriate antibiotic.

The sorted/selected cells are pooled together and expanded. After expansion, a sample of the pooled cells is subjected to T7 endonuclease I (or SURVEYOR) to check for edition efficiency. Briefly, genomic DNA is extracted and the targeted regions are amplified by PCR. The amplicons are purified, denatured, annealed and incubated in the presence of T7 endonuclease I. The heteroduplexes generated by the annealing of non-perfect matching DNA will be cleaved by the endonuclease. The reaction is stopped and the product is purified and resolved by 2% agarose gel. The number of bands visible in the gel can be used for an estimation of the percentage of gene modification.

As soon as edition is found, cells are plated in clonal dilution and the clones, once expanded, are submitted to the same process described above for the pooled cells but, alternatively, the amplified target can be subject directly to sequencing or High-Resolution Melting (HRM) for the profiling of the modification. Clones found to be positive for the edition are subjected to internal quality control (pluripotency, comparative genomic hybridization, mycoplasma). Ultimately, selected clones of edited cells are resubmitted to HLA typing and/or have their whole genome sequenced. The ability of HLA-KO reference cell lines to generate satellite-like cells and myotubes is confirmed. The absence of HLA antigen is confirmed at the satellite cell, myoblast and myotube stage.

## Claims

1. A method of producing cells expressing CD56/Pax3, CD56/Pax7, or Pax3/Pax7, the method comprising providing a pluripotent stem cell in an *in vitro* culture and contacting the pluripotent stem cell in the *in vitro* culture with two or more compounds at the same time, wherein the two or more compounds comprise a GSK3β inhibitor and a TGF-β receptor inhibitor, wherein the contacting the pluripotent stem cell in the *in vitro* culture with the two or more compounds comprising a GSK3β inhibitor and a TGF-β receptor inhibitor, directly results in generation of cells expressing CD56/Pax3, CD56/Pax7, or Pax3/Pax7.

2. The method of claim 1, wherein the cells expressing CD56/Pax3, CD56/Pax7, or Pax3/Pax7 have the potential to form myoblasts.

3. The method of claim 1, wherein the generation of cells expressing CD56/Pax3, CD56/Pax7, or Pax3/Pax7 is not caused by transfection of a nucleic acid.

4. The method of claim 1, wherein the generation of cells expressing CD56/Pax3, CD56/Pax7, or Pax3/Pax7 occurs in a single step.

5. The method of claim 1, wherein the pluripotent stem cell is a human pluripotent stem cell, human embryonic stem cell, or human induced pluripotent stem cell.

6. The method of claim 1, wherein the human pluripotent stem cell is a genetically modified human pluripotent stem cell; a human pluripotent stem cell comprising a mutation associated with a genetic muscle disease or disorder; or the human pluripotent stem cell is genetically modified to correct a phenotype of a subject with a genetic disease or disorder.

7. The method of claim 1, wherein at least a portion of the cells expressing CD56/Pax3, CD56/Pax7, or Pax3/Pax7 are Pax3/Pax7/ CD56 cells.

8. The method of claim 1, wherein less than 20 days from initially contacting the pluripotent stem cell in the *in vitro* culture with the two or more compounds at the same time, greater than five cells expressing CD56/Pax3, CD56/Pax7, or Pax3/Pax7 are produced from the pluripotent stem cell; greater than 10 cells expressing CD56/Pax3, CD56/Pax7, or Pax3/Pax7 are produced from the pluripotent stem cell; or greater than five cells expressing MyoD, MYOG or MYF5 are produced from the pluripotent stem cell.

9. The method of claim 1, wherein at least a portion of the cells expressing CD56/Pax3, CD56/Pax7, or Pax3/Pax7 are satellite-like cells.

10. The method of claim 1, wherein at least a portion of the cells expressing CD56/Pax3, CD56/Pax7, or Pax3/Pax7 have a nucleus with a cross-sectional area of at least about170 um².

11. The method of claim 1, wherein the TGF-β receptor inhibitor is an Alk inhibitor or an A1k5 inhibitor, preferably SB431542 or A83-01.

12. The method of claim 1 or 8, wherein the GSK3β inhibitor is CHIR99021 or AZD1080.

13. The method of claim 1, wherein the two or more compounds further comprise a leucinerich repeat kinase 2 (LRRK2) inhibitor.

14. The method of claim 13, wherein the LRRK2 inhibitor is LRRK2-IN-1.

## Patentansprüche

1. Verfahren zur Herstellung von Zellen, die CD56/Pax3, CD56/Pax7 oder Pax3/Pax7 exprimieren, wobei das Verfahren das Bereitstellen einer pluripotenten Stammzelle in einer *in-vitro-*Kultur und das Kontaktieren der pluripotenten Stammzelle in der *in-vitro-*Kultur mit zwei oder mehr Verbindungen zur gleichen Zeit umfasst, worin die zwei oder mehr Verbindungen einen GSK3β-Inhibitor und einen TGF-β-Rezeptor-Inhibitor umfassen, worin das Kontaktieren der pluripotenten Stammzelle in der *in-vitro-*Kultur mit den zwei oder mehr Verbindungen, die einen GSK3β-Inhibitor und einen TGF-β-Rezeptor-Inhibitor umfassen, direkt zur Erzeugung von Zellen führt, die CD56/Pax3, CD56/Pax7 oder Pax3/Pax7 exprimieren.

2. Verfahren nach Anspruch 1, worin die Zellen, die CD56/Pax3, CD56/Pax7 oder Pax3/Pax7 exprimieren, das Potenzial haben, Myoblasten zu bilden.

3. Verfahren nach Anspruch 1, worin die Erzeugung von Zellen, die CD56/Pax3, CD56/Pax7 oder Pax3/Pax7 exprimieren, nicht durch Transfektion einer Nukleinsäure verursacht wird.

4. Verfahren nach Anspruch 1, worin die Erzeugung von Zellen, die CD56/Pax3, CD56/Pax7 oder Pax3/Pax7 exprimieren, in einem einzigen Schritt erfolgt.

5. Verfahren nach Anspruch 1, worin die pluripotente Stammzelle eine menschliche pluripotente Stammzelle, eine menschliche embryonale Stammzelle oder eine menschliche induzierte pluripotente Stammzelle ist.

6. Verfahren nach Anspruch 1, worin die humane pluripotente Stammzelle eine genetisch modifizierte humane pluripotente Stammzelle ist; eine humane pluripotente Stammzelle eine Mutation umfasst, die mit einer genetischen Muskelkrankheit oder -störung assoziiert ist; oder die humane pluripotente Stammzelle genetisch modifiziert ist, um einen Phänotyp eines Subjekts mit einer genetischen Krankheit oder Störung zu korrigieren.

7. Verfahren nach Anspruch 1, worin mindestens ein Teil der Zellen, die CD56/Pax3, CD56/Pax7 oder Pax3/Pax7 exprimieren, Pax3/Pax7/CD56-Zellen sind.

8. Verfahren nach Anspruch 1, worin weniger als 20 Tage nach dem anfänglichen Kontaktieren der pluripotenten Stammzelle in der *in-vitro-*Kultur mit den zwei oder mehr Verbindungen zur gleichen Zeit mehr als fünf Zellen, die CD56/Pax3, CD56/Pax7 oder Pax3/Pax7 exprimieren, aus der pluripotenten Stammzelle erzeugt werden; mehr als 10 Zellen, die CD56/Pax3, CD56/Pax7 oder Pax3/Pax7 exprimieren, aus der pluripotenten Stammzelle erzeugt werden; oder mehr als fünf Zellen, die MyoD, MYOG oder MYF5 exprimieren, aus der pluripotenten Stammzelle erzeugt werden.

9. Verfahren nach Anspruch 1, worin mindestens ein Teil der Zellen, die CD56/Pax3, CD56/Pax7 oder Pax3/Pax7 exprimieren, satellitenartige Zellen sind.

10. Verfahren nach Anspruch 1, worin mindestens ein Teil der Zellen, die CD56/Pax3, CD56/Pax7 oder Pax3/Pax7, einen Zellkern mit einer Querschnittsfläche von mindestens etwa 170 um² aufweist.

11. Verfahren nach Anspruch 1, worin der TGF-β-Rezeptor-Inhibitor ein Alk-Inhibitor oder ein Alk5-Inhibitor ist, vorzugsweise SB431542 oder A83-01.

12. Das Verfahren nach Anspruch 1 oder 8, worin der GSK3β-Inhibitor CHIR99021 oder AZD1080 ist.

13. Verfahren nach Anspruch 1, worin die zwei oder mehr Verbindungen weiterhin einen Leucin-reichen Repeat-Kinase 2 (LRRK2)-Inhibitor umfassen.

14. Verfahren nach Anspruch 13, worin der LRRK2-Inhibitor LRRK2-IN-1 ist.

## Revendications

1. Un procédé de production de cellules exprimant CD56/Pax3, CD56/Pax7 ou Pax3/Pax7, le procédé comprenant la fourniture d'une cellule souche pluripotente dans une culture *in vitro* et le contact de la cellule souche pluripotente dans la culture *in vitro* avec deux composés ou plus en même temps, dans lequel les deux composés ou plus comprennent un inhibiteur GSK3β et un inhibiteur de récepteur TGF-β, dans lequel le contact de la cellule souche pluripotente dans la culture *in vitro* avec les deux composés ou plus comprenant un inhibiteur GSK3β et un inhibiteur de récepteur TGF-β, aboutit directement à la génération de cellules exprimant CD56/Pax3, CD56/Pax7 ou Pax3/Pax7.

2. Le procédé selon la revendication 1, dans lequel les cellules exprimant CD56/Pax3, CD56/Pax7 ou Pax3/Pax7 peuvent former des myoblastes.

3. Le procédé selon la revendication 1, dans lequel la génération de cellules exprimant CD56/Pax3, CD56/Pax7 ou Pax3/Pax7 n'est pas causée par transfection d'un acide nucléique.

4. Le procédé selon la revendication 1, dans lequel la génération de cellules exprimant CD56/Pax3, CD56/Pax7 ou Pax3/Pax7 se produit en une seule étape.

5. Le procédé selon la revendication 1, dans lequel la cellule souche pluripotente est une cellule souche pluripotente humaine, une cellule source embryonnaire humaine ou une cellule souche pluripotente humaine induite.

6. Le procédé selon la revendication 1, dans lequel la cellule souche pluripotente humaine est une cellule souche pluripotente humaine génétiquement modifiée ; une cellule souche pluripotente humaine comprenant une mutation associée avec une maladie ou un trouble musculaire génétique ; ou la cellule souche pluripotente humaine est génétiquement modifiée pour corriger un phénotype d'un sujet ayant une maladie ou un trouble génétique.

7. Le procédé selon la revendication 1, dans lequel une portion au moins des cellules exprimant CD56/Pax3, CD56/Pax7 ou Pax3/Pax7 sont des cellules Pax3/Pax7/CD56.

8. Le procédé selon la revendication 1, dans lequel moins de 20 jours à compter du contact initial de la cellule souche pluripotente dans la culture *in vitro* avec deux composés ou plus en même temps, plus de cinq cellules exprimant CD56/Pax3, CD56/Pax7 ou Pax3/Pax7 sont produites à partir de la cellule souche pluripotente ; plus de 10 cellules exprimant CD56/Pax3, CD56/Pax7 ou Pax3/Pax7 sont produites à partir de la cellule souche pluripotente ; ou plus de cinq cellules exprimant MyoD, MYOG ou MYF5 sont produites à partir de la cellule souche pluripotente.

9. Le procédé selon la revendication 1, dans lequel une portion au moins des cellules exprimant CD56/Pax3, CD56/Pax7 ou Pax3/Pax7 sont des cellules ressemblant à des cellules satellites.

10. Le procédé selon la revendication 1, dans lequel une portion au moins des cellules exprimant CD56/Pax3, CD56/Pax7 ou Pax3/Pax7 ont un noyau dont la section transversale est d'au moins 170 um².

11. Le procédé selon la revendication 1, dans lequel l'inhibiteur de récepteur TGF-β est un inhibiteur Alk ou un inhibiteur A1k5, de préférence SB431542 ou A83-01.

12. Le procédé selon la revendication 1ou 8, dans lequel l'inhibiteur GSK3β est CHIR99021 ou AZD1080.

13. Le procédé selon la revendication 1, dans lequel les deux composés ou plus comprennent en outre un inhibiteur de la kinase 2 répétée riche en leucine (LRRK2).

14. Le procédé selon la revendication 13, dans lequel l'inhibiteur LRRK2 est LRRK2-IN-1.
